# EUROPEAN PATENT APPLICATION

(11) **EP 1 916 301 A1**
(43) Date of publication of application: **30.04.2008**
(21) Application number: 06782726.1
(22) Date of filing: 08.08.2006
(51) Int. Cl.: C12N 15/09, C12Q 1/02, C12Q 1/68, G01N 33/15, G01N 33/50, A61K 45/00, A61P 35/00

(54) **METHOD FOR EVALUATING COMPOUND USING MOLECULE ON THE RB PATHWAY AS INDEX AND MOLECULAR DIAGNOSTIC METHOD**

(30) Priority: 11.08.2005 JP 2005232971
(71) Applicant: BANYU PHARMACEUTICAL CO., LTD., Tokyo 102-8667 (JP)
(72) Inventor: EGUCHI, Tomohiro, c/o Banyu Pharmaceutical Co., Ltd., Tsukuba-shi, Ibaraki, 300-2611 (JP); KOTANI, Hidehito, c/o Banyu Pharmaceutical Co., Ltd., Tsukuba-shi, Ibaraki, 300-2611 (JP)
(74) Representative: Hussain, Deeba
(86) International application number: PCT/JP2006/315992
(87) International publication number: WO 2007/018309

(57) **Abstract**

It is intended to provide a molecule which is present on the Rb pathway and can be used as a drug discovery target for an anticancer agent or a tumor marker and a method capable of evaluating a compound (a candidate compound for an anticancer agent) using the molecule. The object can be achieved by a method for evaluating a compound which detects the interaction between IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 or KIF18A gene or protein and a test compound.

## Description

### Technical Field

The present invention relates to a novel use of a gene belonging to a gene group whose transcription is regulated by a tumor suppressor gene Rb. More particularly, it relates to a method for evaluating a compound targeting a gene belonging to the gene group and use of the gene as a tumor marker.

### Background Art

Rb, which is known as a tumor suppressor gene, functions as a very important regulatory factor of cell cycle. That is, Rb regulates the transition of cell cycle from the G1 phase to S phase mediated by several transcriptional regulators such as E2F. In general, mammalian cells are arrested in the G0 phase, and enter the G1 phase by the growth stimulation, and the cell cycle progresses through the S phase, G2 phase and M phases in turn. Rb is considered to have a function to arrest the cell cycle at the

### G1 phase and terminate the DNA synthesis in the S phase in the case where an abnormality occurs in the cell cycle progression.

To be more specific, Rb protein arrests the cell cycle at the G1 phase by binding to E2F, which is required for the initiation of the S phase, thereby to stop its function. Rb is not phosphorylated in an arrested state, however, once it is phosphorylated by CDK kinase in middle of the G1 phase, it loses its activity, therefore, it cannot bind to E2F. As a result, E2F functions and the cell cycle progresses to the S phase. The activity of Rb is regulated by receiving the actions of CDK2, CDK4 and CDK6, and the regulatory pathway of a series of these cell cycle events centered on Rb is called Rb pathway. When an abnormality occurs in this pathway, an abnormality occurs also in the cell cycle, resulting in abnormal proliferation of cells to cause malignant transformation of tissues. For example, it is reported that when the Rb-E2F pathway does not function normally, the cell cycle progresses to the S phase (Non-patent document 1).

As described above, the association between cell cycle and malignant transformation is very strong, and drug discovery in which a large number of molecules associated with cell cycle such as molecules on the Rb pathway are used as target molecules for an anticancer agent has been actively advanced.
(Non-patent document 1): Genes & Development, Vol. 11, P. 1479, 1997

### Disclosure of the Invention

However, the present situation is that an anticancer agent based on a new mechanism of action or an unknown diagnostic marker for cancer has been demanded in order to obtain a more effective anticancer agent or diagnostic marker for cancer.

The present invention has been made in view of the above-mentioned problem of the prior art, and has its object to provide a molecule which is present on the Rb pathway and can be used as a drug discovery target for an anticancer agent or a tumor marker and a method capable of evaluating a compound (a candidate compound for an anticancer agent) using the molecule.

The present inventors made intensive studies in order to achieve the above objects, and as a result, they found that various molecules such as IL-8 and ECT2 increase or decrease their expression depending on the activity of Rb, and thus completed the present invention.

That is, the method for evaluating a compound of the invention is a method for evaluating a compound effective in the treatment of cancer, and is characterized by comprising the steps of:
introducing any one gene selected from the group consisting of IL-8, IL-8 receptor, ECT2, CCNE2, FEN1, KIF20A, CDC25C, KIF11, BUB1, NEK2, CCNA2 and KIF18A or a gene functionally equivalent to the gene thereby to prepare a cell expressing a protein, which is a transcription product of the gene; bringing a test compound into contact with the cell; and detecting a specific binding of the test compound to the protein.

Further, the method for evaluating a compound of the invention is a method for evaluating a compound effective in the treatment of cancer, and is characterized by comprising the steps of:
introducing any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene thereby to prepare a cell expressing a protein, which is a transcription product of the gene; bringing a test compound into contact with the cell; measuring the activity of an intracellular signal transducer induced by the contact; and comparing the above-mentioned activity with the activity of the intracellular signal transducer in the absence of contact with the test compound, and detecting a change in the activity of the intracellular signal transducer caused by the contact with the test compound.

Further, the method for evaluating a compound of the invention is a method for evaluating a compound effective in the treatment of cancer, and is characterized by comprising the steps of: introducing any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene thereby to prepare a cell expressing a protein, which is a transcription product of the gene; bringing a test compound into contact with the cell; measuring the expression level of an intracellular signal transducer mediated by the protein; and comparing the above-mentioned expression level with the expression level of the intracellular signal transducer in the absence of contact with the test compound; and detecting a change in the expression level of the intracellular signal transducer caused by the contact with the test compound.

Here, in the above-mentioned three embodiments of the method for evaluating a compound, as the gene, IL-8, IL-8 receptor or ECT2 is preferably used.

Further, the method for evaluating a compound of the invention is a method for evaluating a compound effective in the treatment of cancer, and is characterized by comprising the steps of: bringing a test compound into contact with any one protein selected from the group consisting of IL-8, IL-8 receptor, ECT2, CCNE2, FEN1, CENPE, KIF20A, CDC25C, CDCA8, KIF11, KNTC2, BUB1, NEK2, CCNA2 and KIF18A; and detecting a change in the activity of the protein caused by the contact.

Here, in the above-mentioned method for evaluating a compound, as the protein, IL-8, IL-8 receptor or ECT2 is preferably used.

Further, in the invention, a compound isolated by any one of the above-mentioned methods for evaluating a compound is also included.

Further, the molecular diagnostic method for cancer of the invention is a molecular diagnostic method for cancer, comprising the steps of: measuring the expression level of any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene in a test tissue or a test cell; comparing the above-mentioned expression level of the gene with the expression level of a corresponding gene in a normal tissue or a normal cell; and determining whether or not the expression level of the gene in the test tissue or test cell is significantly higher than the expression level of the gene in the normal tissue or normal cell based on the comparison result.

Here, in the above-mentioned molecular diagnostic method, as the gene, IL-8, IL-8 receptor or ECT2 is preferably used.

Further, the molecular diagnostic method of the invention is characterized by comprising the steps of: measuring the expression level of any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a protein functionally equivalent to the protein in a test tissue or a test cell; comparing the above-mentioned expression level of the protein with the expression level of a corresponding protein in a normal tissue or a normal cell; and determining whether or not the expression level of the protein in the test tissue or test cell is significantly higher than the expression level of the protein in the normal tissue or normal cell based on the comparison result.

Here, in the above-mentioned molecular diagnostic method, as the protein, IL-8, IL-8 receptor or ECT2 protein is preferably used.

Further, in the above-mentioned two embodiments of the molecular diagnostic method, the cancer is preferably cancer caused by an abnormality of Rb or a molecule on the Rb pathway. In each of the molecular diagnostic methods, all genes which belong to a gene group and become targets for measurement are genes present on the Rb pathway, therefore, an accurate diagnosis of such cancer can be realized.

Further, the tumor marker for detecting cancer having an abnormality of Rb or a molecule on the Rb pathway of the invention is characterized in that it is any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a protein functionally equivalent to the protein.

Further, the tumor marker for detecting cancer having an abnormality of Rb or a molecule on the Rb pathway of the invention is characterized in that it is any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene.

Further, the method for detecting a target gene for an anticancer agent of the invention is characterized by comprising the steps of: preparing an Rb expression-suppressed cell in which the expression of Rb has been suppressed by RNA interference using an Rb-expressing cell; measuring the expression level of a desired gene in the cell; and comparing the above-mentioned expression level with the expression level of the gene in the Rb-expressing cell. Here, the Rb-expressing cell is preferably U-2 OS, HCT116 or HepG2. Further, the means of suppressing the expression of Rb in the Rb expression-suppressed cell is preferably through RNA interference, and further, an siRNA to be used in the RNA interference is preferably a sequence represented by SEQ ID NO: 27. By using such a sequence, the Rb expression-suppressed cell can be more reliably prepared.

Further, the method for evaluating a compound using an Rb expression-suppressed cell of the invention is characterized by comprising the steps of: preparing an Rb expression-suppressed cell in which the expression of Rb has been suppressed using an Rb-expressing cell; bringing a test compound into contact with the Rb expression-suppressed cell and the Rb-expressing cell; measuring the activity of an intracellular signal transducer induced by the contact; and comparing the activity in the Rb expression-suppressed cell with the activity in the Rb-expressing cell.

Further, the method for evaluating a compound using an Rb expression-suppressed cell of the invention is characterized by comprising the steps of: preparing an Rb expression-suppressed cell in which the expression of Rb has been suppressed using an Rb-expressing cell; bringing a test compound into contact with the Rb expression-suppressed cell and the Rb-expressing cell; measuring the expression level of an intracellular signal transducer induced by the contact; and comparing the expression level in the Rb expression-suppressed cell with the expression level in the Rb-expressing cell. Here, in the above-mentioned two embodiments of the method for evaluating a compound using an Rb expression-suppressed cell, the Rb-expressing cell is preferably U-2 OS, HCT116 or HepG2. Further, the means of suppressing the expression of Rb in the Rb expression-suppressed cell is preferably through RNA interference, and further, an siRNA to be used in the RNA interference is preferably a sequence represented by SEQ ID NO: 27. By using such a sequence, the Rb expression-suppressed cell can be more reliably prepared.

According to the present invention, it becomes possible to provide a molecule which is present on the Rb pathway and can be used as a drug discovery target for an anticancer agent or a tumor marker and a method capable of evaluating a compound (a candidate compound for an anticancer agent) using the molecule. Accordingly, it becomes possible to provide a therapeutic agent or a diagnostic agent for tumor or cancer caused by an abnormality of Rb or a molecule on the Rb pathway.

### Brief Description of the Drawings

Fig. 1 is a view showing the results of confirming the suppression of Rb expression in a cell into which an Rb shRNA was introduced.
Fig. 2 is a view showing the results of confirming the suppression of Rb expression in a cell into which an Rb shRNA was introduced by a reporter assay using the inhibition of E2F activity as an index.
Fig. 3 is a view showing the results of confirming the effect on the cell cycle when an Rb knockdown cell (a cell having only a vector introduced therein and treated with DMSO) was treated with doxorubicin.
Fig. 4 is a view showing the results of confirming the effect on the cell cycle when an Rb knockdown cell (a cell having only a vector introduced therein and treated with 10 nM doxorubicin for 24 hours) was treated with doxorubicin.
Fig. 5 is a view showing the results of confirming the effect on the cell cycle when an Rb knockdown cell (a cell having only a vector introduced therein and treated with 10 nM doxorubicin for 48 hours) was treated with doxorubicin.
Fig. 6 is a view showing the results of confirming the effect on the cell cycle when an Rb knockdown cell (a cell having an Rb shRNA introduced therein and treated with DMSO) was treated with doxorubicin.
Fig. 7 is a view showing the results of confirming the effect on the cell cycle when an Rb knockdown cell (a cell having an Rb shRNA introduced therein and treated with 10 nM doxorubicin for 24 hours) was treated with doxorubicin.
Fig. 8 is a view showing the results of confirming the effect on the cell cycle when an Rb knockdown cell (a cell having an Rb shRNA introduced therein and treated with 10 nM doxorubicin for 48 hours) was treated with doxorubicin.
Fig. 9 is a view showing the results of confirming the IL-8 expression in an Rb knockdown cell at the mRNA level.
Fig. 10 is a view showing the results of confirming the IL-8 expression in an Rb knockdown cell at the protein level.
Fig. 11 is a view showing the results of confirming various genes whose expression level varies in an Rb knockdown cell using a microarray.
Fig. 12 is a view showing a gene group whose expression level varies in an Rb knockdown cell and which regulates cell cycle progression. The upper column shows a gene group which regulates the G1/S cell cycle progression, and the lower column shows a gene group which regulates the G2/M cell cycle progression.
Fig. 13 is a view showing the results of examining the interaction between ECT2 promoter and E2F.
Fig. 14 is a view showing the results of examining the interaction between ECT2 promoter and E2F.
Fig. 15 is a view showing the results of confirming that Rb inhibits the G2/M phase progression mediated by ECT2.

### Best Mode for Carrying Out the Invention

Hereinafter, a preferred embodiment of the invention will be described in detail.

First, the terms in the invention will be described.

A gene according to the invention is selected from the group consisting of IL-8 (Accession No. NM_000584: SEQ ID NO: 1), IL-8 receptor (Accession No. NM_000634 (IL-8RA): SEQ ID NO: 2 and NM_001557 (IL-8RB): SEQ ID NO: 3), ECT2 (Accession No. NM_018098: SEQ ID NO: 4), MCM2 (Accession No. NM_004526: SEQ ID NO: 5), MCM3 (Accession No. NM_002388: SEQ ID NO: 6), MCM4 (Accession No. NM_182746: SEQ ID NO: 7), MCM5 (Accession No. NM_006739: SEQ ID NO: 8), MCM6 (Accession No. NM_005915: SEQ ID NO: 9), MCM7 (Accession No. NM_005916: SEQ ID NO: 10), CCNE2 (Accession No. NM_004702: SEQ ID NO: 11), FEN1 (Accession No. NM_004111: SEQ ID NO: 12), HMGB2 (Accession No. NM_002129: SEQ ID NO: 13), CENPE (Accession No. NM_001813: SEQ ID NO: 14), KIF20A (Accession No. NM_005733: SEQ ID NO: 15), CDC25C (Accession No. NM_001790: SEQ ID NO: 16), CDCA8 (Accession No. NM_018101: SEQ ID NO: 17), KIF11 (Accession No. NM_004523: SEQ ID NO: 18), HCAP-E (Accession No. NM_006444: SEQ ID NO: 19), HCAP-G (Accession No. NM_022346: SEQ ID NO: 20), KNTC2 (Accession No. NM_006101: SEQ ID NO: 21), HCAP-C (Accession No. NM_005496: SEQ ID NO: 22), BUB1 (Accession No. NM_004336: SEQ ID NO: 23), NEK2 (Accession No. NM_002497: SEQ ID NO: 24), CCNA2 (Accession No. NM_001237: SEQ ID NO: 25) and KIF18A (Accession No. NM_031217: SEQ ID NO: 26). The biological species from which these genes are derived is not particularly limited, and examples thereof include humans, monkeys, mice, rats, dogs and rabbits. Among them, because a subject to which a compound to be evaluated is administered is a human, they are preferably human genes.

Further, in the gene according to the invention, a gene having substitution, deletion, addition or insertion of one or more nucleotides is also included as long as it has a physiological function equivalent to that of the gene. Here, the gene is not particularly limited in terms of its sequence as long as it is a gene encoding such a protein, however, it preferably has a homology of 50% or more, more preferably 70% or more, further more preferably 80% or more, and particularly preferably 90% or more (for example, 91, 92, 93, 94, 95, 96, 97, 98, 99% or more).

Further, in the gene according to the invention, a nucleic acid which is hybridized to the gene under a stringent condition is also included. Here, the phrase "which is hybridized under a stringent condition" means that two nucleic acid fragments are hybridized to each other under the hybridization condition described in Molecular Cloning: A Laboratory Manual 2nd Edition, Cold Spring Harbor (1989) 9.47-9.62 and 11.45-11.61. To be more specific, for example, a condition in which washing is carried out at 50°C with 2.0 x SSC after hybridization is carried out at about 45°C with 6.0 x SSC can be exemplified. In order to select the stringency, the salt concentration in the washing step can be selected from, for example, about 2.0 x SSC at 50°C as a low stringency to about 0.2 x SSC at 50°C as a high stringency. Further, the temperature in the washing step can be raised from room temperature of about 22°C as a low stringency condition to about 65°C as a high stringency condition.

Further, in the protein, which is a transcription product of the gene according to the invention, a protein having substitution, deletion, addition or insertion of one or more nucleotides is also included as long as it has a physiological function equivalent to that of the protein. Here, the protein is not particularly limited in terms of its sequence, however, it preferably has a homology of 50% or more, more preferably 70% or more, further more preferably 80% or more, and particularly preferably 90% or more (for example, 91, 92, 93, 94, 95, 96, 97, 98, 99% or more).

The present inventors found that there is a correlation between the expression level of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A (hereinafter referred to as simply a "gene group" or a "protein group" in some cases) genes and the expression level of Rb. That is, Rb plays a role in regulation of expression of downstream genes mediated by E2F or the like, which is a transcription factor, and it is considered that the gene group listed above is under the control thereof and their transcription is regulated by Rb. The above-mentioned genes in the gene group are classified into any of angiogenesis-related genes, macrotubule attachment, spindle checkpoint, cytokinesis, and cell cycle-related genes, and exhibit their function by the regulation or induction of expression of Rb. Rb is known as a tumor suppressor gene, however, it is considered that Rb exhibits an ability to suppress cancer through the regulation of expression of the above-mentioned gene group, i.e., it plays a role in normal cell proliferation. Therefore, any of the genes in this gene group can be used as a tumor marker for detecting cancer caused by an abnormality of Rb or a molecule on the Rb pathway, and further can be used as a drug discovery target molecule for an anticancer agent. Here, the "abnormality of Rb or a molecule on the Rb pathway" according to the invention is considered to be those caused by deletion (for example, a nonsense mutation), substitution (for example, missense mutation, a point mutation), or insertion of a nucleotide, frame shift or the like in Rb gene or a gene (for example, E2F, CDK family or the like) on the Rb pathway. However, as a target of the invention, the cause of dysfunction is not particularly limited, and can be any of these causes.

Further, specific examples of the dysfunction of Rb protein or a protein on the Rb pathway include a case in which the transcriptional activity of these proteins is lowered or activated and a case in which transcription does not occur at all. Further a case in which a mutation is caused in a coding gene although an abnormality is not observed in the activity is also included. Specifically, for example, retinoblastoma (Birth Defects Orig. Art. Ser., Vol. 18, P. 689, 1982: New Eng. J. Med., Vol. 321, P. 1689, 1989), pinealoma (Arch. Ophthal., Vol. 102, P. 257, 1984), osteogenic sarcoma (Ophthalmologica, Vol. 175, P. 185, 1977), each of which is known to be associated with Rb, can be exemplified.

The "test tissue" in the invention is a tissue which can be extracted from the living body to be tested for cancer, and is not particularly limited in terms of its type as long as it is a cancer tissue required to be examined for the association with Rb or a tissue considered to be required for diagnosis of cancer. Examples of such a tissue include tissues derived from neuroblastoma, retinoblastoma, brain tumors, head and neck cancer, pituitary adenoma, glioblastoma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, liver cancer, pancreatic cancer, pancreatic endocrine tumors, biliary tract cancer, penile cancer, vulvar cancer, renal pelvis and ureter cancer, kidney cancer, testicular cancer, prostate cancer, bladder cancer, uterine cancer, trophoblastic disease, vaginal cancer, ovarian cancer, fallopian tube cancer, ovarian germ cell tumors, skin cancer, mycosis fungoides, malignant melanoma, soft tissue sarcoma, bone neoplasm, malignant lymphoma, leukemia, myelodysplastic syndrome, multiple myeloma and lymphedema.

Similarly, the "test cell" in the invention is a tissue which can be extracted from the living body to be tested for cancer, and is not particularly limited in terms of its type as long as it is a cell derived from a cancer tissue required to be examined for the association with Rb or a cell derived from a tissue considered to be required for diagnosis of cancer. Examples of such a cell include cells derived from neuroblastoma, retinoblastoma, brain tumors, head and neck cancer, pituitary adenoma, glioblastoma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, liver cancer, pancreatic cancer, pancreatic endocrine tumors, biliary tract cancer, penile cancer, vulvar cancer, renal pelvis and ureter cancer, kidney cancer, testicular cancer, prostate cancer, bladder cancer, uterine cancer, trophoblastic disease, vaginal cancer, ovarian cancer, fallopian tube cancer, ovarian germ cell tumors, skin cancer, mycosis fungoides, malignant melanoma, soft tissue sarcoma, bone neoplasm, malignant lymphoma, leukemia, myelodysplastic syndrome, multiple myeloma and lymphedema.

### (1) Evaluation of compound

By using IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 or KIF18A gene or protein, a compound that acts on any of these genes or proteins in the gene group or protein group can be evaluated. Examples of the method of detecting an action against any of these genes or proteins in the gene group or protein group include a method of detecting a specific binding (for example, a binding causing inhibition of an enzymatic activity) of a test compound to a protein belonging to the protein group, a method of detecting the expression level of a gene which is changed by the contact with a test compound and a method of detecting the activity of intracellular signal transduction induced by the contact. Hereinafter, these methods will be described in turn.

First, the method for evaluating a test compound by detecting a specific binding of a test compound to a protein belonging to the protein group will be described.

The method for evaluating a compound of the invention is characterized by comprising the steps of: introducing any one gene belonging to the gene group thereby to prepare a cell expressing the gene; bringing a test compound into contact with the cell; and detecting a specific binding of the test compound to the gene.

Further, the second method for evaluating a compound of the invention is characterized by comprising the steps of: introducing any one gene belonging to the gene group thereby to prepare a cell expressing the gene; bringing a test compound into contact with the cell; measuring the activity of an intracellular signal transducer induced by the contact; and comparing the above-mentioned activity with the activity of the intracellular signal transducer in the absence of contact with the test compound.

The test compound is not particularly limited, and examples thereof include single compounds such as natural compounds, organic compounds, inorganic compounds, proteins and peptides, and compound libraries, expression products of gene libraries, cell extracts, cell culture supernatants, fermentation products of microorganisms, marine organism extracts, plant extracts, prokaryotic cell extracts, eukaryotic single cell extracts, animal cell extracts and the like. The above-mentioned test sample can be used by appropriately labeling if necessary. As the labeling, for example, radiolabeling, fluorescent labeling and the like can be exemplified. Further, in addition to the above-mentioned test samples, a mixture obtained by mixing plural types of these test samples is also included.

Further, the cell expressing the gene can be prepared by a method known to a person skilled in the art. A specific method is not particularly limited, and for example, the following method can be employed. That is, it is prepared by cloning the gene or a nucleic acid consisting of a part thereof into an expression vector containing a suitable promoter and a transcriptional regulatory element, and introducing the vector with the cloned nucleic acid into a host cell. Here, the vector is not particularly limited as long as it is a vector that can be used as an expression vector, and examples thereof include pCMV-Tag, pcDNA3.1, pBlueBacHis2, pCI-neo, pcDNAI, pMC1neo, pXT1, pSG5, pEF1/V5-HisB, pCR2.1, pET11, λgt1 and pCR3.1.

Subsequently, the expression vector into which the gene or the nucleic acid consisting of a part thereof has been introduced is transfected into a host cell. Such a host cell is not particularly limited as long as it is a host cell commonly used in the expression of a gene, and may be any of an animal cell, an insect cell, a plant cell, and a microorganism. Specific examples thereof include COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and specific examples thereof include electroporation, the calcium phosphate method, the DEAE-dextran method, the lipofection method and a gene gun.

Subsequently, a test compound is brought into contact with the thus prepared cell expressing the gene. The method of contact is not particularly limited, and for example, if any one protein belonging to the protein group is in a purified state, the contact can be carried out by adding a test sample to a purified preparation. Further, if it is in a state of being expressed in a cell or in a state of being expressed in a cell extract, the contact can be carried out by adding a test sample to a cell culture solution or the cell extract, respectively. In the case where a test sample is a protein, for example, a vector containing a DNA encoding the protein is transfected into a cell expressing the above-mentioned protein. Alternatively, the contact can also be carried out by adding the vector to a cell extract in which the protein is expressed.

As for the binding of the protein to a test compound, when the protein is a receptor protein such as IL-8 receptor, a method in which the detection is carried out by, for example, a label attached to the bound compound (for example, detection of binding amount by radioactivity or fluorescence intensity) can be employed. Further, when any one protein belonging to the protein group is a molecule involved in intracellular signal transduction, also the expression level or activity of a molecule (including also the protein) on the signal transduction pathway caused by the signal transduction can be used as an index. Here, in the case where the expression level is used as an index, the method of measuring the expression level is not particularly limited, and examples thereof include Northern blotting, Western blotting and a DNA chip. Here, the "expression level" in the invention refers to the absolute amount or relative amount of a transcription product of a gene encoding a protein in the signal transduction pathway mediated by the protein. In this case, in the gene, both DNA and mRNA are included. Further, in the case where the detection target for expression is a protein, the "expression level" refers to the absolute amount or relative amount of a translation product of a protein in the signal transduction pathway mediated by the protein. Further, in the case where the activity of a molecule involved in signal transduction is used as an index, the method of measuring the activity is not particularly limited, and a suitable method may be selected according to the type of a molecule to be measured.

On the other hand, an isolated protein can also be used directly for evaluation of a compound. That is, it is a method comprising bringing a test compound into contact with a protein and detecting a change in the activity of the protein caused by the contact.

The method of such contact is not particularly limited, and specific examples thereof include a method in which the contact is achieved by mixing in a solution such as a buffer solution (a phosphate buffer solution or the like), and a method in which a protein is immobilized on a membrane and the protein is brought into contact with a test compound on the membrane.

Subsequently, a change in the activity of the protein caused by the contact is detected.

As the method of measuring the activity of the protein, a desired method may be selected according to the property of the protein to be used. For example, in the case of IL-8, a method in which the binding activity to IL-8 receptor is detected can be employed, or detection can be carried out by a neutrophil migration assay (Cytokine Experiment Methods, pp. 87-93, 1997, Yodosha Co., Ltd.). In the case of ECT2, because ECT2 increases a GTP-bound form of RhoA or CDC42, it can be evaluated that a test compound inhibits the binding activity of ECT2 through binding to ECT2 by measuring the activity of RhoA. Further, by utilizing the phenomenon that when the activity of ECT2 is inhibited, cell cycle arrest in mitosis is caused, the inhibition of ECT2 can be evaluated by a mitosis arrest assay.

Which method for evaluating a compound of the invention can be applied to the genes belonging to the gene group according to the invention is shown in Table 1. In Table 1, the "type" shows the function of the gene in the living body, the "binding" shows the method for evaluating a compound by a binding of a test compound to a molecule among the methods for evaluating a compound of the invention, the "signal" shows the method for evaluating a compound by using the intracellular signal transduction induced by the contact with a test compound as an index among the methods for evaluating a compound of the invention, and the "activity" shows the method for evaluating a compound by using the activity caused by the contact of a protein molecule with a test compound as an index.

**(Table 1)**

| Gene | Type | Evaluation of compound | | |
|---|---|---|---|---|
| | | Binding | Signal transduction | Activity |
| IL-8 | Secretory protein | ○ | ○ | ○ |
| IL-8 receptor | Receptor | ○ | ○ | ○ |
| ECT2 | GDP/GTP exchange factor | ○ | ○ | ○ |
| MCM2 | DNA binding | × | ○ | × |
| MCM3 | DNA binding | × | ○ | × |
| MCM4 | DNA binding | × | ○ | × |
| MCM5 | DNA binding | × | ○ | × |
| MCM6 | DNA binding | × | ○ | × |
| MCM7 | DNA binding | × | ○ | × |
| CCNE2 | Cyclin | ○ | ○ | ○ |
| FEN1 | Nuclease | ○ | ○ | ○ |
| HMGB2 | DNA binding | × | ○ | × |
| CENPE | Motor protein | × | ○ | ○ |
| KIF20A | Motor protein | ○ | ○ | ○ |
| CDC25C | Phosphatase | ○ | ○ | ○ |
| CDCA8 | Microtubule attachment | × | ○ | ○ |
| KIF11 | Motor protein | ○ | ○ | ○ |
| HCAP-E | Condensin | × | ○ | × |
| HCAP-G | Condensin | × | ○ | × |
| KNTC2 | Microtubule attachment | × | ○ | ○ |
| HCAP-C | Condensin | × | ○ | × |
| BUB1 | Kinase | ○ | ○ | ○ |
| NEK2 | Kinase | ○ | ○ | ○ |
| CCNA2 | Cyclin | ○ | ○ | ○ |
| KIF18A | Motor protein | ○ | ○ | ○ |

As described above, in the case where as a result of evaluating a compound by the method for evaluating a compound of the invention, the activity of the protein in the presence of a test compound shows a lower value than the binding activity in the absence of the test compound (control), the test compound is judged to be an antagonist having an activity of inhibiting the binding between the protein according to the invention and the ligand. The antagonist suppresses the physiological activity of the ligand and its analogs for the protein. Such an antagonist may possibly suppress the abnormal proliferation of cells caused by dysfunction of Rb or a molecule on the Rb pathway, therefore, it is useful as a pharmaceutical composition for the treatment or the like of cancer caused by an abnormality in a signal transduction pathway mediated by Rb.

Further, by the method for evaluating a compound of the invention, screening of a substance that promotes or inhibits signal transduction or transcriptional regulation in the Rb pathway. That is, by evaluating multiple test compounds by the above-mentioned method, a compound that functions as an agonist or an antagonist can be selected. As a result of such selection, if in comparison with the change in signal transduction to downstream in the case where the ligand or its analog is allowed to act in the absence of the test compound, the change is suppressed, the test compound is judged to be a compound that inhibits the signal transduction to the downstream of any one of the proteins belonging to the protein group. On the contrary, if the test compound enhances intracellular signal transduction, the compound is judged to be a compound that promotes the signal transduction after binding of the test compound to the protein. A compound selected by such a screening method is effective in the treatment and diagnosis of cancer caused by dysfunction of Rb or a molecule on the Rb pathway.

Further, by the above-mentioned method for evaluating a compound of the invention, a ligand to be used in PET (positron emission tomography) can be evaluated. PET is a method capable of noninvasively observing a biological function by radiolabeling a ligand for a substance present in the living body such as water, oxygen, glucose or an amino acid, or a desired receptor and administering the ligand in the body, and it is used in studies and clinical practice. PET is characterized in that it enables imaging specific to a function depending on the ligand to be used as a tracer, and the development of a new tracer is essential for elucidation of unknown biological functions or diagnosis of diseases. According to the method for evaluating a compound of the invention, by applying a PET ligand candidate substance as a test compound, in vitro evaluation of the substance can be realized.

### (2) Compound obtained by method for evaluating compound

Further, a compound obtained by the method for evaluating a compound of the invention is also included in the invention. Such a compound is not particularly limited in terms of its property, and examples thereof include single compounds such as natural compounds, organic compounds, inorganic compounds, proteins and peptides, antibodies, antisense, RNAi and ribozymes.

In the case where a compound obtained by the method for evaluating a compound of the invention is used as a medicine for humans or other animals, it is possible to administer any of these substances by formulating it into a preparation by a known pharmaceutical method other than the direct administration of the substance as such to a patient. For example, it can be used orally as a tablet, if necessary coated with a sugar, a capsule, an elixir or a microcapsule, or parenterally in the form of an injection of a sterile solution or a suspension with water or a pharmaceutically acceptable liquid other than water. For example, it is possible to formulate the substance into a preparation by appropriately combining a pharmacologically acceptable carrier or medium, specifically sterile water, physiological saline, a vegetable oil, an emulsifier, a suspending agent, a surfactant, a stabilizer, a flavoring agent, an excipient, a vehicle, a preservative, a binder or the like, and mixing them in a unit dosage form as required by generally admitted pharmaceutical practice.

As an additive which can be mixed in a tablet or a capsule, a binder such as gelatin, cornstarch, tragacanth gum or gum arabic, an excipient such as crystalline cellulose, a swelling agent such as cornstarch, gelatin or alginic acid, a lubricant such as magnesium stearate, a sweetener such as sucrose, lactose or saccharine, or a flavoring agent such as peppermint, acamono oil or cherry is used. In the case where the preparation unit is in the capsule form, a liquid carrier such as fat and oil can further be included in the above-mentioned materials. A sterile composition for injection can be formulated according to usual pharmaceutical practice using a vehicle such as distilled water for injection.

Examples of an aqueous solution for injection include physiological saline and isotonic solutions containing glucose or other adjuvants (for example, D-sorbitol, D-mannose, D-mannitol and sodium chloride), and may be used in combination with an appropriate solubilizing agent such as an alcohol, specifically ethanol, a polyalcohol such as propylene glycol or polyethylene glycol or a nonionic surfactant such as Polysorbate 80 (TM) or HCO-50.

Examples of an oily liquid include sesame oil and soybean oil, and may be used in combination with a solubilizing agent such as benzyl benzoate or benzyl alcohol. In addition, it may be further mixed with a buffer such as a phosphate buffer or a sodium acetate buffer, a soothing agent such as procaine hydrochloride, a stabilizer such as benzyl alcohol or phenol, or an antioxidant. The thus prepared injectable solution is usually filled into an appropriate ampoule.

The administration thereof to a patient can be carried out intranasally, transbronchially, intramuscularly, percutaneously or orally by a method known to a person skilled in the art as well as by way of, for example, intraarterial injection, intravenous injection, subcutaneous injection or the like. The dose varies depending on the body weight or age of a patient, an administration route or the like, however, a person skilled in the art can appropriately select a suitable dose. Further, if the compound can be one encoded by DNA, it is also possible to carry out gene therapy by integrating the DNA into a vector for gene therapy. The dose and administration route vary depending on the body weight, age or symptom of a patient or the like, however, a person skilled in the art can appropriately select them.

The dose of the compound varies depending on the symptom, however, in the case of oral administration, the dose thereof is considered to be about 0.1 to 100 mg per day, preferably about 1.0 to 50 mg per day, more preferably about 1.0 to 20 mg per day generally for an adult (assuming of 60 kg of body weight).

In the case of parenteral administration, a single dose thereof varies depending on the subject to be administered, target organ, symptom, or administration route. However, in the case of, for example, an injection form, it is considered to be convenient to administer the compound in an amount of generally about 0.01 to 30 mg per day, preferably about 0.1 to 20 mg per day, more preferably about 0.1 to about 10 mg per day by intravenous injection generally for an adult (assuming of 60 kg of body weight).

### (3) Molecular diagnostic method for cancer

A first embodiment of the molecular diagnostic method for cancer of the invention is characterized by comprising the steps of: measuring the expression level of any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A in a test tissue or a test cell; comparing the above-mentioned expression level of the gene with the expression level of a corresponding gene in a normal tissue or a normal cell; and determining whether or not the expression level of the gene in the test tissue or test cell is significantly higher than the expression level of the gene in the normal tissue or normal cell based on the comparison result.

Further, a second embodiment of the molecular diagnostic method for cancer of the invention is characterized by comprising the steps of: measuring the expression level of any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A in a test tissue or a test cell; comparing the above-mentioned expression level of the protein with the expression level of a corresponding protein in a normal tissue or a normal cell; and determining whether or not the expression level of the protein in the test tissue or test cell is significantly higher than the expression level of the protein in the normal tissue or normal cell based on the comparison result.

In the invention, first, the expression level of any one gene belonging to the gene group or a protein, which is a transcription product of the gene, in a test tissue or a test cell is measured. The method of measuring the expression level of a gene or a protein is not particularly limited. In the case of a gene, for example, a method in which RT-PCR is carried out by using cDNA extracted from a test tissue or a test cell as a template, a method in which a microarray having the gene plotted therein is used, and Northern blotting can be exemplified. Further, in the case of a protein, for example, a method in which the concentration of the protein contained in the blood collected from a test subject is measured can be exemplified. In the gene or protein group according to the invention, IL-8 which is a secretory protein, IL-8 which is a receptor protein on the cell membrane, ECT2 which is an intracellular signal transduction molecule and the like are included, therefore, a suitable method may be appropriately selected according to the type of molecule thereof.

Here, the "expression level" of gene or protein refers to the absolute amount or relative amount of a gene transcription product or a protein. In the case of a relative amount, the expression level of the gene may be determined in relative comparison with the expression level in a normal tissue described below.

Subsequently, the expression level of the gene measured by the above-mentioned method is compared with the expression level of a corresponding gene in a normal tissue or a normal cell.

Here, the "normal tissue or normal cell" is not particularly limited in terms of its origin as long as it is a tissue or a cell to become a target for comparison with the test tissue or test cell, and it may be derived from either a normal subject or a patient with cancer. Further, it may be a normal tissue or a normal cell present in the vicinity of a cancer tissue.

In this step, the expression level of a target gene expressed in a test tissue or cell is compared with that of a gene (corresponding gene) expressed in a normal tissue or cell, and this comparison can be carried out with the absolute amounts of the expression levels, and also relative values can be calculated by the comparison.

Subsequently, based on the comparison result, it is determined whether or not the expression level of the gene in the test tissue or test cell is significantly higher than the expression level of the gene in the normal tissue or normal cell. The method of determining the significant difference is not particularly limited, and an assay may be carried out using a statistical method known to a person skilled in the art.

In the case where the expression in the test tissue or test cell is judged to be higher or lower with a significant difference as a result of the comparison of the expression level of the gene in the test tissue or test cell with that of the gene in the normal tissue or normal cell, an abnormality occurs in the expression of Rb in the test tissue or test cell and it can be judged that there is a possibility that malignant transformation has been caused. Therefore, diagnosis of cancer known to be associated with Rb can be realized.

### (4) Tumor marker

A first embodiment of the tumor marker for detecting cancer having an abnormality of Rb or a molecule on the Rb pathway of the invention is characterized by containing any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A.

Further, a second embodiment of the tumor marker for detecting cancer having an abnormality of Rb or a molecule on the Rb pathway of the invention is characterized by containing any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A.

A tumor marker is a substance that is produced in a large amount by a tumor cell, is not detected usually and is called also a tumor-associated antigen. Further, an autoantibody against the antigen is produced only when the antigen is produced in a similar manner, therefore, it can also be a tumor marker. Other than these, a tumorigenic cell sometimes produces a large amount of a hormone, an enzyme, a specific low molecular weight compound or the like, and any of such substances is useful for detecting the presence of cancer or tumor. The gene belonging to the gene group according to the invention is a gene whose expression increases when the expression of Rb gene is suppressed, and can be used as a tumor marker.

In an application embodiment as a tumor marker, first, the expression level of any one gene belonging to the gene group or a protein, which is a transcription product of the gene, in a test tissue or a test cell is measured. The method of measuring the expression level of a gene or a protein is not particularly limited. In the case of a gene, for example, a method in which RT-PCR is carried out using cDNA extracted from a test tissue or a test cell as a template, a method in which a microarray having the gene plotted therein is used, and Northern blotting can be exemplified. Further, in the case of a protein, for example, a method in which the concentration of the protein contained in the blood collected from a test subject is measured can be exemplified. In the gene or protein group according to the invention, IL-8 which is a secretory protein, IL-8 which is a receptor protein on the cell membrane, ECT2 which is an intracellular signal transduction molecule and the like are included, therefore, a suitable method may be appropriately selected according to the type of molecule thereof. Here, any one type of genes belonging to the gene group or proteins may be used as a detection target, however, two or more types thereof may be used as detection targets. By using two or more types of molecules as detection targets, more accurate diagnosis can be realized.

Subsequently, the expression level of the gene measured by the above-mentioned method is compared with the expression level of a corresponding gene in a normal tissue or a normal cell. Here, the "normal tissue or normal cell" is not particularly limited in terms of its origin as long as it is a tissue or a cell to become a target for comparison with the test tissue or test cell.

Further, when the expression level of a target gene expressed in a test tissue or cell is compared with that of a gene (corresponding gene) expressed in a normal tissue or cell, the comparison can be carried out with the absolute amounts of the expression levels, and also relative values can be calculated by the comparison.

Subsequently, based on the comparison result, it is determined whether or not the expression level of the gene in the test tissue or test cell is significantly higher than the expression level of the gene in the normal tissue or normal cell. The method of determining the significant difference is not particularly limited, and an assay may be carried out using a statistical method known to a person skilled in the art.

In the case where the expression in the test tissue or test cell is judged to be higher or lower with a significant difference as a result of the comparison of the expression level of the gene in the test tissue or test cell with that of the gene in the normal tissue or normal cell, an abnormality occurs in the expression of Rb in the test tissue or test cell and it can be judged that there is a possibility that malignant transformation has been caused. Therefore, diagnose of cancer known to be associated with Rb can be realized.

In the case where the expression level of the gene in a test tissue or a test cell is significantly high, or in the case where the expression level of the gene in a normal tissue is known, it is not necessary to perform the comparison with the expression level in a normal tissue or a normal cell every time it is used as a tumor marker, and also judgment of tumor can be carried out by measuring only the expression level thereof in a test tissue or a test cell.

The tumor to become a detection target of the tumor marker of the invention is not particularly limited as long as it is a tumor caused by Rb or a molecule on the Rb pathway, and specific examples thereof include retinoblastoma, pinealoma and osteogenic sarcoma.

### (5) Method for detecting target gene for anticancer agent

The detection method of the invention is characterized by comprising the steps of:
preparing an Rb expression-suppressed cell in which the expression of Rb has been suppressed by RNA interference using an Rb-expressing cell; measuring the expression level of a desired gene in the cell; and
comparing the above-mentioned expression level with the expression level of the gene in the Rb-expressing cell.

The "RNA interference" refers to a phenomenon in which a double-stranded RNA introduced into a cell suppresses the expression of a gene having the same sequence as that of the double-stranded RNA thereby to suppress the synthesis of a protein encoded by the gene. In the detection method of the invention, first, RNA interference is induced by using an siRNA targeting Rb, whereby a cell in which the expression of Rb has been suppressed is prepared. The preparation method is not particularly limited, and the preparation can be carried out by a method known to a person skilled in the art. Here, the siRNA used is not particularly limited in terms of its sequence as long as it is a sequence capable of suppressing the expression of Rb, however, it is preferably a sequence specific to Rb. Further, it preferably has a nucleotide length of 21 to 27 nucleotides, more preferably 21 to 23 nucleotides.

Further, it is known that the effect of introduction of siRNA lasts several days to about one week, however, the present inventors have succeeded in constantly suppressing the expression of Rb by introducing an siRNA into a cell by using a specific expression vector. In the detection method of the invention, the method of introducing an siRNA is not particularly limited as long as the expression of Rb is suppressed by RNA interference. However, it is preferably through gene introduction using a vector with which the expression of Rb is constantly suppressed. Here, examples of the vector used include pSuperior-puro, pENTR/U6 and pENTR/H1/TO.

In the thus prepared Rb expression-suppressed cell, the expression and function of Rb, which is a cancer suppressor gene, have been suppressed.

Subsequently, the expression level of a desired gene in the Rb expression-suppressed cell and the Rb-expressing cell is measured. The "desired gene" refers to a candidate gene targeting an anticancer agent, and in this step, a specific candidate gene may be sequentially selected, or comprehensive examination may be carried out by using a DNA chip in which candidate genes have been plotted.

Further, the method of measuring the expression level is not particularly limited, and specific examples thereof include a DNA chip, Northern blotting and PCR.

Subsequently, the expression levels of the gene in the Rb expression-suppressed cell and the Rb-expressing cell measured in the previous step are compared. A gene whose expression levels are different between the both cells as a result of the comparison is a molecule under the influence of the expression level or activity of Rb in vivo, that is, a molecule on the signal transduction pathway mediated by Rb. Such a molecule (a gene or a protein) is a molecule closely associated with the malignant transformation of cells, that is, the proliferation of cells or cell cycle, and there is a possibility that the malignant transformation can be suppressed by suppressing or enhancing the function of the molecule. That is, by targeting such a molecule for an anticancer agent, it becomes possible to develop a therapeutic agent that suppresses cancer or tumor caused by an abnormality of Rb or a molecule on the Rb pathway.

### (6) Method for evaluating compound using Rb expression-suppressed cell

A first embodiment of the method for evaluating a compound using an Rb expression-suppressed cell of the invention is characterized by comprising the steps of: preparing an Rb expression-suppressed cell in which the expression of Rb has been suppressed by RNA interference using an Rb-expressing cell; bringing a test compound into contact with the Rb expression-suppressed cell and the Rb-expressing cell; measuring the activity of an intracellular signal transducer induced by the contact; and comparing the activity in the Rb expression-suppressed cell with the activity in the Rb-expressing cell.

Further, a second embodiment of the method for evaluating a compound using an Rb expression-suppressed cell of the invention is characterized by comprising the steps of: preparing an Rb expression-suppressed cell in which the expression of Rb has been suppressed by RNA interference using an Rb-expressing cell; bringing a test compound into contact with the Rb expression-suppressed cell and the Rb-expressing cell; measuring the expression level of an intracellular signal transducer induced by the contact; and comparing the expression level in the Rb expression-suppressed cell with the expression level in the Rb-expressing cell.

In the method for evaluating a compound using an Rb expression-suppressed cell of the invention, first, an Rb expression-suppressed cell in which the expression of Rb has been suppressed by RNA interference is prepared by using an Rb-expressing cell. The preparation method is not particularly limited, and the preparation can be carried out by a method known to a person skilled in the art. Here, the siRNA used is not particularly limited as long as it is a sequence capable of suppressing the expression of Rb, however, it is preferably a sequence specific to Rb. Further, it preferably has a nucleotide length of 21 to 27 nucleotides, more preferably 21 to 23 nucleotides.

Further, it is known that the effect of introduction of siRNA lasts several days to about one week, however, the present inventors have succeeded in constantly suppressing the expression of Rb by introducing an siRNA into a cell by using a specific expression vector. In the detection method of the invention, the method of introducing an siRNA is not particularly limited as long as the expression of Rb is suppressed by RNA interference. However, it is preferably through gene introduction using a vector with which the expression of Rb is constantly suppressed. Here, examples of the vector used include pSuperior-puro, pENTR/U6 and pENTR/H1/TO.

In the thus prepared Rb expression-suppressed cell, the expression and function of Rb, which is a cancer suppressor gene, are suppressed.

Subsequently, a test compound is brought into contact with the Rb expression-suppressed cell and the Rb-expressing cell. The method of contact is not particularly limited, and for example, the contact can be carried out by adding a test sample to a cell culture solution or a cell extract followed by mixing.

Subsequently, in the first embodiment of the method for evaluating a compound using an Rb expression-suppressed cell of the invention, the activity of an intracellular signal transducer induced by the contact is measured. By bringing the test compound into contact with the cell, a signal enters the cell. In this step, the activity of the intracellular signal transducer is measured in both the Rb expression-suppressed cell and the Rb-expressing cell, and it is examined whether or not there is a difference in the activities based on the presence or absence of the expression of Rb, or high or low expression of Rb. As the method of measuring the activity, a suitable method may be appropriately selected according to a substance to be measured. For example, if a substance to be measured is a phosphokinase, the activity can be measured by a kinase assay.

Further, in the second embodiment of the method for evaluating a compound using an Rb expression-suppressed cell of the invention, the expression level of an intracellular signal transducer induced by the contact is measured. That is, the expression level of the intracellular signal transducer is measured in both the Rb expression-suppressed cell and the Rb-expressing cell. Also, the method of measuring the expression level is not particularly limited, and for example, the measurement can be carried out by using Northern blotting, PCR or a DNA chip.

Subsequently, in the first embodiment of the method for evaluating a compound using an Rb expression-suppressed cell of the invention, the activity of the intracellular signal transducer in the Rb expression-suppressed cell is compared with that in the Rb-expressing cell. In the case where the activity in the Rb expression-suppressed cell is similar to the activity in the Rb-expressing cell as a result of the comparison of activities, the test compound can be judged to be a compound that suppresses malignant transformation of cells and contributes to the signal transduction mediated by normal Rb.

### (Examples)

Hereinafter, the present invention will be described further specifically with reference to Examples, however, the invention is not limited to the following Examples.

### (Establishment of Rb knockdown cell and evaluation of cell)

In order to elucidate a new pathway regulated by Rb, it was attempted to establish a pair of cells in which only the status of Rb gene is different while having the same genetic background. To be more specific, U-2 OS shRb cell was established by introducing pSuperior-puro.shRb vector into U-2 OS cell with wild-type Rb as follows.

### (Construction of shRNA vector for Rb knockdown)

An shRNA vector for Rb knockdown was prepared by inserting the following oligo DNA: 5'-GCAGTTGACCTAGATGAGATTCAAGAGATCTCATCTAGGTCAACTGC-3' (SEQ ID NO: 27) into pSuperior-puro. (OligoEngine).

### (Production of Rb knockdown cell)

Into U-2 OS cells with wild-type Rb, pSuperior-puro.shRb vector was transfected using Funege 6. After the transfected cells were cultured for 24 hours, the cells were cultured such that the number of cells became about one fiftieth, and then, the cells were cultured for about two weeks in the presence of 1.0 µg/mL of puromycin. A cell that formed a colony was isolated by a cylinder cloning method. Hereinafter, the established Rb knockdown cell was referred to as U-2 OS shRb.

### (Confirmation of Rb knockdown by the expression level of mRNA)

RNA was extracted from the U-2 OS cell and U-2 OS shRb cell using the RNeasy kit (Qiagen). By using the extracted RNA, cDNA was prepared with the TaqMan Reverse Transcription Reagents (Applied Biosystems). By using the prepared cDNA as a template, TaqMan RT-PCR was carried out, whereby the mRNA level of Rb in the respective cells was determined.

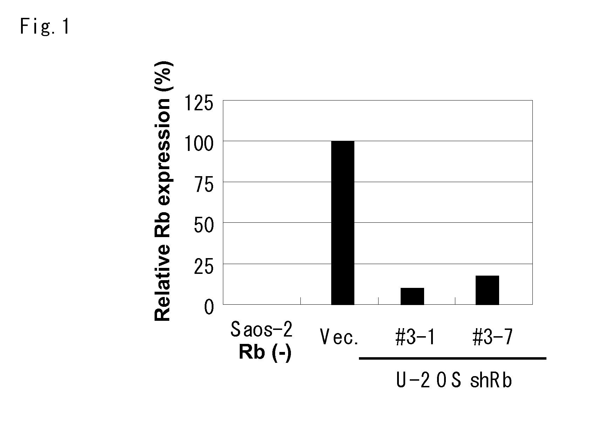

As shown in Fig. 1, it was confirmed that the expression of Rb in the established cell was suppressed by 90% or more compared with the control into which only a vector was introduced.

### (Confirmation of Rb knockdown utilizing E2F reporter assay)

Into the U-2 OS cell and U-2 OS shRb cell, a reporter plasmid under the transcriptional regulation of Rb/E2F pathway and p16 expression plasmid were transfected using Funege 6, and after 48 hours, the activity of SEAP, which is a reporter gene, was measured using the Reporter Assay Kit -SEAP-(TOYOBO). That is, Rb knockdown was confirmed by utilizing the phenomena that the reporter activity is suppressed in Rb+ cell, but it is not suppressed in Rb- cell. The reporter activity was corrected by luciferase.

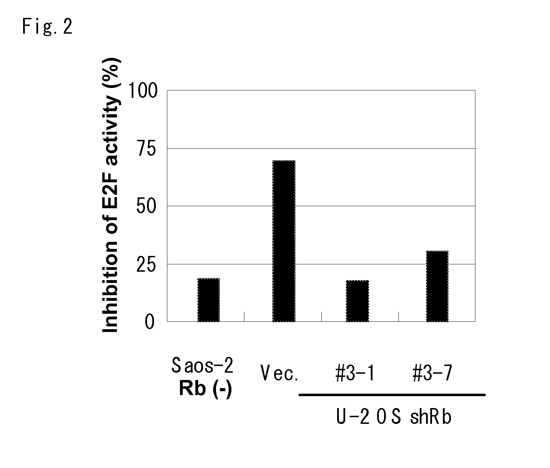

As shown in Fig. 2, while the reporter activity was suppressed in the control cell of Rb+, the reporter activity was not suppressed in the U-2 OS shRb. This result is equivalent to that of Saos-2 cell which was used as a control of Rb-.

### (Effect of doxorubicin (DNA damaging agent) on Rb knockdown cell)

As the function of Rb, it is known that Rb regulates the cell cycle progression properly. Accordingly, the effect of Rb knockdown on cell cycle was examined.

First, the U-2 OS cell and U-2 OS shRb cell were treated with 10 nM doxorubicin. The cells were collected after 24 hours and 48 hours, and the DNA of the cells were stained with the Cycle TESTTM PLUS DNA Reagent Kit (Becton Dickinson), and an analysis was carried out by flow cytometry.

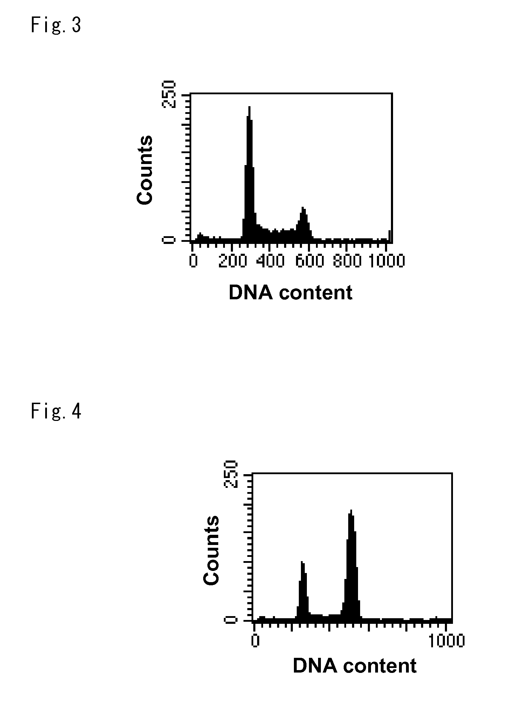

As shown in Fig. 3 to Fig. 8, at 24 hours after the treatment, the both cells were arrested in the G2/M phase. However, at 48 hours after the treatment, while U-2 OS was arrested in the G2/M phase, G2/M phase arrest of U-2 OS shRb was not maintained. From this, it was considered that in the U-2 OS shRb cell, an abnormality occurred in the function of regulating the G2/M phase progression by the inactivation of the function of Rb.

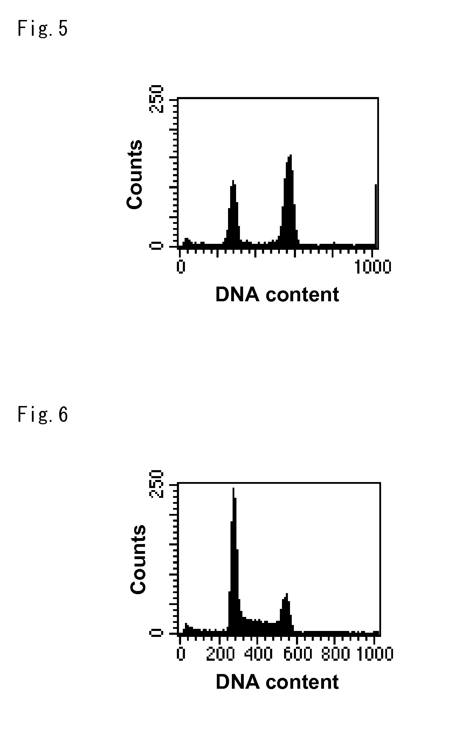

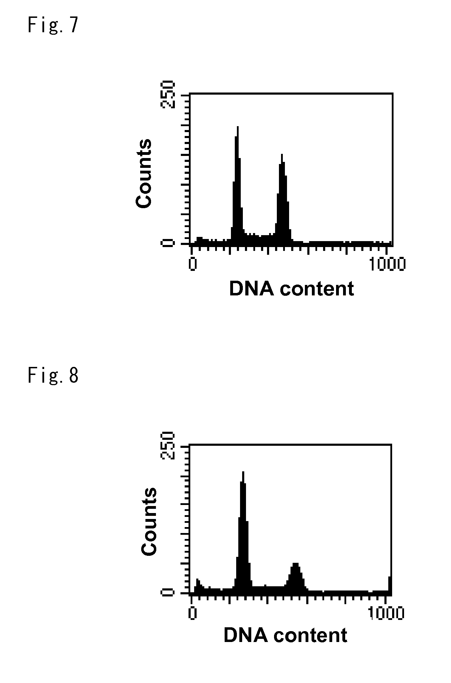

In this connection, Fig. 3 shows the test results using a cell having only a vector introduced therein and treated with DMSO; Fig. 4 shows the test results using a cell having only a vector introduced therein and treated with 10 nM doxorubicin for 24 hours; Fig. 5 shows the test results using a cell having only a vector introduced therein and treated with 10 nM doxorubicin for 48 hours; Fig. 6 shows the test results using a cell having an Rb shRNA introduced therein and treated with DMSO; Fig. 7 shows the test results using a cell having an Rb shRNA introduced therein and treated with 10 nM doxorubicin for 24 hours; and Fig. 8 shows the test results using a cell having an Rb shRNA introduced therein and treated with 10 nM doxorubicin for 48 hours. Further, in what phase the respective cells were maintained in the cell cycle under the respective conditions is shown in Table 2.

**(Table 2)**

| Figure No. | G1 (%) | S (%) | G2/M (%) |
|---|---|---|---|
| 3 | 38 | 45 | 17 |
| 4 | 20 | 11 | 68 |
| 5 | 29 | 5.4 | 66 |
| 6 | 38 | 40 | 22 |
| 7 | 35 | 19 | 45 |
| 8 | 62 | 16 | 23 |

### (Analysis of expression profile in Rb knockdown cell)

In order to analyze the mechanism of malignant transformation due to an abnormality of Rb, the expression profile in the U-2 OS cell was compared with that in the U-2 OS shRb cell using a microarray.

To be more specific, RNA was extracted from the U-2 OS cell and U-2 OS shRb cell in the logarithmic growth phase using the RNeasy kit (Qiagen). Also, RNA was extracted from both cells after treated with 10 nM doxorubicin for 24 hours in the same manner as above. After reverse transcription of the extracted RNA was carried out, an analysis was carried out using the Microarray Analysis Suite (Affymetrix).

As a result, the expression of interleukin 8 (IL8), which is an angiogenesis-related gene increased by 7 fold or more in the U-2 OS shRb cell.

Further, by the analysis of the established cell, an abnormality was found to occur in the function of regulating the G2/M phase progression in the U-2 OS shRb cell. Thus, it was attempted to elucidate the mechanism of G2/M phase progression by Rb. That is, the expression profiles in both cells after treated with 10 nM doxorubicin for 24 hours were compared using a microarray.

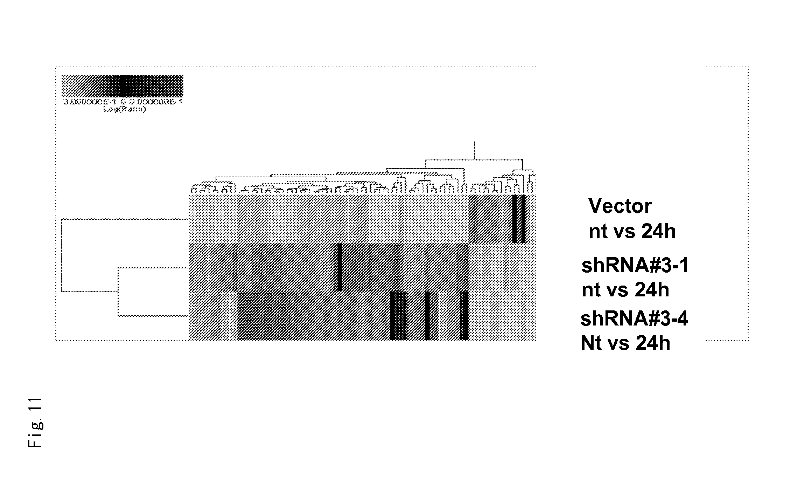

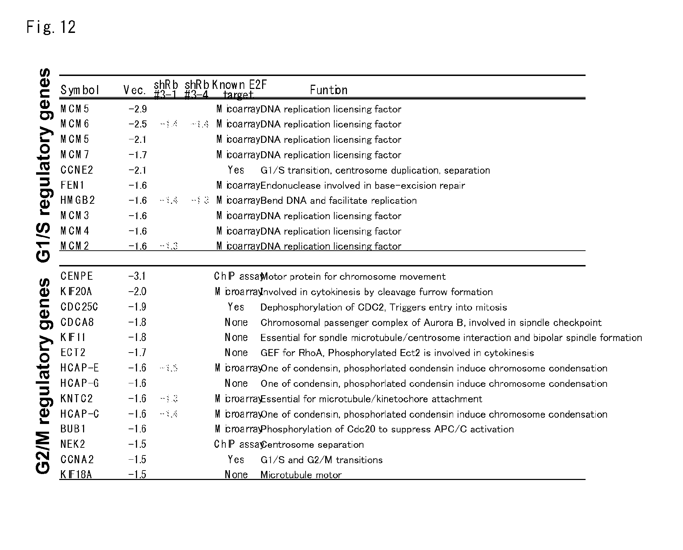

As a result, as shown in Fig. 11, a gene group whose expression decreases in the U-2 OS cell, but does not decrease in the U-2 OS shRb cell was found. As shown in Fig. 12, these genes are composed of a gene group that regulates the G1/S progression and a gene group that regulates the G2/M progression. In these genes, KIF11 involved in microtubule attachment, HCAP-G involved in chromosome condensation, CDCA8 involved in spindle checkpoint, ECT2 and KIF20A involved in cytokinesis, all of which had not been reported to be regulated by Rb, were included. This revealed that Rb regulates the M phase progression by regulating the expression of these molecules. Further, since it is also known that ECT2 is a cancer gene, a new mechanism of malignant transformation caused by an abnormality of Rb was elucidated.

### (Analysis of gene (IL8) in which a change in the expression was observed)

### 1. Determination of IL8 at the mRNA level

RNA was extracted from the U-2 OS cell and U-2 OS shRb cell using the RNeasy kit. By using the extracted RNA, cDNA was prepared with the TaqMan Reverse Transcription Reagents (Applied Biosystems). By using the prepared cDNA as a template, TaqMan RT-PCR was carried out, whereby the mRNA level of IL8 in the respective cells was determined.

### 2. Determination of IL8 at the protein level

Inoculation of each of the U-2 OS cell and U-2 OS shRb cell was carried out such that the inoculated cell number coincided with each other. At 72 hours after the inoculation, the culture supernatant was recovered, and the protein level of IL8 in the respective cells was determined using the Human IL-8 Immunoassay kit (Quantkine).

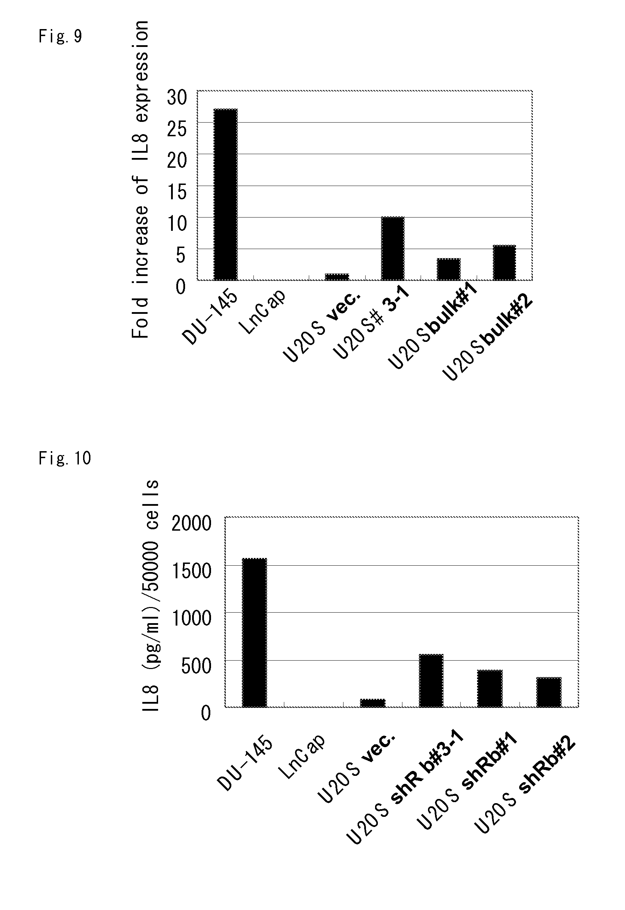

The results obtained by TaqMan PCR are shown in Fig. 9, and the results obtained by ELISA are shown in Fig. 10. As controls, DU-145 which is known to exhibit high IL8 expression and LnCap which is known to exhibit low IL8 expression were used.

As a result, the IL8 expression increased to a physiologically high level. Heretofore, it was considered that the function of Rb was the regulation of proliferation in cells. From the results obtained this time, it was found that Rb had an influence on an extracellular environment mediated by the expression of angiogenesis-related genes and promoted the tumor formation.

### (Analysis of gene (ECT2) in which a change in the expression was observed)

It was verified whether the gene shown in this analysis was regulated by Rb/E2F by focusing attention on ECT2.

### 1. Construction of reporter plasmid having ECT2 promoter inserted therein

By using genomic DNA extracted from the U-2 OS cell as a template, a fragment of 1000 bp from the transcription start site of ECT2 gene was amplified by PCR. The amplified DNA was inserted into pGL3 vector, whereby a reporter plasmid having ECT2 promoter inserted therein was constructed. Hereinafter, the constructed plasmid was referred to as pGL3 ECT2.

### 2. Verification of Rb/E2F regulation of ECT2 promoter

Into the U-2 OS cell, pGL3 ECT2 and E2F1 expression plasmid were introduced using Funege 6. After 24 hours, the activity of luciferase, which is a reporter gene, was measured using the Steady-Glo Luciferase Assay System (Promega). The reporter activity was corrected by Renilla luciferase.

### 3. Verification of E2F binding to ECT2 promoter region

An assay was carried out using the Chromatin Immunoprecipitation (ChIP) Assay Kit (Upstate biotechnology). By treating the U-2 OS cell with formaldehyde, a transcription factor was crosslinked to the promoter. Then, the cells were lysed, and the crosslinked chromatin was sonicated to yield fragments with a DNA size of about 500 bp. The thus prepared soluble chromatin was immunoprecipitated with E2F1 and E2F4 antibodies. Determination was carried out by the PCR method to examine whether or not ECT2 promoter was present in the fraction in which chromatin was immunoprecipitated. In the PCR, the following primers were used.

### Positive control

CCNA2 promoter Fw: 5'-CGCTTTCATTGGTCCATTTC-3' (SEQ ID NO: 28)
CCNA2 promoter Rev.: 5'-CCGGCCAAAGAATAGTCGTA-3' (SEQ ID NO: 29)

### Negative control

CCNA2 negative Fw: 5'-AATCTGTAACAATGAAAGACTGCC-3' (SEQ ID NO: 30)
CCNA2 negative Rev: 5'-GATACCATAATTTGTACTTGGCCA-3' (SEQ ID NO: 31)

### ECT2 promoter

ECT2 promoter Fw: 5'-GTCCAGAGTTATATTGGCAC-3' (SEQ ID NO: 32)
ECT2 promoter Rev: 5'-AACAGCAACAATGAATTTCTC-3' (SEQ ID NO: 33)

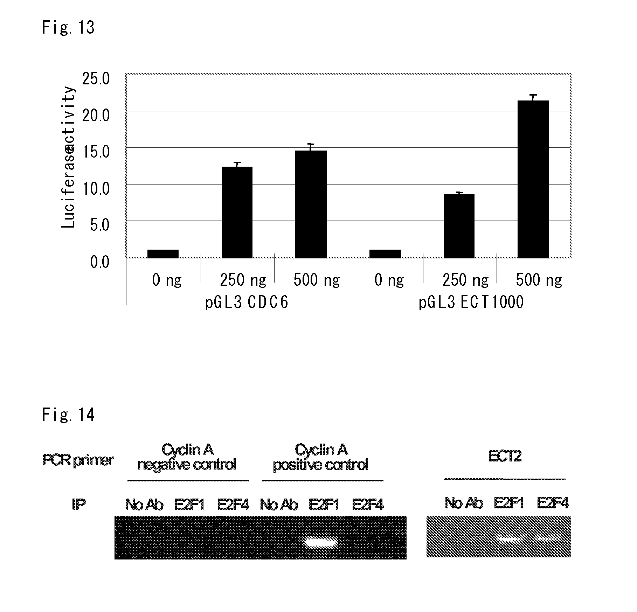

The results of reporter assay of ECT2 promoter are shown in Fig. 13. In the same manner as the CDC6 promoter known to be regulated by Rb/E2F, which was used as the positive control, the transcriptional activity of ECT2 promoter was increased by E2F1. In order to confirm that this result is a direct result, the binding of ECT2 promoter to E2F was examined by the ChIP assay. As shown in Fig. 14, it could be confirmed that in the promoter crosslinked to E2F, ECT2 promoter was included.

### (Effect of ECT2 on cell cycle progression)

From the above results, it was assumed that Rb inhibited the G2/M phase progression by suppressing the expression of a gene associated with the G2/M phase such as ECT2. In order to verify this assumption, the U-2 OS shRb cell was treated with doxorubicin and simultaneously ECT2 was inhibited by an siRNA, and the cell cycle was examined by flow cytometry. To be more specific, into the U-2 OS shRb cell, an siRNA against ECT2 was transfected. At 24 hours thereafter, 10 nM doxorubicin was added thereto, and at 24 hours and 48 hours after the addition, the cells were recovered. The DNA of the cells was stained with Cycle TESTTM PLUS DNA Reagent Kit (Becton Dickinson cat#; 340242), and an analysis was carried out by flow cytometry.

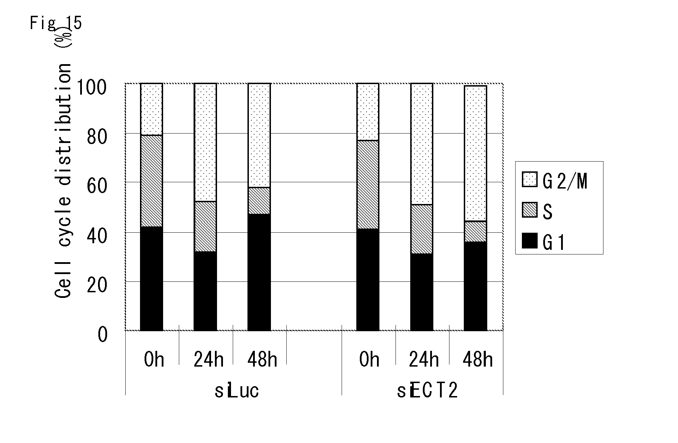

As shown in Fig. 15, while in the cell transfected with Luc siRNA, which was a control, the cells in the G2/M phase decreased more at 48 hours compared with at 24 hours, a decrease in the cells in the G2/M phase was not observed under the inhibition of ECT2 even at 48 hours, and it was found that the G2/M phase progression was inhibited.

### Industrial Applicability

As described above, with the use of the method for evaluating a compound of the invention or the like, it becomes possible to provide a molecule which is present on the Rb pathway and can be used as a drug discovery target for an anticancer agent or a tumor marker and a method capable of evaluating a compound (a candidate compound for an anticancer agent) using the molecule. Accordingly, it becomes possible to provide a therapeutic agent or a diagnostic agent for tumor or cancer caused by an abnormality of Rb or a molecule on the Rb pathway.

## Claims

1. A method for evaluating a compound effective in the treatment of cancer, **characterized by** comprising the steps of:
introducing any one gene selected from the group consisting of IL-8, IL-8 receptor, ECT2, CCNE2, FEN1, KIF20A, CDC25C, KIF11, BUB1, NEK2, CCNA2 and KIF18A or a gene functionally equivalent to the gene thereby to prepare a cell expressing a protein, which is a transcription product of the gene;
bringing a test compound into contact with the cell; and
detecting a specific binding of the test compound to the protein.

2. A method for evaluating a compound effective in the treatment of cancer, **characterized by** comprising the steps of:
introducing any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene thereby to prepare a cell expressing a protein, which is a transcription product of the gene;
bringing a test compound into contact with the cell;
measuring the activity of an intracellular signal transducer induced by the contact; and
comparing said activity with the activity of the intracellular signal transducer in the absence of contact with the test compound, and detecting a change in the activity of the intracellular signal transducer caused by the contact with the test compound.

3. A method for evaluating a compound effective in the treatment of cancer, **characterized by** comprising the steps of:
introducing any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene thereby to prepare a cell expressing a protein, which is a transcription product of the gene;
bringing a test compound into contact with the cell;
measuring the expression level of an intracellular signal transducer mediated by the protein; and
comparing said expression level with the expression level of the intracellular signal transducer in the absence of contact with the test compound, and detecting a change in the expression level of the intracellular signal transducer caused by the contact with the test compound.

4. The method for evaluating a compound according to any one of claims 1 to 3, wherein the gene is IL-8, IL-8 receptor or ECT2.

5. A method for evaluating a compound effective in the treatment of cancer, **characterized by** comprising the steps of:
bringing a test compound into contact with any one protein selected from the group consisting of IL-8, IL-8 receptor, ECT2, CCNE2, FEN1, CENPE, KIF20A, CDC25C, CDCA8, KIF11, KNTC2, BUB1, NEK2, CCNA2 and KIF18A or a protein functionally equivalent to the protein; and
detecting a change in the activity of the protein caused by the contact.

6. The method for evaluating a compound according to claim 5, wherein the protein is IL-8, IL-8 receptor or ECT2.

7. A compound, **characterized in that** it is isolated by the method for evaluating a compound according to any one of claims 1 to 6.

8. A molecular diagnostic method for cancer, comprising the steps of:
measuring the expression level of any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene in a test tissue or a test cell;
comparing said expression level of the gene with the expression level of a corresponding gene in a normal tissue or a normal cell; and
determining whether or not the expression level of the gene in the test tissue or test cell is significantly higher than the expression level of the gene in the normal tissue or normal cell based on the comparison result.

9. The molecular diagnostic method for cancer according to claim 8, wherein the gene is IL-8, IL-8 receptor or ECT2.

10. A molecular diagnostic method for cancer, comprising the steps of:
measuring the expression level of any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a protein functionally equivalent to the protein in a test tissue or a test cell;
comparing said expression level of the protein with the expression level of a corresponding protein in a normal tissue or a normal cell; and
determining whether or not the expression level of the protein in the test tissue or test cell is significantly higher than the expression level of the protein in the normal tissue or normal cell based on the comparison result.

11. The molecular diagnostic method for cancer according to claim 10, wherein the protein is IL-8, IL-8 receptor or ECT2 protein.

12. The molecular diagnostic method for cancer according to any one of claims 8 to 11, wherein the cancer is cancer caused by an abnormality of Rb or a molecule on the Rb pathway.

13. A tumor marker for detecting cancer having an abnormality of Rb or a molecule on the Rb pathway, which is any one protein selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a protein functionally equivalent to the protein.

14. A tumor marker for detecting cancer having an abnormality of Rb or a molecule on the Rb pathway, which is any one gene selected from the group consisting of IL-8, IL-8 receptor, ECT2, MCM2, MCM3, MCM4, MCM5, MCM6, MCM7, CCNE2, FEN1, HMGB2, CENPE, KIF20A, CDC25C, CDCA8, KIF11, HCAP-E, HCAP-G, KNTC2, HCAP-C, BUB1, NEK2, CCNA2 and KIF18A or a gene functionally equivalent to the gene.

15. A method for detecting a target gene for an anticancer agent, comprising the steps of:
preparing an Rb expression-suppressed cell in which the expression of Rb has been suppressed by RNA interference using an Rb-expressing cell;
measuring the expression level of a desired gene in the cell; and
comparing said expression level with the expression level of the gene in the Rb-expressing cell.

16. The method for detecting a target gene according to claim 15, wherein the Rb-expressing cell is U-2 OS, HCT116 or HepG2.

17. The method for detecting a target gene according to claim 15 or 16, wherein an siRNA to be used in the RNA interference is a sequence represented by SEQ ID NO: 27.

18. A method for evaluating a compound, comprising the steps of:
preparing an Rb expression-suppressed cell in which the expression of Rb has been suppressed using an Rb-expressing cell;
bringing a test compound into contact with the Rb expression-suppressed cell and the Rb-expressing cell;
measuring the activity of an intracellular signal transducer induced by the contact; and
comparing the activity in the Rb expression-suppressed cell with the activity in the Rb-expressing cell.

19. A method for evaluating a compound, comprising the steps of:
preparing an Rb expression-suppressed cell in which the expression of Rb has been suppressed using an Rb-expressing cell;
bringing a test compound into contact with the Rb expression-suppressed cell and the Rb-expressing cell;
measuring the expression level of an intracellular signal transducer induced by the contact; and
comparing the expression level in the Rb expression-suppressed cell with the expression level in the Rb-expressing cell.

20. The method for evaluating a compound according to claim 18 or 19, wherein the means of suppressing the expression of Rb in the Rb expression-suppressed cell is through RNA interference.

21. The method for evaluating a compound according to any one of claims 18 to 20, wherein the Rb-expressing cell is U-2 OS, HCT116 or HepG2.

22. The method for evaluating a compound according to any one of claims 18 to 21, wherein an siRNA to be used in the RNA interference is a sequence represented by SEQ ID NO: 27.


SEQUENCE LISTING

<110>  Banyu pharmaceutical co.,ltd.

<120>  Evaluation method and molecular diagnosis method of compound based on a
molecule on Rb pathway

<130>  BY0154

<160>  33

<170>  PatentIn version 3.1

<210>  1
<211>  1666
<212>  DNA
<213>  Homo sapiens

<400>  1
ctccataagg cacaaacttt cagagacagc agagcacaca agcttctagg acaagagcca      60

ggaagaaacc accggaagga accatctcac tgtgtgtaaa catgacttcc aagctggccg     120

tggctctctt ggcagccttc ctgatttctg cagctctgtg tgaaggtgca gttttgccaa     180

ggagtgctaa agaacttaga tgtcagtgca taaagacata ctccaaacct ttccacccca     240

aatttatcaa agaactgaga gtgattgaga gtggaccaca ctgcgccaac acagaaatta     300

ttgtaaagct ttctgatgga agagagctct gtctggaccc caaggaaaac tgggtgcaga     360

gggttgtgga gaagtttttg aagagggctg agaattcata aaaaaattca ttctctgtgg     420

tatccaagaa tcagtgaaga tgccagtgaa acttcaagca aatctacttc aacacttcat     480

gtattgtgtg ggtctgttgt agggttgcca gatgcaatac aagattcctg gttaaatttg     540

aatttcagta aacaatgaat agtttttcat tgtaccatga aatatccaga acatacttat     600

atgtaaagta ttatttattt gaatctacaa aaaacaacaa ataattttta aatataagga     660

ttttcctaga tattgcacgg gagaatatac aaatagcaaa attgaggcca agggccaaga     720

gaatatccga actttaattt caggaattga atgggtttgc tagaatgtga tatttgaagc     780

atcacataaa aatgatggga caataaattt tgccataaag tcaaatttag ctggaaatcc     840

tggatttttt tctgttaaat ctggcaaccc tagtctgcta gccaggatcc acaagtcctt     900

gttccactgt gccttggttt ctcctttatt tctaagtgga aaaagtatta gccaccatct     960

tacctcacag tgatgttgtg aggacatgtg gaagcacttt aagttttttc atcataacat    1020

aaattatttt caagtgtaac ttattaacct atttattatt tatgtattta tttaagcatc    1080

aaatatttgt gcaagaattt ggaaaaatag aagatgaatc attgattgaa tagttataaa    1140

gatgttatag taaatttatt ttattttaga tattaaatga tgttttatta gataaatttc    1200

aatcagggtt tttagattaa acaaacaaac aattgggtac ccagttaaat tttcatttca    1260

gataaacaac aaataatttt ttagtataag tacattattg tttatctgaa attttaattg    1320

aactaacaat cctagtttga tactcccagt cttgtcattg ccagctgtgt tggtagtgct    1380

gtgttgaatt acggaataat gagttagaac tattaaaaca gccaaaactc cacagtcaat    1440

attagtaatt tcttgctggt tgaaacttgt ttattatgta caaatagatt cttataatat    1500

tatttaaatg actgcatttt taaatacaag gctttatatt tttaacttta agatgttttt 1560

atgtgctctc caaatttttt ttactgtttc tgattgtatg gaaatataaa agtaaatatg 1620

aaacatttaa aatataattt gttgtcaaag taaaaaaaaa aaaaaa 1666

<210> 2
<211> 2502
<212> DNA
<213> Homo sapiens

<400> 2
   tattcatcaa gtgccctcta gctgttaagt cactctgatc tctgactgca gctcctactg 60
   ttggacacac ctggccggtg cttcagttag atcaaaccat tgctgaaact gaagaggaca 120
   tgtcaaatat tacagatcca cagatgtggg attttgatga tctaaatttc actggcatgc 180
   cacctgcaga tgaagattac agcccctgta tgctagaaac tgagacactc aacaagtatg 240
   ttgtgatcat cgcctatgcc ctagtgttcc tgctgagcct gctgggaaac tccctggtga 300
   tgctggtcat cttatacagc agggtcggcc gctccgtcac tgatgtctac ctgctgaacc 360
   tggccttggc cgacctactc tttgccctga ccttgcccat ctgggccgcc tccaaggtga 420
   atggctggat ttttggcaca ttcctgtgca aggtggtctc actcctgaag gaagtcaact 480
   tctacagtgg catcctgctg ttggcctgca tcagtgtgga ccgttacctg gccattgtcc 540
   atgccacacg cacactgacc cagaagcgtc acttggtcaa gtttgtttgt cttggctgct 600
   ggggactgtc tatgaatctg tccctgccct tcttcctttt ccgccaggct taccatccaa 660
   acaattccag tccagtttgc tatgaggtcc tgggaaatga cacagcaaaa tggcggatgg 720
   tgttgcggat cctgcctcac acctttggct tcatcgtgcc gctgtttgtc atgctgttct 780
   gctatggatt caccctgcgt acactgttta aggcccacat ggggcagaag caccgagcca 840
   tgagggtcat ctttgctgtc gtcctcatct tcctgctttg ctggctgccc tacaacctgg 900
   tcctgctggc agacaccctc atgaggaccc aggtgatcca ggagagctgt gagcgccgca 960
   acaacatcgg ccgggccctg gatgccactg agattctggg atttctccat agctgcctca 1020
   accccatcat ctacgccttc atcggccaaa attttcgcca tggattcctc aagatcctgg 1080
   ctatgcatgg cctggtcagc aaggagttct tggcacgtca tcgtgttacc tcctacactt 1140
   cttcgtctgt caatgtctct tccaacctct gaaaaccatc gatgaaggaa tatctcttct 1200
   cagaaggaaa gaataaccaa caccctgagg ttgtgtgtgg aaggtgatct ggctctggac 1260
   aggcactatc tgggttttgg ggggacgcta taggatgtgg ggaagttagg aactggtgtc 1320
   ttcaggggcc acaccaacct tctgaggagc tgttgaggta cctccaagga ccggcctttg 1380
   cacctccatg gaaacgaagc accatcattc ccgttgaacg tcacatcttt aacccactaa 1440
   ctggctaatt agcatggcca catctgagcc ccgaatctga cattagatga gagaacaggg 1500
   ctgaagctgt gtcctcatga gggctggatg ctctcgttga ccctcacagg agcatctcct 1560
   caactctgag tgttaagcgt tgagccacca agctggtggc tctgtgtgct ctgatccgag 1620
   ctcagggggg tggttttccc atctcaggtg tgttgcagtg tctgctggag acattgaggc 1680
   aggcactgcc aaaacatcaa cctgccagct ggccttgtga ggagctggaa acacatgttc 1740
   cccttggggg tggtggatga acaaagagaa agagggtttg gaagccagat ctatgccaca 1800
   agaaccccct ttacccccat gaccaacatc gcagacacat gtgctggcca cctgctgagc 1860
   cccaagtgga acgagacaag cagcccttag cccttcccct ctgcagcttc caggctggcg 1920
   tgcagcatca gcatccctag aaagccatgt gcagccacca gtccattggg caggcagatg 1980
   ttcctaataa agcttctgtt ccgtgcttgt ccctgtggaa gtatcttggt tgtgacagag 2040
   tcaagggtgt gtgcagcatt gttggctgtt cctgcagtag aatgggggca gcacctccta 2100
   agaaggcacc tctctgggtt gaagggcagt gttccctggg gctttaactc ctgctagaac 2160
   agtctcttga ggcacagaaa ctcctgttca tgcccatacc cctggccaag gaagatccct 2220
   ttgtccacaa gtaaaaggaa atgctcctcc agggagtctc agcttcaccc tgaggtgagc 2280
   atcatcttct gggttaggcc ttgcctaggc atagccctgc ctcaagctat gtgagctcac 2340
   cagtccctcc ccaaatgctt tccatgagtt gcagtttttt cctagtctgt tttccctcct 2400
   tggagacagg gccctgtcgg tttattcact gtatgtcctt ggtgcctgga gcctactaaa 2460
   tgctcaataa ataatgatca caggaaaaaa aaaaaaaaaa aa 2502

<210> 3
<211> 2859
<212> DNA
<213> Homo sapiens

<400> 3
cattcagaga cagaaggtgg atagacaaat ctccaccttc agactggtag gctcctccag 60

aagccatcag acaggaagat gtgaaaatcc ccagcactca tcccagaatc actaagtggc 120

acctgtcctg ggccaaagtc ccaggacaga cctcattgtt cctctgtggg aatacctccc 180

caggagggca tcctggattt cccccttgca acccaggtca gaagtttcat cgtcaaggtt 240

gtttcatctt ttttttcctg tctaacagct ctgactacca cccaaccttg aggcacagtg 300

aagacatcgg tggccactcc aataacagca ggtcacagct gctcttctgg aggtgtccta 360

caggtgaaaa gcccagcgac ccagtcagga tttaagttta cctcaaaaat ggaagatttt 420

aacatggaga gtgacagctt tgaagatttc tggaaaggtg aagatcttag taattacagt 480

tacagctcta ccctgccccc ttttctacta gatgccgccc catgtgaacc agaatccctg 540

gaaatcaaca agtattttgt ggtcattatc tatgccctgg tattcctgct gagcctgctg 600

ggaaactccc tcgtgatgct ggtcatctta tacagcaggg tcggccgctc cgtcactgat 660

gtctacctgc tgaacctagc cttggccgac ctactctttg ccctgacctt gcccatctgg 720

gccgcctcca aggtgaatgg ctggattttt ggcacattcc tgtgcaaggt ggtctcactc 780

ctgaaggaag tcaacttcta tagtggcatc ctgctactgg cctgcatcag tgtggaccgt 840

tacctggcca ttgtccatgc cacacgcaca ctgacccaga agcgctactt ggtcaaattc 900

atatgtctca gcatctgggg tctgtccttg ctcctggccc tgcctgtctt acttttccga 960

aggaccgtct actcatccaa tgttagccca gcctgctatg aggacatggg caacaataca 1020

gcaaactggc ggatgctgtt acggatcctg ccccagtcct ttggcttcat cgtgccactg 1080
ctgatcatgc tgttctgcta cggattcacc ctgcgtacgc tgtttaaggc ccacatgggg 1140
cagaagcacc gggccatgcg ggtcatcttt gctgtcgtcc tcatcttcct gctctgctgg 1200
ctgccctaca acctggtcct gctggcagac accctcatga ggacccaggt gatccaggag 1260
acctgtgagc gccgcaatca catcgaccgg gctctggatg ccaccgagat tctgggcatc 1320
cttcacagct gcctcaaccc cctcatctac gccttcattg gccagaagtt tcgccatgga 1380
ctcctcaaga ttctagctat acatggcttg atcagcaagg actccctgcc caaagacagc 1440
aggccttcct ttgttggctc ttcttcaggg cacacttcca ctactctcta agacctcctg 1500
cctaagtgca gccccgtggg gttcctccct tctcttcaca gtcacattcc aagcctcatg 1560
tccactggtt cttcttggtc tcagtgtcaa tgcagccccc attgtggtca caggaagtag 1620
aggaggccac gttcttacta gtttcccttg catggtttag aaagcttgcc ctggtgcctc 1680
accccttgcc ataattacta tgtcatttgc tggagctctg cccatcctgc ccctgagccc 1740
atggcactct atgttctaag aagtgaaaat ctacactcca gtgagacagc tctgcatact 1800
cattaggatg gctagtatca aaagaaagaa aatcaggctg gccaacgggg tgaaaccctg 1860
tctctactaa aaatacaaaa aaaaaaaaaa attagccggg cgtggtggtg agtgcctgta 1920
atcacagcta cttgggaggc tgagatggga gaatcacttg aacccgggag gcagaggttg 1980
cagtgagccg agattgtgcc cctgcactcc agcctgagcg acagtgagac tctgtctcag 2040
tccatgaaga tgtagaggag aaactggaac tctcgagcgt tgctgggggg gattgtaaaa 2100
tggtgtgacc actgcagaag acagtatggc agctttcctc aaaacttcag acatagaatt 2160
aacacatgat cctgcaattc cacttatagg aattgaccca caagaaatga aagcagggac 2220
ttgaacccat atttgtacac caatattcat agcagcttat tcacaagacc caaaaggcag 2280
aagcaaccca aatgttcatc aatgaatgaa tgaatggcta agcaaaatgt gatatgtacc 2340
taacgaagta tccttcagcc tgaaagagga atgaagtact catacatgtt acaacacgga 2400
cgaaccttga aaactttatg ctaagtgaaa taagccagac atcaacagat aaatagttta 2460
tgattccacc tacatgaggt actgagagtg aacaaattta cagagacaga aagcagaaca 2520
gtgattacca gggactgagg ggaggggagc atgggaagtg acggtttaat gggcacaggg 2580
tttatgttta ggatgttgaa aaagttctgc agataaacag tagtgatagt tgtaccgcaa 2640
tgtgacttaa tgccactaaa ttgacactta aaaatggttt aaatggtcaa ttttgttatg 2700
tatattttat atcaatttaa aaaaaaacct gagccccaaa aggtatttta atcaccaagg 2760
ctgattaaac caaggctaga accacctgcc tatatttttt gttaaatgat ttcattcaat 2820
atcttttttt taataaacca tttttacttg ggtgtttat 2859

<210> 4
<211> 3916
<212> DNA
<213> Homo sapiens

<400> 4

agagtgctga tttagaagaa tacaaatcat ggctgaaaat agtgtattaa catccactac 60
tgggaggact agcttggcag actcttccat ttttgattct aaagttactg agatttccaa 120
ggaaaactta cttattggat ctacttcata tgtagaagaa gagatgcctc agattgaaac 180
aagagtgata ttggttcaag aagctggaaa acaagaagaa cttataaaag ccttaaagga 240
cattaaagtg ggctttgtaa agatggagtc agtggaagaa tttgaaggtt tggattctcc 300
ggaatttgaa aatgtatttg tagtcacgga ctttcaggat tctgtcttta atgacctcta 360
caaggctgat tgtagagtta ttggaccacc agttgtatta aattgttcac aaaaaggaga 420
gcctttgcca ttttcatgtc gcccgttgta ttgtacaagt atgatgaatc tagtactatg 480
ctttactgga tttaggaaaa aagaagaact agtcaggttg gtgacattgg tccatcacat 540
gggtggagtt attcgaaaag actttaattc aaaagttaca catttggtgg caaattgtac 600
acaaggagaa aaattcaggg ttgctgtgag tctaggtact ccaattatga agccagaatg 660
gatttataaa gcttgggaaa ggcggaatga acaggatttc tatgcagcag ttgatgactt 720
tagaaatgaa tttaaagttc ctccatttca agattgtatt ttaagtttcc tgggattttc 780
agatgaagag aaaaccaata tggaagaaat gactgaaatg caaggaggta aatatttacc 840
gcttggagat gaaagatgca ctcaccttgt agttgaagag aatatagtaa aagatcttcc 900
ctttgaacct tcaaagaaac tttatgttgt caagcaagag tggttctggg gaagcattca 960
aatggatgcc cgagctggag aaactatgta tttatatgaa aaggcaaata ctcctgagct 1020
caagaaatca gtgtcaatgc tttctctaaa tacccctaac agcaatcgca aacgacgtcg 1080
tttaaaagaa acacttgctc agctttcaag agagacagac gtgtcaccat ttccaccccg 1140
taagcgccca tcagctgagc attccctttc catagggtca ctcctagata tctccaacac 1200
accagagtct agcattaact atggagacac cccaaagtct tgtactaagt cttctaaaag 1260
ctccactcca gttccttcaa agcagtcagc aaggtggcaa gttgcaaaag agctttatca 1320
aactgaaagt aattatgtta atatattggc aacaattatt cagttatttc aagtaccatt 1380
ggaagaggaa ggacaacgtg gtggacctat ccttgcacca gaggagatta agactatttt 1440
tggtagcatc ccagatatct ttgatgtaca cactaagata aaggatgatc ttgaagacct 1500
tatagttaat tgggatgaga gcaaaagcat tggtgacatt tttctgaaat attcaaaaga 1560
tttggtaaaa acctaccctc cctttgtaaa cttctttgaa atgagcaagg aaacaattat 1620
taaatgtgaa aaacagaaac caagatttca tgcttttctc aagataaacc aagcaaaacc 1680
agaatgtgga cggcagagcc ttgttgaact tcttatccga ccagtacaga ggttacccag 1740
tgttgcatta cttttaaatg atcttaagaa gcatacagct gatgaaaatc cagacaaaag 1800
cactttagaa aaagctattg gatcactgaa ggaagtaatg acgcatatta atgaggataa 1860
gagaaaaaca gaagctcaaa agcaaatttt tgatgttgtt tatgaagtag atggatgccc 1920
agctaatctt ttatcttctc accgaagctt agtacagcgg gttgaaacaa tttctctagg 1980
tgagcacccc tgtgacagag gagaacaagt aactctcttc ctcttcaatg attgcctaga 2040
gatagcaaga aaacggcaca aggttattgg cacttttagg agtcctcatg gccaaacccg 2100

acccccagct tctcttaagc atattcacct aatgcctctt tctcagatta agaaggtatt 2160
ggacataaga gagacagaag attgccataa tgcttttgcc ttgcttgtga ggccaccaac 2220
agagcaggca aatgtgctac tcagtttcca gatgacatca gatgaacttc caaaagaaaa 2280
ctggctaaag atgctgtgtc gacatgtagc taacaccatt tgtaaagcag atgctgagaa 2340
tcttatttat actgctgatc cagaatcctt tgaagtaaat acaaaagata tggacagtac 2400
attgagtaga gcatcaagag caataaaaaa gacttcaaaa aaggttacaa gagcattctc 2460
tttctccaaa actccaaaaa gagctcttcg aagggctctt atgacatccc acggctcagt 2520
ggagggaaga agtccttcca gcaatgataa gcatgtaatg agtcgtcttt ctagcacatc 2580
atcattagca ggtatccctt ctccctccct tgtcagcctt ccttccttct ttgaaaggag 2640
aagtcatacg ttaagtagat ctacaactca tttgatatga agcgttacca aaatcttaaa 2700
ttatagaaat gtatagacac ctcatactca aataagaaac tgacttaaat ggtacttgta 2760
attagcactt ggtgaaagct ggaaggaaga taaataacac taaactatgc tatttgattt 2820
ttcttcttga aagagtaagg tttacctgtt acattttcaa gttaattcat gtaaaaaatg 2880
atagtgattt tgatgtaatt tatctcttgt ttgaatctgt cattcaaagg ccaataattt 2940
aagttgctat cagctgatat tagtagcttt gcaaccctga tagagtaaat aaattttatg 3000
ggcgggtgcc aaatactgct gtgaatctat ttgtatagta tccatgaatg aatttatgga 3060
aatagatatt tgtgcagctc aatttatgca gagattaaat gacatcataa tactggatga 3120
aaacttgcat agaattctga ttaaatagtg ggtctgtttc acatgtgcag tttgaagtat 3180
ttaaataacc actcctttca cagtttattt tcttctcaag cgttttcaag atctagcatg 3240
tggattttaa aagatttgcc ctcattaaca agaataacat ttaaaggaga ttgtttcaaa 3300
atatttttgc aaattgagat aaggacagaa agattgagaa acattgtata ttttgcaaaa 3360
acaagatgtt tgtagctgtt tcagagagag tacggtatat ttatggtaat tttatccact 3420
agcaaatctt gatttagttt gatagtgtgt ggaattttat tttgaaggat aagaccatgg 3480
gaaaattgtg gtaaagactg tttgtaccct tcatgaaata attctgaagt tgccatcagt 3540
tttactaatc ttctgtgaaa tgcatagata tgcgcatgtt caacttttta ttgtggtctt 3600
ataattaaat gtaaaattga aaattcattt gctgtttcaa agtgtgatat ctttcacaat 3660
agccttttta tagtcagtaa ttcagaataa tcaagttcat atggataaat gcatttttat 3720
ttcctatttc tttagggagt gctacaaatg tttgtcactt aaatttcaag tttctgtttt 3780
aatagttaac tgactataga ttgttttcta tgccatgtat gtgccacttc tgagagtagt 3840
aaatgactct ttgctacatt ttaaaagcaa ttgtattagt aagaactttg taaataaata 3900
cctaaaaccc aagtgt 3916

<210> 5
<211> 3453
<212> DNA
<213> Homo sapiens

<400> 5
acttttcgcg cgaaacctgg ttgttgctgt agtggcggag aggatcgtgg tactgctatg 60
gcggaatcat cggaatcctt caccatggca tccagcccgg cccagcgtcg gcgaggcaat 120
gatcctctca cctccagccc tggccgaagc tcccggcgta ctgatgccct cacctccagc 180
cctggccgtg accttccacc atttgaggat gagtccgagg ggctcctagg cacagagggg 240
cccctggagg aagaagagga tggagaggag ctcattggag atggcatgga aagggactac 300
cgcgccatcc cagagctgga cgcctatgag gccgagggac tggctctgga tgatgaggac 360
gtagaggagc tgacggccag tcagagggag gcagcagagc gggccatgcg gcagcgtgac 420
cgggaggctg gccggggcct gggccgcatg cgccgtgggc tcctgtatga cagcgatgag 480
gaggacgagg agcgccctgc ccgcaagcgc cgccaggtgg agcgggccac ggaggacggc 540
gaggaggacg aggagatgat cgagagcatc gagaacctgg aggatctcaa aggccactct 600
gtgcgcgagt gggtgagcat ggcgggcccc cggctggaga tccaccaccg cttcaagaac 660
ttcctgcgca ctcacgtcga cagccacggc cacaacgtct tcaaggagcg catcagcgac 720
atgtgcaaag agaaccgtga gagcctggtg gtgaactatg aggacttggc agccagggag 780
cacgtgctgg cctacttcct gcctgaggca ccggcggagc tgctgcagat ctttgatgag 840
gctgccctgg aggtggtact ggccatgtac cccaagtacg accgcatcac caaccacatc 900
catgtccgca tctcccacct gcctctggtg gaggagctgc gctcgctgag gcagctgcat 960
ctgaaccagc tgatccgcac cagtggggtg gtgaccagct gcactggcgt cctgccccag 1020
ctcagcatgg tcaagtacaa ctgcaacaag tgcaatttcg tcctgggtcc tttctgccag 1080
tcccagaacc aggaggtgaa accaggctcc tgtcctgagt gccagtcggc cggccccttt 1140
gaggtcaaca tggaggagac catctatcag aactaccagc gtatccgaat ccaggagagt 1200
ccaggcaaag tggcggctgg ccggctgccc cgctccaagg acgccattct cctcgcagat 1260
ctggtggaca gctgcaagcc aggagacgag atagagctga ctggcatcta tcacaacaac 1320
tatgatggct ccctcaacac tgccaatggc ttccctgtct ttgccactgt catcctagcc 1380
aaccacgtgg ccaagaagga caacaaggtt gctgtagggg aactgaccga tgaagatgtg 1440
aagatgatca ctagcctctc caaggatcag cagatcggag agaagatctt tgccagcatt 1500
gctccttcca tctatggtca tgaagacatc aagagaggcc tggctctggc cctgttcgga 1560
ggggagccca aaaacccagg tggcaagcac aaggtacgtg gtgatatcaa cgtgctcttg 1620
tgcggagacc ctggcacagc gaagtcgcag tttctcaagt atattgagaa agtgtccagc 1680
cgagccatct tcaccactgg ccagggggcg tcggctgtgg gcctcacggc gtatgtccag 1740
cggcaccctg tcagcaggga gtggaccttg gaggctgggg ccctggttct ggctgaccga 1800
ggagtgtgtc tcattgatga atttgacaag atgaatgacc aggacagaac cagcatccat 1860
gaggccatgg agcaacagag catctccatc tcgaaggctg gcatcgtcac ctccctgcag 1920
gctcgctgca cggtcattgc tgccgccaac cccataggag ggcgctacga cccctcgctg 1980
actttctctg agaacgtgga cctcacagag cccatcatct cacgctttga catcctgtgt 2040

gtggtgaggg acaccgtgga cccagtccag gacgagatgc tggcccgctt cgtggtgggc 2100
agccacgtca gacaccaccc cagcaacaag gaggaggagg ggctggccaa tggcagcgct 2160
gctgagcccg ccatgcccaa cacgtatggc gtggagcccc tgccccagga ggtcctgaag 2220
aagtacatca tctacgccaa ggagagggtc cacccgaagc tcaaccagat ggaccaggac 2280
aaggtggcca agatgtacag tgacctgagg aaagaatcta tggcgacagg cagcatcccc 2340
attacggtgc ggcacatcga gtccatgatc cgcatggcgg aggcccacgc gcgcatccat 2400
ctgcgggact atgtgatcga agacgacgtc aacatggcca tccgcgtgat gctggagagc 2460
ttcatagaca cacagaagtt cagcgtcatg cgcagcatgc gcaagacttt tgcccgctac 2520
ctttcattcc ggcgtgacaa caatgagctg ttgctcttca tactgaagca gttagtggca 2580
gagcaggtga catatcagcg caaccgcttt ggggcccagc aggacactat tgaggtccct 2640
gagaaggact tggtggataa ggctcgtcag atcaacatcc acaacctctc tgcattttat 2700
gacagtgagc tcttcaggat gaacaagttc agccacgacc tgaaaaggaa aatgatcctg 2760
cagcagttct gaggccctat gccatccata aggattcctt gggattctgg tttggggtgg 2820
tcagtgccct ctgtgcttta tggacacaaa accagagcac ttgatgaact cggggtacta 2880
gggtcagggc ttatagcagg atgtctggct gcacctggca tgactgtttg tttctccaag 2940
cctgctttgt gcttctcacc tttgggtggg atgccttgcc agtgtgtctt acttggttgc 3000
tgaacatctt gccacctccg agtgctttgt ctccactcag taccttggat cagagctgct 3060
gagttcagga tgcctgcgtg tggtttaggt gttagccttc ttacatggat gtcaggagag 3120
ctgctgccct cttggcgtga gttgcgtatt caggctgctt ttgctgcctt tggccagaga 3180
gctggttgaa gatgtttgta atcgttttca gtctcctgca ggtttctgtg cccctgtggt 3240
ggaagagggc acgacagtgc cagcgcagcg ttctgggctc ctcagtcgca ggggtgggat 3300
gtgagtcatg cggattatcc actcgccaca gttatcagct gccattgctc cctgtctgtt 3360
tccccactct cttatttgtg cattcggttt ggtttctgta gttttaattt ttaataaagt 3420
tgaataaaat ataaaaaaaa aaaaaaaaaa aaa 3453

<210> 6
<211> 3143
<212> DNA
<213> Homo sapiens

<400> 6
ctcgaggtaa cctttggtgg cgggaagagt tcggaagttt tcgcgccggg gtggagtcat 60
cctgggaacc tccacgcgac tttggtggag gtagttcttt ggcagcgggc atggcgggta 120
ccgtggtgct ggacgatgtg gagctgcggg aggctcagag agattacctg gacttcctgg 180
acgacgagga agaccaggga atttatcaga gcaaagttcg ggagctgatc agtgacaacc 240
aataccggct gattgtcaat gtgaatgacc tgcgcaggaa aaacgagaag agggctaacc 300
ggcttctgaa caatgccttt gaggagctgg ttgccttcca gcgggcctta aaggattttg 360
tggcctccat tgatgctacc tatgccaagc agtatgagga gttctacgta ggactggaag 420

gcagctttgg ctccaagcac gtctccccgc ggactcttac ctcctgcttc ctcagctgtg 480
tggtctgtgt ggagggcatt gtcactaaat gttctctagt tcgtcccaaa gtcgtccgca 540
gtgtccacta ctgtcctgct actaagaaga ccatagagcg acgttattct gatctcacca 600
ccctggtggc ctttccctcc agctctgtct atcctaccaa ggatgaggag aacaatcccc 660
ttgagacaga atatggcctt tctgtctaca aggatcacca gaccatcacc atccaggaga 720
tgccggagaa ggccccagcc ggccagctcc cccgctctgt ggacgtcatt ctggatgatg 780
acttggtgga taaagcgaag cctggtgacc gggttcaggt ggtgggaacc taccgttgcc 840
ttcctggaaa gaagggaggc tacacctctg ggaccttcag gactgtcctg attgcctgta 900
atgttaagca gatgagcaag gatgctcagc cctctttctc tgctgaggat atagccaaga 960
tcaagaagtt cagtaaaacc cgatccaagg atatctttga ccagctggcc aagtcattgg 1020
ccccaagtat ccatgggcat gactatgtca agaaagcaat cctctgcttg ctcttgggag 1080
gggtggaacg agacctagaa aatggcagcc acatccgtgg ggacatcaat attcttctaa 1140
taggagaccc atccgttgcc aagtctcagc ttctgcggta tgtgctttgc actgcacccc 1200
gagctatccc caccactggc cggggctcct ctggagtggg tctgacggct gctgtcacca 1260
cagaccagga aacaggagag cgccgtctgg aagcaggggc catggtcctg gctgaccgag 1320
gcgtggtttg cattgatgaa tttgacaaaa tgtctgacat ggatcgcaca gccatccatg 1380
aagtgatgga gcagggtcga gtgaccattg ccaaggctgg catccatgct cggctgaatg 1440
cccgctgcag tgttttggca gctgccaacc ctgtctacgg caggtatgac cagtataaga 1500
ctccaatgga gaacattggg ctacaggact cactgctgtc acgatttgac ttgctcttca 1560
tcatgctgga tcagatggat cctgagcagg atcgggagat ctcagaccat gtccttcgga 1620
tgcaccgtta cagagcacct ggggagcagg atggcgatgc tatgcccttg ggtagtgctg 1680
tggatatcct ggccacagat gatcccaact ttagccagga agatcagcag gacacccaga 1740
tttatgagaa gcatgacaac cttctacatg ggaccaagaa gaaaaaggag aagatggtga 1800
gtgcagcatt catgaagaag tacatccatg tggccaaaat catcaagcct gtcctgacac 1860
aggagtcggc cacctacatt gcagaagagt attcacgcct gcgcagccag gatagcatga 1920
gctcagacac cgccaggaca tctccagtta cagcccgaac actggaaact ctgattcgac 1980
tggccacagc ccatgcgaag gcccgcatga gcaagactgt ggacctgcag gatgcagagg 2040
aagctgtgga gttggtccag tatgcttact ttaagaaggt tctggagaag gagaagaaac 2100
gtaagaagcg aagtgaggat gaatcagaga cagaagatga agaggagaaa agccaagagg 2160
accaggagca gaagaggaag agaaggaaga ctcgccagcc agatgccaaa gatggggatt 2220
catacgaccc ctatgacttc agtgacacag aggaggaaat gcctcaagta cacactccaa 2280
agacggcaga ctcacaggag accaaggaat cccagaaagt ggagttgagt gaatccaggt 2340
tgaaggcatt caaggtggcc ctcttggatg tgttccggga agctcatgcg cagtcaatcg 2400
gcatgaatcg cctcacagaa tccatcaacc gggacagcga agagcccttc tcttcagttg 2460
agatccaggc tgctctgagc aagatgcagg atgacaatca ggtcatggtg tctgagggca 2520

tcatcttcct catctgagga ggcctcgtct ctgaacttgg gttgtgccga gagagtttgt 2580

tctgtgtttc ccaccctctc cctgacccaa gtctttgcct ctactccctt aacagtgttg 2640

aattcaactg aaggcgagga atgttggtga tgaagctgag ttcaggactc ggtggaccct 2700

ttgggaatgg gtcatgaaag ctgccatggg gtgaggaaag aggagacagt gggagaggac 2760

aatgactatt gcatcttcat tgcaaaagca ctggctcatc cgccctactt cccatcccac 2820

acaaacccaa ttgtaaataa catatgactt ctgagtactt ttgggggcac aactgttttc 2880

tgtttgctgt ttttttgttt tgtttttttt ctccagagca ctttggtcta gactaggctt 2940

tgggtggttc caattggtgg agagaagctc tgaggcacgt catgcaggtc aagaaagctt 3000

tctttgcagt agcaccagtt aaggtgaata tgtattgtat cacaaaacaa acccaatatc 3060

cagatgaata tccgagatgt tgaataaact tagccatttc gtacacatgg taaaaaaaaa 3120

aaaaaaaaaa aaaaaaaaaa aaa 3143

<210> 7
<211> 3417
<212> DNA
<213> Homo sapiens

<400> 7
gggtctcgcg gtttgggagc gctactcgcc aggtggactc ggagtccgcg agcgtcgtcg 60

gcaagcggcc gcctttccac ggtactccga gcactatgtc gtccccggcg tcgaccccga 120

gccgccgcgg cagccggcgt ggaagggcca cccccgccca gacgcctcgg agtgaggatg 180

ccaggtcatc tccctctcag agacgtagag gcgaggattc cacctccacg ggggagttgc 240

agccgatgcc aacctcgcct ggagtggacc tgcagagccc tgctgcgcag gacgtgctgt 300

tttccagccc tccccaaatg cattcttcag ctatccctct tgactttgat gttagttcac 360

cactgacata cggcactccc agctctcggg tagagggaac cccaagaagt ggtgttaggg 420

gcacacctgt gagacagagg cctgacctgg gctctgcaca gaagggcctg caagtggatc 480

tgcagtctga cggggcagca gcagaagata tagtggcaag tgagcagtct ctaggccaaa 540

aacttgtgat ctggggaaca gatgtaaatg tggcagcatg caaagaaaac tttcagagat 600

ttcttcagcg ttttattgac cctctggcta aagaagaaga aaatgttggc atagatatta 660

ctgaacctct atacatgcaa cgacttgggg agattaatgt tattggtgag ccatttttaa 720

atgtgaactg tgaacacatc aaatcatttg acaaaaattt gtacagacaa ctcatctctt 780

acccacagga agttattcca acttttgaca tggctgtcaa tgaaatcttc tttgaccgtt 840

accctgactc aatcttagaa catcagattc aagtaagacc attcaacgca ttgaagacta 900

agaatatgag aaacctgaat ccagaagaca ttgaccagct catcaccatc agcggcatgg 960

tgatcaggac atcccagctg attcccgaga tgcaggaggc cttcttccag tgccaagtgt 1020

gtgcccacac gacccgggtg gagatggacc gcggccgcat tgcagagccc agtgtgtgcg 1080

ggcgctgcca caccacccac agcatggcac tcatccacaa ccgctccctc ttctctgaca 1140

agcagatgat caagcttcag gagtctccgg aagacatgcc tgcagggcag acaccacaca 1200

cagttatcct gtttgctcac aatgatctcg ttgacaaggt ccagcctggg gacagagtga 1260
atgttacagg catctatcga gctgtgccta ttcgagtcaa tccaagagtg agtaatgtga 1320
agtctgtcta caaaacccac attgatgtca ttcattatcg gaaaacggat gcaaaacgtc 1380
tgcatggcct tgatgaagaa gcagaacaga aacttttttc agagaaacgt gtggaattgc 1440
ttaaggaact ttccaggaaa ccagacattt atgagaggct tgcttcagcc ttggctccaa 1500
gcatttatga acatgaagat ataaagaagg gaattttgct tcagctcttt ggcgggacaa 1560
ggaaggattt tagtcacact ggaaggggca aatttcgggc tgagatcaac atcttgctgt 1620
gtggcgaccc tggtaccagc aagtcccagc tgctgcagta cgtgtacaac ctcgtcccca 1680
ggggccagta cacgtctggg aagggctcca gtgcagttgg cctcactgcg tacgtaatga 1740
aagaccctga gacaaggcag ctggtcctgc agacaggtgc tcttgtcctg agtgacaacg 1800
gcatctgctg tatcgatgag ttcgacaaga tgaatgaaag tacaagatcg gtattgcatg 1860
aagtcatgga acagcagact ctgtccattg caaaggctgg gatcatctgt cagctcaatg 1920
cgcgcacctc tgtcctggca gcagcaaatc ccattgagtc tcagtggaat cctaaaaaaa 1980
caaccattga aaacatccag ctgcctcata ctttattatc aaggtttgat ttgatcttcc 2040
tcttgctgga ccctcaggac gaagcctatg acaggcgtct ggctcaccac ctggtcgcac 2100
tgtactacca gagcgaggag caggcagagg aggagctcct ggacatggcg gtgctaaagg 2160
actacattgc ctacgcgcac agcaccatca tgccgcggct aagtgaggaa gccagccagg 2220
ctctcatcga ggcttatgta gacatgagga agattggcag tagccgggga atggtttctg 2280
cataccctcg acagctagag tcattaatcc gcttagcaga agcccatgct aaagtaagat 2340
tgtctaacaa agttgaagcc attgatgtgg aagaggccaa acgcctccat cgggaagctc 2400
tgaagcagtc tgcaactgat ccccggactg gcatcgtgga catatctatt cttactacgg 2460
ggatgagtgc cacctctcgt aaacggaaag aagaattagc tgaagcattg aaaaagctta 2520
ttttatctaa gggcaaaaca ccagctctaa aataccagca actttttgaa gatattcggg 2580
gacaatctga catagcaatt actaaagata tgtttgaaga agcactgcgt gccctggcag 2640
atgatgattt cctgacagtg actgggaaga ccgtgcgctt gctctgaagc cttgtgagca 2700
aggaaggctc cctgcatgtc ctgcttgctg cacgccacat gggtgtggtc tgcatctcag 2760
ttggccgcca tcagtgtaaa tagagcttaa agtcatggtt tggctgcata aaaattttct 2820
aacttgggtt caatatttgt agtgaagtat ctgttttcat ttttttcacg ttataaataa 2880
aaatactatg ctggccgggc gcggtggctc acacctgtaa tcccagcact ttgggaggcc 2940
aatgtgggtg gatcatgagg tcaggagttc aagaccagcc tagccaagat ggtgaaaccc 3000
cgtctctagt aaagataaca aaaaattagc tgggcttgat ggcatgcgcc tgtaatccca 3060
gctactcggg aggttgaggc aggagaatcg cttaaaccca ggcggcagag gttgcagtga 3120
gccaagatcg cgccactgca ctccagcctc agcaatagag tgagactgtc tcaaaaaaaa 3180
aaaaaaaaaa aaaacctgcc aattttcaaa cataccgtag agattatttt caggtgccat 3240

tttatagtat agcagcaggg cttttactct gtgtatgcac agatgcagtc tggggcatgg 3300

tttgtgtgct ggactttctc atggccatca tcagtatgct tatggatttg atgacaggca 3360

tagcctgggc atatcacctc attggtaaag ggctagagcc tttctttttt atggcac 3417

<210> 8
<211> 2548
<212> DNA
<213> Homo sapiens

<400> 8
ttttcccgcg aaactcggcg gctgagcgtg gaggttcttg tctcccctgg tttgtgaagt 60

gcggaaaacc agaggcgcag tcatgtcggg attcgacgat cctggcattt tctacagcga 120

cagcttcggg ggcgacgccc aggccgacga ggggcaggcc cgcaaatcgc agctgcagag 180

gcgcttcaag gagttcctgc ggcagtaccg agtgggcacc gaccgcacgg gcttcacctt 240

caaatacagg gatgaactca agcggcatta caacctgggg gagtactgga ttgaggtgga 300

gatggaggat ctggccagct ttgatgagga cctggccgac tacttgtaca agcagccagc 360

cgagcacctg cagctgctgg aggaagctgc caaggaggta gctgatgagg tgacccggcc 420

ccggccttct ggggaggagg tgctccagga catccaggtc atgctcaagt cggacgccag 480

cccttccagc attcgtagcc tgaagtcgga catgatgtca cacctggtga agatccctgg 540

catcatcatc gcggcctctg cggtccgtgc caaggccacc cgcatctcta tccagtgccg 600

cagctgccgc aacaccctca ccaacattgc catgcgccct ggcctcgagg gctatgccct 660

gcccaggaag tgcaacacag atcaggctgg gcgccccaaa tgcccattgg acccgtactt 720

catcatgccc gacaaatgca aatgcgtgga cttccagacc ctgaagctgc aggagctgcc 780

tgatgcagtc ccccacgggg agatgcccag acacatgcag ctctactgcg acaggtacct 840

gtgtgacaag gtcgtccctg ggaacagggt taccatcatg ggcatctact ccatcaagaa 900

gtttggcctg actaccagca ggggccgtga cagggtgggc gtgggcatcc gaagctccta 960

catccgtgtc ctgggcatcc aggtggacac agatggctct ggccgcagct ttgctggggc 1020

cgtgagcccc caggaggagg aggagttccg tcgcctggct gccctcccaa atgtctatga 1080

ggtcatctcc aagagcatcg ccccctccat ctttgggggc acagacatga agaaggccat 1140

tgcctgcctg ctctttgggg gctcccgaaa gaggctccct gatggactta ctcgccgagg 1200

agacatcaac ctgctgatgc taggggaccc tgggacagcc aagtcccagc ttctgaagtt 1260

tgtggagaag tgttctccca ttggggtata cacgtctggg aaaggcagca gcgcagctgg 1320

actgacagcc tcggtgatga gggacccttc gtcccggaat ttcatcatgg agggcggagc 1380

catggtcctg gccgatggtg gggtcgtctg tattgacgag tttgacaaga tgcgagaaga 1440

tgaccgtgtg gcaatccacg aagccatgga gcagcagacc atctctatcg ccaaggctgg 1500

gatcaccacc accctgaact cccgctgctc cgtcctggct gctgccaact cagtgttcgg 1560

ccgctgggat gagacgaagg gggaggacaa cattgacttc atgcccacca tcttgtcgcg 1620

cttcgacatg atcttcatcg tcaaggatga gcacaatgag gagagggatg tgatgctggc 1680

caagcatgtc atcactctgc acgtgagcgc actgacacag acacaggctg tggagggcga 1740

gattgacctg gccaagctga agaagtttat tgcctactgc cgagtgaagt gtggcccccg 1800

gctgtcagca gaggctgcag agaaactgaa gaaccgctac atcatcatgc ggagcggggc 1860

ccgtcagcac gagagggaca gtgaccgccg ctccagcatc cccatcactg tgcggcagct 1920

ggaggccatt gtgcgcatcg cggaagccct cagcaagatg aagctgcagc ccttcgccac 1980

agaggcagat gtggaggagg ccctgcggct cttccaagtg tccacgttgg atgctgcctt 2040

gtccggtacc ctgtcagggg tggagggctt caccagccag gaggaccagg agatgctgag 2100

ccgcatcgag aagcagctca agcgccgctt tgccattggc tcccaggtgt ctgagcacag 2160

catcatcaag gacttcacca agcagaaata cccggagcac gccatccaca aggtgctgca 2220

gctcatgctg cggcgcggcg agatccagca tcgcatgcag cgcaaggttc tctaccgcct 2280

caagtgagtc gcgccgcctc actggactca tggactcgcc cacgcctcgc ccctcctgcc 2340

gctgcctgcc attgacaatg ttgctgggac ctctgcctcc ccactgcagc cctcgaactt 2400

cccaggcacc ctcctttctg ccccagagga aggagctgta gtgtcctgct gcctctgggc 2460

gcccgcctct agcgcggttc tgggaagtgt gcttttggca tccgttaata ataaagccac 2520

ggtgtgttca ggtaaaaaaa aaaaaaaa 2548

<210> 9
<211> 3769
<212> DNA
<213> Homo sapiens

<400> 9
aaagctgcag cgtctggaaa aaagcgactt gtggcggtcg agcgtggcgc aggcgaatcc 60

tcggcactaa gcaaatatgg acctcgcggc ggcagcggag ccgggcgccg gcagccagca 120

cctggaggtc cgcgacgagg tggccgagaa gtgccagaaa ctgttcctgg acttcttgga 180

ggagtttcag agcagcgatg gagaaattaa atacttgcaa ttagcagagg aactgattcg 240

tcctgagaga aacacattgg ttgtgagttt tgtggacctg gaacaattta accagcaact 300

ttccaccacc attcaagagg agttctatag agtttaccct tacctgtgtc gggccttgaa 360

aacattcgtc aaagaccgta aagagatccc tcttgccaag gatttttatg ttgcattcca 420

agacctgcct accagacaca agattcgaga gctcacctca tccagaattg gtttgctcac 480

tcgcatcagt gggcaggtgg tgcggactca cccagttcac ccagagcttg tgagcggaac 540

ttttctgtgc ttggactgtc agacagtgat cagggatgta gaacagcagt tcaaatacac 600

acagccaaac atctgccgaa atccagtttg tgccaacagg aggagattct tactggatac 660

aaataaatca agatttgttg attttcaaaa ggttcgtatt caagagaccc aagctgagct 720

tcctcgaggg agtatccccc gcagtttaga agtaatttta agggctgaag ctgtggaatc 780

agctcaagct ggtgacaagt gtgactttac agggacactg attgttgtgc ctgacgtctc 840

caagcttagc acaccaggag cacgtgcaga aactaattcc cgtgtcagtg gtgttgatgg 900

atatgagaca gaaggcattc gaggactccg ggcccttggt gttagggacc tttcttatag 960

gctggtcttt cttgcctgct gtgttgcgcc aaccaaccca aggtttgggg ggaaagagct 1020
cagagatgag gaacagacag ctgagagcat taagaaccaa atgactgtga aagaatggga 1080
gaaagtgttt gagatgagtc aagataaaaa tctataccac aatctttgta ccagcctgtt 1140
ccctactata catggcaatg atgaagtaaa acggggtgtc ctgctgatgc tctttggtgg 1200
cgttccaaag acaacaggag aagggacctc tcttcgaggg gacataaatg tttgcattgt 1260
tggtgaccca agtacagcta agagccaatt tctcaagcac gtggaggagt tcagccccag 1320
agctgtctac accagtggta aagcgtccag tgctgctggc ttaacagcag ctgttgtgag 1380
agatgaagaa tctcatgagt ttgtcattga ggctggagct ttgatgttgg ctgataatgg 1440
tgtgtgttgt attgatgaat ttgataagat ggacgtgcgg gatcaagttg ctattcatga 1500
agctatggaa cagcagacca tatccatcac taaagcagga gtgaaggcta ctctgaacgc 1560
ccggacgtcc attttggcag cagcaaaccc aatcagtgga cactatgaca gatcaaaatc 1620
attgaaacag aatataaatt tgtcagctcc catcatgtcc cgattcgatc tcttctttat 1680
ccttgtggat gaatgtaatg aggttacaga ttatgccatt gccaggcgca tagtagattt 1740
gcattcaaga attgaggaat caattgatcg tgtctattcc ctcgatgata tcagaagata 1800
tcttctcttt gcaagacagt ttaaacccaa gatttccaaa gagtcagagg acttcattgt 1860
ggagcaatat aaacatctcc gccagagaga tggttctgga gtgaccaagt cttcatggag 1920
gattacagtg cgacagcttg agagcatgat tcgtctctct gaagctatgg ctcggatgca 1980
ctgctgtgat gaggtccaac ctaaacatgt gaaggaagct ttccggttac tgaataaatc 2040
aatcatccgt gtggaaacac ctgatgtcaa tctagatcaa gaggaagaga tccagatgga 2100
ggtagatgag ggtgctggtg gcatcaatgg tcatgctgac agccctgctc ctgtgaacgg 2160
gatcaatggc tacaatgaag acataaatca agagtctgct cccaaagcct ccttaaggct 2220
gggcttctct gagtactgcc gaatctctaa ccttattgtg cttcacctca gaaaggtgga 2280
agaagaagag gacgagtcag cattaaagag gagcgagctt gttaactggt acttgaagga 2340
aatcgaatca gagatagact ctgaagaaga acttataaat aaaaaaagaa tcatagagaa 2400
agttattcat cgactcacac actatgatca tgttctaatt gagctcaccc aggctggatt 2460
gaaaggctcc acagagggaa gtgagagcta tgaagaagat ccctacttgg tagttaaccc 2520
taactacttg ctcgaagatt gagatagtga aagtaactga ccagagctga ggaactgtgg 2580
cacagcacct cgtggcctgg agcctggctg gagctctgct agggacagaa gtgtttctgg 2640
aagtgatgct tccaggattt gttttcagaa acaagaattg agttgatggt cctatgtgtc 2700
acattcatca caggtttcat accaacacag gcttcagcac ttcctttggt gtgtttcctg 2760
tcccagtgaa gttggaacca aataatgtgt agtctctata accaatacct ttgttttcat 2820
gtgtaagaaa aggcccatta cttttaaggt atgtgctgtc ctattgagca aataactttt 2880
tttcaattgc cagctactgc ttttattcat caaaataaaa taacttgttc tgaagttgtc 2940
tattggattt ctttctactg taccctgatt attacttcca tctacttctg aatgtgagac 3000
tttccctttt tgcttaacct ggagtgaaga ggtagaactg tggtattatg gatgaggttt 3060

ctatgagaag gagtcattag agaactcata tgaaagctag aggccttaga gatgactttc 3120

caaggttaat tccagttgtt tttttttttt tttaagttta taaaagttta ttatactttt 3180

ttaaaattac tctttagtaa tttattttac ttctgtgtcc taagggtaat ttctcaggat 3240

tgttttcaaa ttgctttttt aggggaaata ggtcatttgc tatattacaa gcaatcccca 3300

aattttatgg tcttccagga aaagttatta ccgtttatga tactaacagt tcctgagact 3360

tagctatgat cagtatgttc atgaggtgga gcagttcctg tgttgcagct tttaacaaca 3420

gatggcattc attaaatcac aaagtatgtt aaaggtcaca aaagcaaaat aactgtctga 3480

ggctaaggcc cacgtgggac agtctaatac ccatgagtac tcaacttgcc ttgatgtctg 3540

agctttccag tgcaatgtga atttgagcag ccagaaatct attagtagaa agcaagacag 3600

attaatatag gttaaaacaa tgatttaaat atgtttctcc caataattat ctctttccct 3660

ggaatcaact tgtatgaaac cttgtcaaaa tgtactccac aagtatgtac aattaagtat 3720

tttaaaaata aatggcaaac attaaaaaca aaaaaaaaaa aaaaaaaaa 3769

<210> 10
<211> 2821
<212> DNA
<213> Homo sapiens

<400> 10
cgccccttcc cagccccaag ggtctaggat acagtctttg tagatgagcg ggtccccctt 60

ggaggacaga atgaagaatt gggaaatcat ggccgttctg gagagtagac aagaagacgg 120

cgaaagtcgg gcctgccccg ccctgcggcc ccggaacaaa agaacgcgtg tgcgctggcc 180

ctttaagagc gattctcctc cgcccgcgcc agctcggacc gcgggaaacc cggcgcctgc 240

actaccccgc ccggagattc ccttccgacg cccgcaccgc ctccccgtca ctcattctag 300

gcccgcacgg tgattggctt gcggctagcg ggaggtgaag aaggccgcct tgtccgattg 360

gcccgcacgc agtggcgccg gtcacgtggg gggcgacgtt tcgcgccaat ttcggttggc 420

cggccacagt ccaccgcgcg gagattctca gcttccccag gagcaagacc tctgagcccg 480

ccaagcgcgg ccgcacggcc ctcggcagcg atggcactga aggactacgc gctagagaag 540

gaaaaggtta agaagttctt acaagagttc taccaggatg atgaactcgg gaagaagcag 600

ttcaagtatg ggaaccagtt ggttcggctg gctcatcggg aacaggtggc tctgtatgtg 660

gacctggacg acgtagccga ggatgacccc gagttggtgg actcaatttg tgagaatgcc 720

aggcgctacg cgaagctctt tgctgatgcc gtacaagagc tgctgcctca gtacaaggag 780

agggaagtgg taaataaaga tgtcctggac gtttacattg agcatcggct aatgatggag 840

cagcggagtc gggaccctgg gatggtccga agcccccaga accagtaccc tgctgaactc 900

atgcgcagat ttgagctgta ttttcaaggc cctagcagca acaagcctcg tgtgatccgg 960

gaagtgcggg ctgactctgt ggggaagttg gtaactgtgc gtggaatcgt cactcgtgtc 1020

tctgaagtca aacccaagat ggtggtggcc acttacactt gtgaccagtg tggggcagag 1080

acctaccagc cgatccagtc tcccactttc atgcctctga tcatgtgccc aagccaggag 1140

tgccaaacca accgctcagg agggcggctg tatctgcaga cacggggctc cagattcatc 1200
aaattccagg agatgaagat gcaagaacat agtgatcagg tgcctgtggg aaatatccct 1260
cgtagtatca cggtgctggt agaaggagag aacacaagga ttgcccagcc tggagaccac 1320
gtcagcgtca ctggtatttt cttgccaatc ctgcgcactg ggttccgaca ggtggtacag 1380
ggtttactct cagaaaccta cctggaagcc catcggattg tgaagatgaa caagagtgag 1440
gatgatgagt ctggggctgg agagctcacc agggaggagc tgaggcaaat tgcagaggag 1500
gatttctacg aaaagctggc agcttcaatc gccccagaaa tatacggcca tgaagatgtg 1560
aagaaggcac tgctgctcct gctagtcggg ggtgtggacc agtctcctcg aggcatgaaa 1620
atccggggca acatcaacat ctgtctgatg ggggatcctg gtgtggccaa gtctcagctc 1680
ctgtcataca ttgatcgact ggcgcctcgc agccagtaca caacaggccg gggctcctca 1740
ggagtggggc ttacggcagc tgtgctgaga gactccgtga gtggagaact gaccttagag 1800
ggtggggccc tggtgctggc tgaccagggt gtgtgctgca ttgatgagtt cgacaagatg 1860
gctgaggccg accgcacagc catccacgag gtcatggagc agcagaccat ctccattgcc 1920
aaggccggca ttctcaccac actcaatgcc cgctgctcca tcctggctgc cgccaaccct 1980
gcctacgggc gctacaaccc tcgccgcagc ctggagcaga acatacagct acctgctgca 2040
ctgctctccc ggtttgacct cctctggctg attcaggacc ggcccgaccg agacaatgac 2100
ctacggttgg cccagcacat cacctatgtg caccagcaca gccggcagcc cccctcccag 2160
tttgaacctc tggacatgaa gctcatgagg cgttacatag ccatgtgccg cgagaagcag 2220
cccatggtgc cagagtctct ggctgactac atcacagcag catacgtgga gatgaggcga 2280
gaggcttggg ctagtaagga tgccacctat acttctgccc ggaccctgct ggctatcctg 2340
cgcctttcca ctgctctggc acgtctgaga atggtggatg tggtggagaa agaagatgtg 2400
aatgaagcca tcaggctaat ggagatgtca aaggactctc ttctaggaga caaggggcag 2460
acagctagga ctcagagacc agcagatgtg atatttgcca ccgtccgtga actggtctca 2520
gggggccgaa gtgtccggtt ctctgaggca gagcagcgct gtgtatctcg tggcttcaca 2580
cccgcccagt tccaggcggc tctggatgaa tatgaggagc tcaatgtctg gcaggtcaat 2640
gcttcccgga cacggatcac ttttgtctga ttccagcctg cttgcaaccc tggggtcctc 2700
ttgttccctg ctggcctgcc ccttgggaag gggcagtgat gcctttgagg ggaaggagga 2760
gcccctcttt ctcccatgct gcacttactc cttttgctaa taaaagtgtt tgtagattgt 2820
c 2821

<210> 11
<211> 2536
<212> DNA
<213> Homo sapiens

<400> 11
   agcgggtgcg gggcgggacc ggcccggcct atatattggg ttggcgccgg cgccagctga 60
   gccgagcggt agctggtctg gcgaggtttt atacacctga aagaagagaa tgtcaagacg 120
   aagtagccgt ttacaagcta agcagcagcc ccagcccagc cagacggaat ccccccaaga 180
   agcccagata atccaggcca agaagaggaa aactacccag gatgtcaaaa gaagtctggc 240
   taaacatgtt aaaaaaggag agcagatatg ttcatgacaa acattttgaa gttctgcatt 300
   ctgacttgga accacagatg aggtccatac ttctagactg gcttttagag gtatgtgaag 360
   tatacacact tcatagggaa acattttatc ttgcacaaga cttttttgat agatttatgt 420
   tgacacaaaa ggatataaat aaaaatatgc ttcaactcat tggaattacc tcattattca 480
   ttgcttccaa acttgaggaa atctatgctc ctaaactcca agagtttgct tacgtcactg 540
   atggtgcttg cagtgaagag gatatcttaa ggatggaact cattatatta aaggctttaa 600
   aatgggaact ttgtcctgta acaatcatct cctggctaaa tctctttctc caagttgatg 660
   ctcttaaaga tgctcctaaa gttcttctac ctcagtattc tcaggaaaca ttcattcaaa 720
   tagctcagct tttagatctg tgtattctag ccattgattc attagagttc cagtacagaa 780
   tactgactgc tgctgccttg tgccatttta cctccattga agtggttaag aaagcctcag 840
   gtttggagtg ggacagtatt tcagaatgtg tagattggat ggtacctttt gtcaatgtag 900
   taaaaagtac tagtccagtg aagctgaaga cttttaagaa gattcctatg gaagacagac 960
   ataatatcca gacacataca aactatttgg ctatgctgga ggaagtaaat tacataaaca 1020
   ccttcagaaa agggggacag ttgtcaccag tgtgcaatgg aggcattatg acaccaccga 1080
   agagcactga aaaaccacca ggaaaacact aaagaagata actaagcaaa caagttggaa 1140
   ttcaccaaga ttgggtagaa ctggtatcac tgaactacta aagttttaca gaaagtagtg 1200
   ctgtgattga ttgccctagc caattcacaa gttacactgc cattctgatt ttaaaactta 1260
   caattggcac taaagaatac atttaattat ttcctatgtt agctgttaaa gaaacagcag 1320
   gacttgttta caaagatgtc ttcattccca aggttactgg atagaagcca accacagtct 1380
   ataccatagc aatgtttttc ctttaatcca gtgttactgt gtttatcttg ataaactagg 1440
   aattttgtca ctggagtttt ggactggata agtgctacct taaagggtat actaagtgat 1500
   acagtacttt gaatctagtt gttagattct caaaattcct acactcttga ctagtgcaat 1560
   ttggttcttg aaaattaaat ttaaacttgt ttacaaaggt ttagttttgt aataaggtga 1620
   ctaatttatc tatagctgct atagcaagct attataaaac ttgaatttct acaaatggtg 1680
   aaatttaatg ttttttaaac tagtttattt gccttgccat aacacatttt ttaactaata 1740
   aggcttagat gaacatggtg ttcaacctgt gctctaaaca gtgggagtac caaagaaatt 1800
   ataaacaaga taaatgctgt ggctccttcc taactggggc tttcttgaca tgtaggttgc 1860
   ttggtaataa cctttttgta tatcacaatt tgggtgaaaa acttaagtac cctttcaaac 1920
   tatttatatg aggaagtcac tttactactc taagatatcc ctaaggaatt ttttttttta 1980
   atttagtgtg actaaggctt tatttatgtt tgtgaaactg ttaaggtcct ttctaaattc 2040
   ctccattgtg agataaggac agtgtcaaag tgataaagct taacacttga cctaaacttc 2100
   tattttctta aggaagaaga gtattaaata tatactgact cctagaaatc tatttattaa 2160
   aaaaagacat gaaaacttgc tgtacatagg ctagctattt ctaaatattt taaattagct 2220
   tttctaaaaa aaaaatccag cctcataaag tagattagaa aactagattg ctagtttatt 2280
   ttgttatcag atatgtgaat ctcttctccc tttgaagaaa ctatacattt attgttacgg 2340
   tatgaagtct tctgtatagt ttgtttttaa actaatattt gtttcagtat tttgtctgaa 2400
   aagaaaacac cactaattgt gtacatatgt attatataaa cttaaccttt taatactgtt 2460
   tatttttagc ccattgttta aaaaataaaa gttaaaaaaa tttaactgct taaaagtaaa 2520
   aaaaaaaaaa aaaaaa 2536

<210> 12
<211> 2265
<212> DNA
<213> Homo sapiens

<400> 12
agtcctgcga tttcgggtgt agagggagca ggggcctgcg gggacctggt gtgggtggag 60

tggggacaag cggtggagaa gggtacgcca gggtcgctga gagactctgt tctccctgga 120

gggactggtt gccatgagag cagccgtctg aggggacgca gcctgcacta cgcgccccaa 180

gaggctgtgc gtggcgagca ggtcacgtga cgggagcgcg ggctttggaa ggcggctgaa 240

cgtcaggcca cccgccgcta agctgagaag ggagagcgag cttaggaccg cctgcccggg 300

gcaaccccga accaagcttt agccgccgag gccgcgtgtc ccaaaggcca gtcatccctc 360

ctctgtgttg ccatgggaat tcaaggcctg gccaaactaa ttgctgatgt ggcccccagt 420

gccatccggg agaatgacat caagagctac tttggccgta aggtggccat tgatgcctct 480

atgagcattt atcagttcct gattgctgtt cgccagggtg gggatgtgct gcagaatgag 540

gagggtgaga ccaccagcca cctgatgggc atgttctacc gcaccattcg catgatggag 600

aacggcatca agcccgtgta tgtctttgat ggcaagccgc cacagctcaa gtcaggcgag 660

ctggccaaac gcagtgagcg gcgggctgag gcagagaagc agctgcagca ggctcaggct 720

gctggggccg agcaggaggt ggaaaaattc actaagcggc tggtgaaggt cactaagcag 780

cacaatgatg agtgcaaaca tctgctgagc ctcatgggca tcccttatct tgatgcaccc 840

agtgaggcag aggccagctg tgctgccctg gtgaaggctg gcaaagtcta tgctgcggct 900

accgaggaca tggactgcct caccttcggc agccctgtgc taatgcgaca cctgactgcc 960

agtgaagcca aaaagctgcc aatccaggaa ttccacctga gccggattct gcaggagctg 1020

ggcctgaacc aggaacagtt tgtggatctg tgcatcctgc taggcagtga ctactgtgag 1080

agtatccggg gtattgggcc caagcgggct gtggacctca tccagaagca caagagcatc 1140

gaggagatcg tgcggcgact tgaccccaac aagtaccctg tgccagaaaa ttggctccac 1200

aaggaggctc accagctctt cttggaacct gaggtgctgg acccagagtc tgtggagctg 1260

aagtggagcg agccaaatga agaagagctg atcaagttca tgtgtggtga aaagcagttc 1320

tctgaggagc gaatccgcag tggggtcaag aggctgagta agagccgcca aggcagcacc 1380

cagggccgcc tggatgattt cttcaaggtg accggctcac tctcttcagc taagcgcaag 1440

gagccagaac ccaagggatc cactaagaag aaggcaaaga ctggggcagc agggaagttt 1500

aaaaggggaa aataaatgtg tttccccatt atacctcctt caccccagaa tatttgccgt 1560

cttgtaccct taagagctac agctagagaa accttcacgg ggtggagaga ggattctaag 1620

gcttttctag cgtgaccctt ttcagtagtg ctagtccctt ttttacttga tcttaatggc 1680

aagaaggcca cagaggtact tttccttttt tagctcagga aaatatgtca ggctcaaacc 1740

acttctcagg cagtttaatg gacactaagt ccattgttac atgaaagtga tagatagcaa 1800

caagttttgg agaagagaga gggagataaa agggggagac aaaagatgta cagaaatgat 1860

ttcctggctg gccaactggt ggccagtggg aggtgatggt ggacctagac tgtgcttttc 1920

tgtcttgttc agccttgacc caccttgaga gagagccacc aggaaggcgc atcttagcag 1980

atgggaggaa ctgctgagag aagatgggca gaaagctgga gcccctggag ttggctgtgt 2040

ctgtgtttgt gactgattac tggctgtgtc ttgggtgggc agaaactcga acttgctatg 2100

taatttgtgt ctagttattc agaggagtaa gatggtgatg ttcacctggc aatcagctga 2160

gttgagactt tggaataaga cactggtttt catgcgctgt ttttgtttta aagttatgaa 2220

gaaaaaagtc aataaaattc taaaagtaaa aaaaaaaaaa aaaaa 2265

<210> 13
<211> 1277
<212> DNA
<213> Homo sapiens

<400> 13
gatgtggccc gtggcctagc tcgtcaagtt gccgtggcgc ggagaactct gcaaaacaag 60

aggctgagga ttgcgttaga gataaaccag ttcacgccgg agccccgtga gggaagcgtc 120

tccgttgggt ccggccgctc tgcgggactc tgaggaaaag ctcgcaccag gtggacgcgg 180

atctgtcaac atgggtaaag gagaccccaa caagccgcgg ggcaaaatgt cctcgtacgc 240

cttcttcgtg cagacctgcc gggaagagca caagaagaaa cacccggact cttccgtcaa 300

tttcgcggaa ttctccaaga agtgttcgga gagatggaag accatgtctg caaaggagaa 360

gtcgaagttt gaagatatgg caaaaagtga caaagctcgc tatgacaggg agatgaaaaa 420

ttacgttcct cccaaaggtg ataagaaggg gaagaaaaag gaccccaatg ctcctaaaag 480

gccaccatct gccttcttcc tgttttgctc tgaacatcgc ccaaagatca aaagtgaaca 540

ccctggccta tccattgggg atactgcaaa gaaattgggt gaaatgtggt ctgagcagtc 600

agccaaagat aaacaaccat atgaacagaa agcagctaag ctaaaggaga aatatgaaaa 660

ggatattgct gcatatcgtg ccaagggcaa aagtgaagca ggaaagaagg gccctggcag 720

gccaacaggc tcaaagaaga agaacgaacc agaagatgag gaggaggagg aggaagaaga 780

agatgaagat gaggaggaag aggatgaaga tgaagaataa atggctatcc tttaatgatg 840

cgtgtggaat gtgtgtgtgt gctcaggcaa ttattttgct aagaatgtga attcaagtgc 900

agctcaatac tagcttcagt ataaaaactg tacagatttt tgtatagctg ataagattct 960

ctgtagagaa aatactttta aaaaatgcag gttgtagctt tttgatgggc tactcataca 1020

gttagatttt acagcttctg atgttgaatg ttcctaaata tttaatggtt tttttaattt 1080

ctgtgtgtat ggtagcacag caaacttgta ggaattagta tcaatagtaa attttgggtt 1140

ttttaggatg ttgcatttcg tttttttaaa aaaaattttg taataaaatt atgtatatta 1200

tttctattgt ctttgtctta atatgctaag ttaattttca ctttaaaaaa gccatttgaa 1260

gaccaaaaaa aaaaaaa 1277

<210> 14
<211> 8630
<212> DNA
<213> Homo sapiens

<400> 14
taaatttaaa ggcggggcgg cctgtgagcc ctgaagtgcc ggccgcggag ggtcctggcc 60

attttcctgg gaccagttca gcctgatagg atggcggagg aaggagccgt ggccgtctgc 120

gtgcgagtgc ggccgctgaa cagcagagaa gaatcacttg gagaaactgc ccaagtttac 180

tggaaaactg acaataatgt catttatcaa gttgatggaa gtaaatcctt caattttgat 240

cgtgtctttc atggtaatga aactaccaaa aatgtgtatg aagaaatagc agcaccaatc 300

atcgattctg ccatacaagg ctacaatggt actatatttg cctatggaca gactgcttca 360

ggaaaaacat ataccatgat gggttcagaa gatcatttgg gagttatacc cagggcaatt 420

catgacattt tccaaaaaat taagaagttt cctgataggg aatttctctt acgtgtatct 480

tacatggaaa tatacaatga aaccattaca gatttactct gtggcactca aaaaatgaaa 540

cctttaatta ttcgagaaga tgtcaatagg aatgtgtatg ttgctgatct cacagaagaa 600

gttgtatata catcagaaat ggctttgaaa tggattacaa agggagaaaa gagcaggcat 660

tatggagaaa caaaaatgaa tcaaagaagc agtcgttctc ataccatctt taggatgatt 720

ttggaaagca gagagaaggg tgaaccttct aattgtgaag gatctgttaa ggtatcccat 780

ttgaatttgg ttgatcttgc aggcagtgaa agagctgctc aaacaggcgc tgcaggtgtg 840

cggctcaagg aaggctgtaa tataaatcga agcttattta ttttgggaca agtgatcaag 900

aaacttagtg atggacaagt tggtggtttc ataaattatc gagatagcaa gttaacacga 960

attctccaga attccttggg aggaaatgca aagacacgta ttatctgcac aattactcca 1020

gtatcttttg atgaaacact tactgctctc cagtttgcca gtactgctaa atatatgaag 1080

aatactcctt atgttaatga ggtatcaact gatgaagctc tcctgaaaag gtatagaaaa 1140

gaaataatgg atcttaaaaa acaattagag gaggtttctt tagagacgcg ggctcaggca 1200

atggaaaaag accaattggc ccaacttttg gaagaaaaag atttgcttca gaaagtacag 1260

aatgagaaaa ttgaaaactt aacacggatg ctggtgacct cttcttccct cacgttgcaa 1320

caggaattaa aggctaaaag aaaacgaaga gttacttggt gccttggcaa aattaacaaa 1380

atgaagaact caaactatgc agatcaattt aatataccaa caaatataac aacaaaaaca 1440

cataagcttt ctataaattt attacgagaa attgatgaat ctgtctgttc agagtctgat 1500

gttttcagta acactcttga tacattaagt gagatagaat ggaatccagc aacaaagcta 1560

ctaaatcagg agaatataga aagtgagttg aactcacttc gtgctgacta tgataatctg 1620
gtattagact atgaacaact acgaacagaa aaagaagaaa tggaattgaa attaaaagaa 1680
aagaatgatt tggatgaatt tgaggctcta gaaagaaaaa ctaaaaaaga tcaagagatg 1740
caactaattc atgaaatttc gaacttaaag aatttagtta agcatgcaga agtatataat 1800
caagatcttg agaatgaact cagttcaaaa gtagagctgc ttagagaaaa ggaagaccag 1860
attaagaagc tacaggaata catagactct caaaagctag aaaatataaa aatggacttg 1920
tcatactcat tggaaagcat tgaagaccca aaacaaatga agcagactct gtttgatgct 1980
gaaactgtag cccttgatgc caagagagaa tcagcctttc ttagaagtga aaatctggag 2040
ctgaaggaga aaatgaaaga acttgcaact acatacaagc aaatggaaaa tgatattcag 2100
ttatatcaaa gccagttgga ggcaaaaaag aaaatgcaag ttgatctgga gaaagaatta 2160
caatctgctt ttaatgagat aacaaaactc acctccctta tagatggcaa agttccaaaa 2220
gatttgctct gtaatttgga attggaagga aagattactg atcttcagaa agaactaaat 2280
aaagaagttg aagaaaatga agctttgcgg gaagaagtca ttttgctttc agaattgaaa 2340
tctttacctt ctgaagtaga aaggctgagg aaagagatac aagacaaatc tgaagagctc 2400
catataataa catcagaaaa agataaattg ttttctgaag tagttcataa ggagagtaga 2460
gttcaaggtt tacttgaaga aattgggaaa acaaaagatg acctagcaac tacacagtcg 2520
aattataaaa gcactgatca agaattccaa aatttcaaaa cccttcatat ggactttgag 2580
caaaagtata agatggtcct tgaggagaat gagagaatga atcaggaaat agttaatctc 2640
tctaaagaag cccaaaaatt tgattcgagt ttgggtgctt tgaagaccga gctttcttac 2700
aagacccaag aacttcagga gaaaacacgt gaggttcaag aaagactaaa tgagatggaa 2760
cagctgaagg aacaattaga aaatagagat tctacgctgc aaactgtaga aagggagaaa 2820
acactgatta ctgagaaact gcagcaaact ttagaagaag taaaaacttt aactcaagaa 2880
aaagatgatc taaaacaact ccaagaaagc ttgcaaattg agagggacca actcaaaagt 2940
gatattcacg atactgttaa catgaatata gatactcaag aacaattacg aaatgctctt 3000
gagtctctga aacaacatca agaaacaatt aatacactaa aatcgaaaat ttctgaggaa 3060
gtttccagga atttgcatat ggaggaaaat acaggagaaa ctaaagatga atttcagcaa 3120
aagatggttg gcatagataa aaaacaggat ttggaagcta aaaataccca aacactaact 3180
gcagatgtta aggataatga gataattgag caacaaagga agatattttc tttaatacag 3240
gagaaaaatg aactccaaca aatgttagag agtgttatag cagaaaagga acaattgaag 3300
actgacctaa aggaaaatat tgaaatgacc attgaaaacc aggaagaatt aagacttctt 3360
ggggatgaac ttaaaaagca acaagagata gttgcacaag aaaagaacca tgccataaag 3420
aaagaaggag agctttctag gacctgtgac agactggcag aagttgaaga aaaactaaag 3480
gaaaagagcc agcaactcca agaaaaacag caacaacttc ttaatgtaca agaagagatg 3540
agtgagatgc agaaaaagat taatgaaata gagaatttaa agaatgaatt aaagaacaaa 3600
gaattgacat tggaacatat ggaaacagag aggcttgagt tggctcagaa acttaatgaa 3660

aattatgagg aagtgaaatc tataaccaaa gaaagaaaag ttctaaagga attacagaag 3720
tcatttgaaa cagagagaga ccaccttaga ggatatataa gagaaattga agctacaggc 3780
ctacaaacca aagaagaact aaaaattgct catattcacc taaaagaaca ccaagaaact 3840
attgatgaac taagaagaag cgtatctgag aagacagctc aaataataaa tactcaggac 3900
ttagaaaaat cccataccaa attacaagaa gagatcccag tgcttcatga ggaacaagag 3960
ttactgccta atgtgaaaga agtcagtgag actcaggaaa caatgaatga actggagtta 4020
ttaacagaac agtccacaac caaggactca acaacactgg caagaataga aatggaaagg 4080
ctcaggttga atgaaaaatt tcaagaaagt caggaagaga taaaatctct aaccaaggaa 4140
agagacaacc ttaaaacgat aaaagaagcc cttgaagtta aacatgacca gctgaaagaa 4200
catattagag aaactttggc taaaatccag gagtctcaaa gcaaacaaga acagtcctta 4260
aatatgaaag aaaaagacaa tgaaactacc aaaatcgtga gtgagatgga gcaattcaaa 4320
cccaaagatt cagcactact aaggatagaa atagaaatgc tcggattgtc caaaagactt 4380
caagaaagtc atgatgaaat gaaatctgta gctaaggaga aagatgacct acagaggctg 4440
caagaagttc ttcaatctga aagtgaccag ctcaaagaaa acataaaaga aattgtagct 4500
aaacacctgg aaactgaaga ggaacttaaa gttgctcatt gttgcctgaa agaacaagag 4560
gaaactatta atgagttaag agtgaatctt tcagagaagg aaactgaaat atcaaccatt 4620
caaaagcagt tagaagcaat caatgataaa ttacagaaca agatccaaga gatttatgag 4680
aaagaggaac aatttaatat aaaacaaatt agtgaggttc aggaaaaagt gaatgaactg 4740
aaacaattca aggagcatcg caaagccaag gattcagcac tacaaagtat agaaagtaag 4800
atgctcgagt tgaccaacag acttcaagaa agtcaagaag aaatacaaat tatgattaag 4860
gaaaaagagg aaatgaaaag agtacaggag gcccttcaga tagagagaga ccaactgaaa 4920
gaaaacacta aagaaattgt agctaaaatg aaagaatctc aagaaaaaga atatcagttt 4980
cttaagatga cagctgtcaa tgagactcag gagaaaatgt gtgaaataga acacttgaag 5040
gagcaatttg agacccagaa gttaaacctg gaaaacatag aaacggagaa tataaggttg 5100
actcagatac tacatgaaaa ccttgaagaa atgagatctg taacaaaaga aagagatgac 5160
cttaggagtg tggaggagac tctcaaagta gagagagacc agctcaagga aaaccttaga 5220
gaaactataa ctagagacct agaaaaacaa gaggagctaa aaattgttca catgcatctg 5280
aaggagcacc aagaaactat tgataaacta agagggattg tttcagagaa aacaaatgaa 5340
atatcaaata tgcaaaagga cttagaacac tcaaatgatg ccttaaaagc acaggatctg 5400
aaaatacaag aggaactaag aattgctcac atgcatctga aagagcagca ggaaactatt 5460
gacaaactca gaggaattgt ttctgagaag acagataaac tatcaaatat gcaaaaagat 5520
ttagaaaatt caaatgctaa attacaagaa aagattcaag aacttaaggc aaatgaacat 5580
caacttatta cgttaaaaaa agatgtcaat gagacacaga aaaaagtgtc tgaaatggag 5640
caactaaaga aacaaataaa agaccaaagc ttaactctga gtaaattaga aatagagaat 5700

ttaaatttgg ctcagaaact tcatgaaaac cttgaagaaa tgaaatctgt aatgaaagaa 5760
agagataatc taagaagagt agaggagaca ctcaaactgg agagagacca actcaaggaa 5820
agcctgcaag aaaccaaagc tagagatctg gaaatacaac aggaactaaa aactgctcgt 5880
atgctatcaa aagaacacaa agaaactgtt gataaactta gagaaaaaat ttcagaaaag 5940
acaattcaaa tttcagacat tcaaaaggat ttagataaat caaaagatga attacagaaa 6000
aagatccaag aacttcagaa aaaagaactt caactgctta gagtgaaaga agatgtcaat 6060
atgagtcata aaaaaattaa tgaaatggaa cagttgaaga agcaatttga ggcccaaaac 6120
ttatctatgc aaagtgtgag aatggataac ttccagttga ctaagaaact tcatgaaagc 6180
cttgaagaaa taagaattgt agctaaagaa agagatgagc taaggaggat aaaagaatct 6240
ctcaaaatgg aaagggacca attcatagca accttaaggg aaatgatagc tagagaccga 6300
cagaaccacc aagtaaaacc tgaaaaaagg ttactaagtg atggacaaca gcaccttacg 6360
gaaagcctga gagaaaagtg ctctagaata aaagagcttt tgaagagata ctcagagatg 6420
gatgatcatt atgagtgctt gaatagattg tctcttgact tggagaagga aattgaattc 6480
caaaaagagc tttcaatgag agttaaagca aacctctcac ttccctattt acaaaccaaa 6540
cacattgaaa aactttttac tgcaaaccag agatgctcca tggaattcca cagaatcatg 6600
aagaaactga agtatgtgtt aagctatgtt acaaaaataa aagaagaaca acatgaatcc 6660
atcaataaat ttgaaatgga ttttattgat gaagtggaaa agcaaaagga attgctaatt 6720
aaaatacagc accttcaaca agattgtgat gtaccatcca gagaattaag ggatctcaaa 6780
ttgaaccaga atatggatct acatattgag gaaattctca aagatttctc agaaagtgag 6840
ttccctagca taaagactga atttcaacaa gtactaagta ataggaaaga aatgacacag 6900
tttttggaag agtggttaaa tactcgtttt gatatagaaa agcttaaaaa tggcatccag 6960
aaagaaaatg ataggatttg tcaagtgaat aacttcttta ataacagaat aattgccata 7020
atgaatgaat caacagagtt tgaggaaaga agtgctacca tatccaaaga gtgggaacag 7080
gacctgaaat cactgaaaga gaaaaatgaa aaactattta aaaactacca aacattgaag 7140
acttccttgg catctggtgc ccaggttaat cctaccacac aagacaataa gaatcctcat 7200
gttacatcaa gagctacaca gttaaccaca gagaaaattc gagagctgga aaattcactg 7260
catgaagcta aagaaagtgc tatgcataag gaaagcaaga ttataaagat gcagaaagaa 7320
cttgaggtga ctaatgacat aatagcaaaa cttcaagcca aagttcatga atcaaataaa 7380
tgccttgaaa aaacaaaaga gacaattcaa gtacttcagg acaaagttgc tttaggagct 7440
aagccatata aagaagaaat tgaagatctc aaaatgaagc ttgtgaaaat agacctagag 7500
aaaatgaaaa atgccaaaga atttgaaaag gaaatcagtg ctacaaaagc cactgtagaa 7560
tatcaaaagg aagttataag gctattgaga gaaaatctca gaagaagtca acaggcccaa 7620
gatacctcag tgatatcaga acatactgat cctcagcctt caaataaacc cttaacttgt 7680
ggaggtggca gcggcattgt acaaaacaca aaagctctta ttttgaaaag tgaacatata 7740
aggctagaaa aagaaatttc taagttaaag cagcaaaatg aacagctaat aaaacaaaag 7800

aatgaattgt taagcaataa tcagcatctt tccaatgagg tcaaaacttg gaaggaaaga 7860

acccttaaaa gagaggctca caaacaagta acttgtgaga attctccaaa gtctcctaaa 7920

gtgactggaa cagcttctaa aaagaaacaa attacaccct ctcaatgcaa ggaacggaat 7980

ttacaagatc ctgtgccaaa ggaatcacca aaatcttgtt tttttgatag ccgatcaaag 8040

tctttaccat cacctcatcc agttcgctat tttgataact caagtttagg cctttgtcca 8100

gaggtgcaaa atgcaggagc agagagtgtg gattctcagc caggtccttg gcacgcctcc 8160

tcaggcaagg atgtgcctga gtgcaaaact cagtagactc ctctttgtca cttctctgga 8220

gatccagcat tccttatttg gaaatgactt tgtttatgtg tctatccctg gtaatgatgt 8280

tgtagtgcag cttaatttca attcagtctt tactttgcca ctagagttga aagataaggg 8340

aacaggaaat gaatgcattg tggtaattta gaatggtgat agcaatacct tcttcttgca 8400

tatggtaata cttttaaaag ttgaattgtt ttatttattt gtatattttg taaagaataa 8460

agttattgaa agaaatgtaa agttatctac atgacttagc atattccaaa gcataataca 8520

tacattaata taaaacatca ttttattaac aaaattgtaa atgtttttaa taccttacac 8580

attcaataaa tgtttagtag ttctgaatca ccaaaaaaaa aaaaaaaaaa 8630

<210> 15
<211> 2972
<212> DNA
<213> Homo sapiens

<400> 15
tcttcggacc taggctgccc tgccgtcatg tcgcaaggga tcctttctcc gccagcgggc 60

ttgctgtccg atgacgatgt cgtagtttct cccatgtttg agtccacagc tgcagatttg 120

gggtctgtgg tacgcaagaa cctgctatca gactgctctg tcgtctctac ctccctagag 180

gacaagcagc aggttccatc tgaggacagt atggagaagg tgaaagtata cttgagggtt 240

aggcccttgt taccttcaga gttggaacga caggaagatc agggttgtgt ccgtattgag 300

aatgtggaga cccttgttct acaagcaccc aaggactcgt ttgccctgaa gagcaatgaa 360

cggggaattg gccaagccac acacaggttc accttttccc agatctttgg gccagaagtg 420

ggacaggcat ccttcttcaa cctaactgtg aaggagatgg taaaggatgt actcaaaggg 480

cagaactggc tcatctatac atatggagtc actaactcag ggaaaaccca cacgattcaa 540

ggtaccatca aggatggagg gattctcccc cggtccctgg cgctgatctt caatagcctc 600

caaggccaac ttcatccaac acctgatctg aagcccttgc tctccaatga ggtaatctgg 660

ctagacagca agcagatccg acaggaggaa atgaagaagc tgtccctgct aaatggaggc 720

ctccaagagg aggagctgtc cacttccttg aagaggagtg tctacatcga aagtcggata 780

ggtaccagca ccagcttcga cagtggcatt gctgggctct cttctatcag tcagtgtacc 840

agcagtagcc agctggatga aacaagtcat cgatgggcac agccagacac tgccccacta 900

cctgtcccgg caaacattcg cttctccatc tggatctcat tctttgagat ctacaacgaa 960

ctgctttatg acctattaga accgcctagc caacagcgca agaggcagac tttgcggcta 1020

tgcgaggatc aaaatggcaa tccctatgtg aaagatctca actggattca tgtgcaagat 1080
gctgaggagg cctggaagct cctaaaagtg ggtcgtaaga accagagctt tgccagcacc 1140
cacctcaacc agaactccag ccgcagtcac agcatcttct caatcaggat cctacacctt 1200
cagggggaag gagatatagt ccccaagatc agcgagctgt cactctgtga tctggctggc 1260
tcagagcgct gcaaagatca gaagagtggt gaacggttga aggaagcagg aaacattaac 1320
acctctctac acaccctggg ccgctgtatt gctgcccttc gtcaaaacca gcagaaccgg 1380
tcaaagcaga acctggttcc cttccgtgac agcaagttga ctcgagtgtt ccaaggtttc 1440
ttcacaggcc gaggccgttc ctgcatgatt gtcaatgtga atccctgtgc atctacctat 1500
gatgaaactc ttcatgtggc caagttctca gccattgcta gccagcttgt gcatgcccca 1560
cctatgcaac tgggattccc atccctgcac tcgttcatca aggaacatag tcttcaggta 1620
tcccccagct tagagaaagg ggctaaggca gacacaggcc ttgatgatga tattgaaaat 1680
gaagctgaca tctccatgta tggcaaagag gagctcctac aagttgtgga agccatgaag 1740
acactgcttt tgaaggaacg acaggaaaag ctacagctgg agatgcatct ccgagatgaa 1800
atttgcaatg agatggtaga acagatgcaa cagcgggaac agtggtgcag tgaacatttg 1860
gacacccaaa aggaactatt ggaggaaatg tatgaagaaa aactaaatat cctcaaggag 1920
tcactgacaa gtttttacca agaagagatt caggagcggg atgaaaagat tgaagagcta 1980
gaagctctct tgcaggaagc cagacaacag tcagtggccc atcagcaatc agggtctgaa 2040
ttggccctac ggcggtcaca aaggttggca gcttctgcct ccacccagca gcttcaggag 2100
gttaaagcta aattacagca gtgcaaagca gagctaaact ctaccactga agagttgcat 2160
aagtatcaga aaatgttaga accaccaccc tcagccaagc ccttcaccat tgatgtggac 2220
aagaagttag aagagggcca gaagaatata aggctgttgc ggacagagct tcagaaactt 2280
ggtgagtctc tccaatcagc agagagagct tgttgccaca gcactggggc aggaaaactt 2340
cgtcaagcct tgaccacttg tgatgacatc ttaatcaaac aggaccagac tctggctgaa 2400
ctgcagaaca acatggtgct agtgaaactg gaccttcgga agaaggcagc atgtattgct 2460
gagcagtatc atactgtgtt gaaactccaa ggccaggttt ctgccaaaaa gcgccttggt 2520
accaaccagg aaaatcagca accaaaccaa caaccaccag ggaagaaacc attccttcga 2580
aatttacttc cccgaacacc aacctgccaa agctcaacag actgcagccc ttatgcccgg 2640
atcctacgct cacggcgttc ccctttactc aaatctgggc cttttggcaa aaagtactaa 2700
ggctgtgggg aaagagaaga gcagtcatgg ccctgaggtg ggtcagctac tctcctgaag 2760
aaataggtct cttttatgct ttaccatata tcaggaatta tatccaggat gcaatactca 2820
gacactagct tttttctcac ttttgtatta taaccaccta tgtaatctca tgttgttgtt 2880
tttttttatt tacttatatg atttctatgc acacaaaaac agttatatta aagatattat 2940
tgttcacatt ttttattgaa aaaaaaaaaa aa 2972

<210> 16

<211>  2115
<212>  DNA
<213>  Homo sapiens

<400>  16
ggtcaacgcc tgcggctgtt gatattcttg ctcagaggcc gtaactttgg ccttctgctc      60

agggaagact ctgagtccga cgttggccta cccagtcgga aggcagagct gcaatctagt     120

taactacctc ctttccccta gatttccttt cattctgctc aagtcttcgc ctgtgtccga     180

tccctatcta ctttctctcc tcttgtagca agcctcagac tccaggcttg agctaggttt     240

tgtttttctc ctggtgagaa ttcgaagacc atgtctacgg aactcttctc atccacaaga     300

gaggaaggaa gctctggctc aggacccagt tttaggtcta atcaaaggaa aatgttaaac     360

ctgctcctgg agagagacac ttcctttacc gtctgtccag atgtccctag aactccagtg     420

ggcaaatttc ttggtgattc tgcaaaccta agcattttgt ctggaggaac cccaaaatgt     480

tgcctcgatc tttcgaatct tagcagtggg gagataactg ccactcagct taccacttct     540

gcagaccttg atgaaactgg tcacctggat tcttcaggac ttcaggaagt gcatttagct     600

gggatgaatc atgaccagca cctaatgaaa tgtagcccag cacagcttct ttgtagcact     660

ccgaatggtt tggaccgtgg ccatagaaag agagatgcaa tgtgtagttc atctgcaaat     720

aaagaaaatg acaatggaaa cttggtggac agtgaaatga aatatttggg cagtcccatt     780

actactgttc caaaattgga taaaaatcca aacctaggag aagaccaggc agaagagatt     840

tcagatgaat taatggagtt ttccctgaaa gatcaagaag caaaggtgag cagaagtggc     900

ctatatcgct ccccgtcgat gccagagaac ttgaacaggc caagactgaa gcaggtggaa     960

aaattcaagg acaacacaat accagataaa gttaaaaaaa agtatttttc tggccaagga    1020

aagctcagga agggcttatg tttaaagaag acagtctctc tgtgtgacat tactatcact    1080

cagatgctgg aggaagattc taaccagggg cacctgattg gtgatttttc caaggtatgt    1140

gcgctgccaa ccgtgtcagg gaaacaccaa gatctgaagt atgtcaaccc agaaacagtg    1200

gctgccttac tgtcggggaa gttccagggt ctgattgaga agttttatgt cattgattgt    1260

cgctatccat atgagtatct gggaggacac atccagggag ccttaaactt atatagtcag    1320

gaagaactgt ttaacttctt tctgaagaag cccatcgtcc ctttggacac ccagaagaga    1380

ataatcatcg tgttccactg tgaattctcc tcagagaggg gcccccgaat gtgccgctgt    1440

ctgcgtgaag aggacaggtc tctgaaccag tatcctgcat tgtactaccc agagctatat    1500

atccttaaag gcggctacag agacttcttt ccagaatata tggaactgtg tgaaccacag    1560

agctactgcc ctatgcatca tcaggaccac aagactgagt tgctgaggtg tcgaagccag    1620

agcaaagtgc aggaagggga gcggcagctg cgggagcaga ttgcccttct ggtgaaggac    1680

atgagcccat gataacattc cagccactgg ctgctaacaa gtcaccaaaa agacactgca    1740

gaaaccctga gcagaaagag gccttctgga tggccaaacc caagattatt aaaagatgtc    1800

tctgcaaacc aacaggctac caacttgtat ccaggcctgg gaatggatta ggtttcagca    1860

gagctgaaag ctggtggcag agtcctggag ctggctctat aaggcagcct tgagttgcat    1920

agagatttgt attggttcag ggaactctgg cattcctttt cccaactcct catgtcttct 1980

cacaagccag ccaactcttt ctctctgggc ttcgggctat gcaagagcgt tgtctacctt 2040

ctttctttgt attttccttc tttgtttccc cctctttctt ttttaaaaat ggaaaaataa 2100

acactacaga atgag 2115

<210> 17
<211> 2319
<212> DNA
<213> Homo sapiens

<400> 17
gtggagtttg aattgggtgg cggttgactg tagagccgct ctctctcact ggcacagcga 60

ggttttgctc agcccttgtc tcgggaccgc agcctccgcc gagcgccatg gctcctagga 120

agggcagtag tcgggtggcc aagaccaact ccttacggag gcggaagctc gcctcctttc 180

tgaaagactt cgaccgtgaa gtggaaatac gaatcaagca aattgagtca gacaggcaga 240

acctcctcaa ggaggtggat aacctctaca acatcgagat cctgcggctc cccaaggctc 300

tgcgcgagat gaactggctt gactacttcg cccttggagg aaacaaacag gccctggaag 360

aggcggcaac agctgacctg gatatcaccg aaataaacaa actaacagca gaagctattc 420

agacacccct gaaatctgcc aaaacacgaa aggtaataca ggtagatgaa atgatagtgg 480

aagaggaaga agaagaagaa aatgaacgta agaatcttca aactgcaaga gtcaaaaggt 540

gtcctccatc caagaagaga actcagtcca tacaaggaaa aggaaaaggg aaaaggtcaa 600

gccgtgctaa cactgttacc ccagccgtgg gccgattgga ggtgtccatg gtcaaaccaa 660

ctccaggcct gacacccagg tttgactcaa gggtcttcaa gacccctggc ctgcgtactc 720

cagcagcagg agagcggatt tacaacatct cagggaatgg cagccctctt gctgacagca 780

aagagatctt cctcactgtg ccagtgggcg gcggagagag cctgcgatta ttggccagtg 840

acttgcagag gcacagtatt gcccagctgg atccagaggc cttgggaaac attaagaagc 900

tctccaaccg tctcgcccaa atctgcagca gcatacggac ccacaaatga gacaccaaag 960

ttgacaggat ggacttttaa tgggcacttc tgggaccctg aagagacttc ttcccttcag 1020

gcttattgtt tgagtgtgaa gttccagagc aaggagccat gttcctctaa gggaattcag 1080

gaattcagac gtgctagtcc cacaccagtt aggtagagct gtctgttcac cctcccatcc 1140

cagctgatcc cagtcactgc ttgctggggc catgccatgg aagcttccca tcagtctccc 1200

agctgaatcc tccctgctct ctgagctgct gccttttgcc tcctgcaact caacatcctc 1260

ttcaccctgc cctgcctgca gttgaggggg cgaagaagaa ccctgtgttc tcaggaagac 1320

tgcctccacc accgctaccc agagaacctc tgcatctggc atttctgctc tctatgcttg 1380

agaccgggag gtttaggctc agataagtga gctctgggcc atgagagggt aggtccagaa 1440

ggtgggggga actgtacaga tcagcagagc aggacagttg gcagcagtga cctcagtagg 1500

gaacatgtcc gtctaccctc tcgcactcat gacacctccc cctaccagcc ctcctcttcc 1560

tcctcctcct cctcctgtgg gaggtggtca gtgggactta gggatctttc acctgctgtg 1620

cccagtagtt ctgaagtctg cttgtggagc agtgttttat gtttatccct gtttactgaa 1680
gaccaaatac tggtttggag acaacttcca tgtcttgctc ttctacctcc ctagttagtg 1740
gaaatttgga taagggaact gtagggccca gattctggag gttttatgtc attggccaca 1800
gaataactgt ctctaagcta tccatggtcc agtggtccct gccaagtctg tagacttcag 1860
agagcacttc tctcttatgg ggttcatggg aacaggggtg ggtgtgactt gcttggtggc 1920
ctcattccat gtgtgcctgt gcctggggca tggactttgt taagcagagt cagcagtgag 1980
gtcctcattc tccagccagc ctctctgccc tggagaatca tgtgctatgt tctaagaatt 2040
tgagaactag agtcctcatc cccaggcttg aaggcacatg gctttctcat gtagggctct 2100
ctgtggtatt tgttattatt ttgcaacaag accattttag taaaacagtc ctgttcaagt 2160
tgtattcttt taagttcttt tattctcctt tccctgagat ttttgtatat attgttctga 2220
gtaatggtat ctttgagctg attgttctaa tcagagctgg tacctacttt caataaattc 2280
tggttttgtg ttttcttttg taaaaaaaaa aaaaaaaaa 2319

<210> 18
<211> 4908
<212> DNA
<213> Homo sapiens

<400> 18
acctgcgtgc agtcggtcct ccaggccacg cagcgcccga gagtaccagg gagactccgg 60
cccctgtcgg cgccaagccc ctccgcccct cacagcgccc aggtccgcgg ccgggccttg 120
attttttggc ggggaccgtc atggcgtcgc agccaaattc gtctgcgaag aagaaagagg 180
agaaggggaa gaacatccag gtggtggtga gatgcagacc atttaatttg gcagagcgga 240
aagctagcgc ccattcaata gtagaatgtg atcctgtacg aaaagaagtt agtgtacgaa 300
ctggaggatt ggctgacaag agctcaagga aaacatacac ttttgatatg gtgtttggag 360
catctactaa acagattgat gtttaccgaa gtgttgtttg tccaattctg gatgaagtta 420
ttatgggcta taattgcact atctttgcgt atggccaaac tggcactgga aaaactttta 480
caatggaagg tgaaaggtca cctaatgaag agtatacctg ggaagaggat cccttggctg 540
gtataattcc acgtaccctt catcaaattt ttgagaaact tactgataat ggtactgaat 600
tttcagtcaa agtgtctctg ttggagatct ataatgaaga gctttttgat cttcttaatc 660
catcatctga tgtttctgag agactacaga tgtttgatga tccccgtaac aagagaggag 720
tgataattaa aggtttagaa gaaattacag tacacaacaa ggatgaagtc tatcaaattt 780
tagaaaaggg ggcagcaaaa aggacaactg cagctactct gatgaatgca tactctagtc 840
gttcccactc agttttctct gttacaatac atatgaaaga aactacgatt gatggagaag 900
agcttgttaa aatcggaaag ttgaacttgg ttgatcttgc aggaagtgaa aacattggcc 960
gttctggagc tgttgataag agagctcggg aagctggaaa tataaatcaa tccctgttga 1020
ctttgggaag ggtcattact gcccttgtag aaagaacacc tcatgttcct tatcgagaat 1080
ctaaactaac tagaatcctc caggattctc ttggagggcg tacaagaaca tctataattg 1140

caacaatttc tcctgcatct ctcaatcttg aggaaactct gagtacattg gaatatgctc 1200
atagagcaaa gaacatattg aataagcctg aagtgaatca gaaactcacc aaaaaagctc 1260
ttattaagga gtatacggag gagatagaac gtttaaaacg agatcttgct gcagcccgtg 1320
agaaaaatgg agtgtatatt tctgaagaaa attttagagt catgagtgga aaattaactg 1380
ttcaagaaga gcagattgta gaattgattg aaaaaattgg tgctgttgag gaggagctga 1440
atagggttac agagttgttt atggataata aaaatgaact tgaccagtgt aaatctgacc 1500
tgcaaaataa aacacaagaa cttgaaacca ctcaaaaaca tttgcaagaa actaaattac 1560
aacttgttaa agaagaatat atcacatcag ctttggaaag tactgaggag aaacttcatg 1620
atgctgccag caagctgctt aacacagttg aagaaactac aaaagatgta tctggtctcc 1680
attccaaact ggatcgtaag aaggcagttg accaacacaa tgcagaagct caggatattt 1740
ttggcaaaaa cctgaatagt ctgtttaata atatggaaga attaattaag gatggcagct 1800
caaagcaaaa ggccatgcta gaagtacata agaccttatt tggtaatctg ctgtcttcca 1860
gtgtctctgc attagatacc attactacag tagcacttgg atctctcaca tctattccag 1920
aaaatgtgtc tactcatgtt tctcagattt ttaatatgat actaaaagaa caatcattag 1980
cagcagaaag taaaactgta ctacaggaat tgattaatgt actcaagact gatcttctaa 2040
gttcactgga aatgatttta tccccaactg tggtgtctat actgaaaatc aatagtcaac 2100
taaagcatat tttcaagact tcattgacag tggccgataa gatagaagat caaaaaaagg 2160
aactagatgg ctttctcagt atactgtgta acaatctaca tgaactacaa gaaaatacca 2220
tttgttcctt ggttgagtca caaaagcaat gtggaaacct aactgaagac ctgaagacaa 2280
taaagcagac ccattcccag gaactttgca agttaatgaa tctttggaca gagagattct 2340
gtgctttgga ggaaaagtgt gaaaatatac agaaaccact tagtagtgtc caggaaaata 2400
tacagcagaa atctaaggat atagtcaaca aaatgacttt tcacagtcaa aaattttgtg 2460
ctgattctga tggcttctca caggaactca gaaattttaa ccaagaaggt acaaaattgg 2520
ttgaagaatc tgtgaaacac tctgataaac tcaatggcaa cctggaaaaa atatctcaag 2580
agactgaaca gagatgtgaa tctctgaaca caagaacagt ttatttttct gaacagtggg 2640
tatcttcctt aaatgaaagg gaacaggaac ttcacaactt attggaggtt gtaagccaat 2700
gttgtgaggc ttcaagttca gacatcactg agaaatcaga tggacgtaag gcagctcatg 2760
agaaacagca taacattttt cttgatcaga tgactattga tgaagataaa ttgatagcac 2820
aaaatctaga acttaatgaa accataaaaa ttggtttgac taagcttaat tgctttctgg 2880
aacaggatct gaaactggat atcccaacag gtacgacacc acagaggaaa agttatttat 2940
acccatcaac actggtaaga actgaaccac gtgaacatct ccttgatcag ctgaaaagga 3000
aacagcctga gctgttaatg atgctaaact gttcagaaaa caacaaagaa gagacaattc 3060
cggatgtgga tgtagaagag gcagttctgg ggcagtatac tgaagaacct ctaagtcaag 3120
agccatctgt agatgctggt gtggattgtt catcaattgg cggggttcca tttttccagc 3180
ataaaaaatc acatggaaaa gacaaagaaa acagaggcat taacacactg gagaggtcta 3240

aagtggaaga aactacagag cacttggtta caaagagcag attacctctg cgagcccaga 3300

tcaaccttta attcacttgg gggttggcaa ttttattttt aaagaaaact taaaaataaa 3360

acctgaaacc ccagaacttg agccttgtgt atagatttta aaagaatata tatatcagcc 3420

gggcgcggtg gctcatgcct gtaatcccag cactttggga ggctgaggcg ggtggattgc 3480

ttgagcccag gagtttgaga ccagcctggc caacgtggca aaacctcgtc tctgttaaaa 3540

attagccggg cgtggtggca cactcctgta atcccagcta ctggggaggc tgaggcacga 3600

gaatcacttg aacccaggaa gcggggttgc agtgagccaa aggtacacca ctacactcca 3660

gcctgggcaa cagagcaaga ctcggtctca aaaacaaaat ttaaaaaaga tataaggcag 3720

tactgtaaat tcagttgaat tttgatatct acccattttt ctgtcatccc tatagttcac 3780

tttgtattaa attgggtttc atttgggatt tgcaatgtaa atacgtattt ctagttttca 3840

tataaagtag ttcttttata acaaatgaaa agtatttttc ttgtatatta ttaagtaatg 3900

aatatataag aactgtactc ttctcagctt gagcttaaca taggtaaata tcaccaacat 3960

ctgtccttag aaaggaccat ctcatgtttt ttttcttgct atgacttgtg tattttcttg 4020

catcctccct agacttccct atttcgcttt ctcctcggct cactttctcc ctttttattt 4080

ttcaccaaac catttgtaga gctacaaaac ctatcctttc ttattttcag tagtcagaat 4140

tttatctaga aatcttttaa caccttttta gtggttattt ctaaaatcac tgtcaacaat 4200

aaatctaacc ctagttgtat ccctccttta agtatttaaa acttgttgcc ccaaatgtga 4260

aagcatttaa ttcctttaag aggcctaact cattcaccct gacagagttc acaaaaagcc 4320

cactttagag tatacattgc tattatggga gaccacccag acatctgact aatggctctg 4380

tgccacactc caagacctgt gccttttaga gaagctcaca atgatttaag gactgtttga 4440

aacttccaat tatgtctata atttatattc ttttgtttac atgatgaaac tttttgttgt 4500

tgcttgtttg tatataatac aatgtgtaca tgtatctttt tctcgattca aatcttaacc 4560

cttaggactc tggtattttt gatctggcaa ccatatttct ggaagttgag atgtttcagc 4620

ttgaagaacc aaaacagaag gaatatgtac aaagaataaa ttttctgctc acgatgagtt 4680

tagtgtgtaa agtttagaga catctgactt tgatagctaa attaaaccaa accctattga 4740

agaattgaat atatgctact tcaagaaact aaattgatct cgtagaatta tcttaataaa 4800

ataatggcta taatttctct gcaaaatcag atgtcagcat aagcgatgga taatacctaa 4860

taaactgccc tcagtaaatc catggttaat aaatgtggtt tctacatt 4908

<210> 19
<211> 4166
<212> DNA
<213> Homo sapiens

<400> 19
cggcgcgggt gttgagagcg gtgtggtagg tgttgtagcc gctatggtga agttcgcttt 60

gtagcggccc cggctagaga gttggcctgt tccctgcctt tgtgacccgg aggagctttt 120

gggggtgcgt caagcccctg gcctgaggca gcgaactggt ttgtggcctg tttgattcct 180

gtcagaggtt tgctgaccca agacagtatc gaaaatgcat attaagtcaa ttattctaga 240
gggattcaag tcctatgctc agaggaccga agtcaatggt tttgaccccc tcttcaatgc 300
tatcactggc ttaaatggta gtgggaaatc caacatattg gactccatct gctttttact 360
gggcatctcc aacctgtctc aggttcgggc ttctaattta caagatttag tttacaaaaa 420
tgggcaggct ggtattacca aagcctctgt gtcaatcact tttgataatt ctgacaaaaa 480
gcaaagtcct ttaggatttg aggttcatga tgaaatcaca gtaacaaggc aggtggttat 540
tggtggtaga aataaatatt taatcaatgg agtcaatgcc aacaacacca gagtacagga 600
tctcttctgt tctgttggcc ttaatgttaa caaccctcac tttctcatca tgcagggccg 660
aattacaaaa gtattgaata tgaaaccacc agagatttta tccatgatag aagaagcagc 720
tggaaccagg atgtatgaat acaaaaaaat agctgcacag aaaactatag aaaaaaagga 780
ggctaagctg aaagaaatta agacgatact tgaagaagag attactccaa ccattcaaaa 840
attaaaagag gaaagatcgt cctacttgga gtaccaaaaa gtaatgagag aaatagaaca 900
tttgagtcgt ttatatattg cttatcagtt tttgctggct gaagatacca aagtacgctc 960
agctgaggaa ttaaaagaaa tgcaagataa agttataaag cttcaggaag aattgtctga 1020
gaatgataaa aaaataaaag cacttaatca tgaaatagaa gaattggaaa aaagaaaaga 1080
taaggaaact ggagttatac ttcgatcttt agaagatgct cttgcagagg ctcagcgagt 1140
taatactaaa tctcaaagcg catttgatct caagaagaaa aatctggcat gtgaggaaag 1200
caaacgcaaa gagctggaaa aaaatatggt tgaggactca aaaactttag cagcaaagga 1260
aaaagaggtt aaaaagataa cagatggact gcatgccctt caagaagcaa gtaataaaga 1320
tgctgaagct ctggcagctg cacagcagca cttcaatgct gtttccgctg gcctgtccag 1380
taatgaagat ggagcagaag caactcttgc tggtcaaatg atggcctgta aaaatgatat 1440
aagtaaagct cagacagaag ccaaacaggc tcagatgaag ttgaagcatg ctcaacagga 1500
attaaagaat aaacaagctg aagttaagaa gatggatagt ggctacagga aggatcaaga 1560
agctctagaa gctgtaaaaa gacttaaaga aaaacttgaa gctgaaatga aaaagctaaa 1620
ttatgaagaa aataaagagg aaagcctttt ggaaaagcgc aggcagctgt ctcgtgatat 1680
tggtagattg aaagaaacat atgaagctct attagccaga tttcccaatc ttcgatttgc 1740
atacaaggat ccagagaaga actggaatag aaattgtgtg aaaggacttg tggcttctct 1800
gattagtgtg aaagacactt ctgcaaccac agctttagaa ttagtggctg gagaacgact 1860
ctacaatgtt gtagtagaca cagaagttac tggtaaaaag ctactagaaa ggggggaact 1920
gaaacgtcga tacactataa ttccactcaa taaaatttca gccagatgta ttgcaccaga 1980
aactctgaga gttgctcaga atcttgttgg ccctgacaac gttcatgtgg ctctttcctt 2040
ggttgaatat aaaccagaac ttcagaaagc aatggagttt gtctttggaa caacatttgt 2100
ttgtgacaat atggataatg ccaaaaaagt ggcctttgat aagaggataa tgactagaac 2160
tgtaactctc ggaggtgatg tgtttgatcc tcatgggaca ttgagtggag gtgctcgatc 2220

ccaggcagct tccattttaa ccaagtttca agaactcaaa gatgttcagg atgaactgag 2280
aatcaaagag aatgagctgc gggctctaga agaggaatta gcaggtctta aaaacactgc 2340
tgaaaagtat cgccaactaa aacagcagtg ggagatgaaa actgaagagg cagatttatt 2400
acaaaccaag ctccagcaaa gctcatatca caagcaacaa gaagaattag atgcccttaa 2460
aaaaaccatt gaggaaagtg aggagacttt gaaaaacact aaagaaatcc aaagaaaagc 2520
agaagaaaaa tatgaagtat tggaaaataa aatgaaaaat gcagaagctg aaagagagcg 2580
agaactgaaa gatgctcaga aaaaactgga ttgtgccaaa acaaaggcag atgcatctag 2640
caagaagatg aaagaaaaac aacaggaagt tgaagctatc actctggaac tggaagagct 2700
caagagagag catacatctt acaaacaaca gcttgaagct gtaaatgaag ctatcaaatc 2760
ctatgaaagt cagattgaag taatggcagc tgaggtggct aaaaataagg agtcagtaaa 2820
taaagctcaa gaagaggtga ccaagcaaaa agaggtgata acagcccaag acactgtaat 2880
taagctaaat atgcagaagt ggcaaaacac aaggagcaaa acaatgattc tcagccttaa 2940
aattaaggaa ttagaccacc acatcagcaa acataaacgg gaggctgaag atggtgctgc 3000
aaaggtatcc aaaatgttga aagattatga ctggattaat gcagagagac acctctttgg 3060
ccaacccaat agtgcctatg atttcaaaac taacaaccct aaagaagctg gtcagagact 3120
tcagaagttg caagaaatga aggagaaact aggaagaaat gtcaatatga gagctatgaa 3180
tgtattgaca gaagctgaag agcgatgcaa tgacttgatg aagaagaaga gaattgtaga 3240
aaatgacaaa tccaaaattc ttacaactat agaagacctt gaccagaaga aaaaccaagc 3300
cctaaatatt gcatggcaaa aggtgaacaa ggactttggg tctatttttt ctactctttt 3360
gcctggtgct aatgctatgc ttgcaccacc agagggtcaa actgttttgg atggtctgga 3420
gttcaaggtt gccttaggaa atacctggaa agaaaaccta actgaactta gtggtggtca 3480
gaggtcttta gtggccttgt cattaatact gtccatgctt ctcttcaaac ctgctccaat 3540
ttatatcctt gatgaggtag atgcagcctt ggatctttct catacccaaa acattggaca 3600
gatgctgcgt actcatttca cacattctca gttcattgtg gtgtcactaa aagaaggtat 3660
gttcaacaat gcaaacgttc ttttcaaaac caagtttgtg gatggtgttt ctacagtagc 3720
cagatttact caatgtcaaa atggaaagat ttcaaaggaa gcaaaatcca aggcaaaacc 3780
acccaaagga gcacatgtgg aagtttaaac tacaaagtta tttcttcatc ttgacctgtt 3840
tttttaaatg taaactttta aggacttgag ataactaatt tgtttatata caaaaattaa 3900
tgttactgtg ttacttaacc catgttttct ctttatataa tcacttatcg cttacaaatg 3960
agcatatatt cctcatctct taactagtct aattatggtc caattattgt ggttgtgatt 4020
ttatgcatat ccatcaaaat gttttttttc ttatgcgggt cttttatata ttagggatcc 4080
tgagataccc gattctatat gtaaaagcta atatacaaaa aagcagatta aattacatga 4140
taaatgtagc tgaaaaaaaa aaaaaa 4166

<210> 20
<211> 4682

<212>  DNA
<213>  Homo sapiens

<400>  20
cttacggcag tctcgcggga tttccccctc tcgcgggaat tatttgaacg ttcgagcggt      60

aaatactccc tggggctgtc atagaagact actcggagag cgctgcctct gggttggcgg     120

gctggcaggc tgtagccgag cgcgggcagg actcgtcccg gcagggttcc agagccatgg     180

gagcggaaag gaggctgctg tcgattaagg aggcctttcg gctggcgcag cagccgcacc     240

agaaccaggc gaagctggtg gtggcgctga gccgcaccta ccgcacgatg gatgataaga     300

cagtttttca tgaggagttc attcattacc ttaaatatgt tatggtggtc tataaacgtg     360

aaccagctgt ggagagggta atagaatttg cagcaaagtt tgttacctca tttcaccaat     420

cagatatgga agatgatgag gaagaggaag atggtggcct tttaaattat ttgtttactt     480

ttctcttaaa gtctcatgaa gcaaacagca atgcagtgag atttagagtg tgcctgctca     540

taaacaagct tttgggaagt atgccagaaa atgctcagat tgatgatgat gtgtttgata     600

aaattaataa agccatgctt attagattga aagataagat tccaaatgtg agaatacagg     660

cagttctggc gctttcacga cttcaggatc ccaaggatga tgaatgccca gtggttaatg     720

catatgctac tttgattgaa aatgattcaa atccagaagt tagacgggca gtgttatcat     780

gtattgcacc atcagcaaag actttgccaa aaattgtagg gcgcaccaag gatgtgaaag     840

aggctgtcag aaagctggct tatcaggttt tagctgaaaa ggttcatatg agagctatgt     900

ccattgctca gagagtaatg ctccttcaac aaggtcttaa tgacagatca gatgctgtga     960

aacaagctat gcagaagcat cttcttcaag gctggttacg gttctctgaa ggaaatatct    1020

tagagttgct ccatcggttg gatgtagaaa attcttctga agtggcagtc tctgttctca    1080

atgccttgtt ttcaataact cctctcagtg aactggtggg actctgtaaa aacaatgatg    1140

gcaggaaatt gattccagtg gaaacattaa ctcctgaaat tgctttgtat tggtgtgccc    1200

tttgtgaata tttgaaatca aaaggagatg aaggtgaaga atttttagag cagattttgc    1260

cagagcctgt agtatatgca gactatttat tgagttacat ccagagcatt ccagttgtta    1320

atgaagaaca cagaggtgat ttttcctata ttggaaattt gatgacaaaa gaattcatag    1380

gtcaacaatt gattctaatt attaagtctt tggataccag tgaagaagga ggaagaaaaa    1440

aactgctggc tgttttacag gagattctta ttttacccac aatcccaata tccctggttt    1500

cttttcttgt tgaaagacta ctccacatca ttatagatga taataagaga acacaaattg    1560

ttacagaaat tatctcagag attcgggcgc ccattgttac tgttggtgtt aataacgatc    1620

cagctgatgt aagaaagaaa gaactcaaga tggctgaaat aaaagttaag cttatcgaag    1680

ccaaagaagc tttggaaaat tgcattacct tacaggattt taatcggcca tcagaattaa    1740

aagaagaaat aaaagcatta gaagatgcca gaataaacct tttgaaagag acagagcaac    1800

ttgaaattaa agaagtccac atagagaaga atgatgctga aacattgcag aaatgtctta    1860

ttttatgcta tgaactgttg aagcagatgt ccatttcaac aggcttaagt gcaaccatga    1920

atggaatcat cgaatctttg attcttcctg gaataataag tattcatcct gttgtaagaa    1980

acctggctgt tttatgcttg ggatgctgtg gactacagaa tcaggatttt gcaaggaaac 2040
acttcgtatt actattgcag gttttgcaaa ttgatgatgt cacaataaaa ataagtgctt 2100
taaaggcaat ctttgaccaa ctgatgacgt tcgggattga accatttaaa actaaaaaaa 2160
tcaaaacact tcattgtgaa ggtacagaaa taaacagtga tgatgagcaa gaatcaaaag 2220
aagttgaaga gactgctaca gctaagaatg ttctgaaact cctttctgat ttcttagata 2280
gtgaggtatc tgaacttagg actggagctg cagaaggact agccaagctg atgttctctg 2340
ggcttttggt cagcagcagg attctttctc gtcttatttt gttatggtac aatcctgtga 2400
ctgaagagga tgttcaactt cgacattgcc taggcgtgtt cttccccgtg tttgcttatg 2460
caagcaggac taatcaggaa tgctttgaag aagcttttct tccaaccctg caaacactgg 2520
ccaatgcccc tgcatcttct cctttagctg aaattgatat cacaaatgtt gctgagttac 2580
ttgtagattt gacaagacca agtggattaa atcctcaggc caagacttcc caagattatc 2640
aggccttaac agtacatgac aatttggcta tgaaaatttg caatgagatc ttaacaagtc 2700
cgtgctcgcc agaaattcga gtctatacaa aagccttgag ttctttagaa ctcagtagcc 2760
atcttgcaaa agatcttctg gttctattga atgagattct ggagcaagta aaagatagga 2820
catgtctgag agctttggag aaaatcaaga ttcagttaga aaaaggaaat aaagaatttg 2880
gtgaccaagc tgaagcagca caggatgcca ccttgactac aactactttc caaaatgaag 2940
atgaaaagaa taaagaagta tatatgactc cactcagggg tgtaaaagca acccaagcat 3000
caaagtctac tcagctaaag actaacagag gacagagaaa agtgacagtt tcagctagga 3060
cgaacaggag gtgtcagact gctgaagccg actctgaaag tgatcatgaa gttccagaac 3120
cagaatcaga aatgaagatg agactaccaa gacgagccaa aaccgcagca ctagaaaaaa 3180
gtaaacttaa ccttgcccaa tttctcaatg aagatctaag ttaggaaaga cgatggaggt 3240
ggaatccttt aagattatgt ccagttattt gctttaataa agaagaagtt acccttgtca 3300
aaatcagaac aaacctgatg tctttctgaa gattttctgc tgtgcgcttc cacgttactt 3360
tggcctgtat taaagcagta gagcagcatc agttattata gtccagaaaa agtgtgcatc 3420
agtcagtcac acagatttat cacaatctga ggtgggccta ggaatctcat ttttaaatag 3480
tctctccaag tgattcttat gaactcttta tgtttaaaat catgtcatta tggaaaactt 3540
acaagtgtaa ctagctagta gcttgcattt gagaagctta tgacttagat gggcagaatc 3600
aacaaagatg aaaccgcctg aggacacatt taacaagtaa catttctagg gaaaatgaag 3660
gaagtaccac aaactggcta gaaaggagct tatcaatcac cagtgaggaa gaccagtata 3720
acgttcaaca acagttattt tgacaaaaac ttattttgtg attcctacag tgaaaacatt 3780
tttggtgata tctgcctggg aaatctctct tcctaaagta tttgtatatg ggagtccttg 3840
tttgtgaatg tttcctggat tagggaggtg tcaacataaa tgtattatta accatgaagc 3900
tgctcgctat atttttggca taacaaaata atatttattt actgtggata ataattctag 3960
tgggaatata atgtgacagg aacttctctt tatatacgct accaatttat gagcactatt 4020

cactgtcaat ttcatttctt gtcttttgaa attgacactt ggcctgactt acgaaacttg 4080

tactatatga aattggtcct cttttctgca atacccaacg aaacaccttt tctctttatt 4140

attcagaaat gtcctaacat ggatctgttt gttttaataa ttgtgctttt tttaggctta 4200

tcatctacta gaggccattt acttaaggtg aaattttaag atggagctaa agtaagatca 4260

ctggttttta gaaccaaatt gctatacata tgtgcctcat agaacttata aaaggagtca 4320

aagtttcaaa gcaagatagt tattaagcaa aaggaaaaat ggtaatgata gaaagtcagt 4380

taaaaataga tgattgttct tcattctgtt tgttggctct gtgttctcct gtgcttcaga 4440

ttccttatgt gttgttgttt taaagacaat ttgcaggggg ttgggagaag gactgaaaag 4500

gtacattaag tgtgctgtaa ggaaaagtct tagaaacata ataagctaaa atcccattca 4560

cacatggcca ggctatccaa aaagaaagga gccatgttct catgtggttt accataccaa 4620

agcttgcttt ctctggcatg ggaaaaataa atttaagcac caaaaaaaaa aaaaaaagaa 4680

aa 4682

<210> 21
<211> 2150
<212> DNA
<213> Homo sapiens

<400> 21
ctcgagccac gaaggccccg ctgtcctgtc tagcagatac ttgcacggtt tacagaaatt 60

cggtccctgg gtcgtgtcag gaaactggaa aaaaggtcat aagcatgaag cgcagttcag 120

tttccagcgg tggtgctggc cgcctctcca tgcaggagtt aagatcccag gatgtaaata 180

aacaaggcct ctatacccct caaaccaaag agaaaccaac ctttggaaag ttgagtataa 240

acaaaccgac atctgaaaga aaagtctcgc tatttggcaa aagaactagt ggacatggat 300

cccggaatag tcaacttggt atattttcca gttctgagaa aatcaaggac ccgagaccac 360

ttaatgacaa agcattcatt cagcagtgta ttcgacaact ctgtgagttt cttacagaaa 420

atggttatgc acataatgtg tccatgaaat ctctacaagc tccctctgtt aaagacttcc 480

tgaagatctt cacatttctt tatggcttcc tgtgcccctc atacgaactt cctgacacaa 540

agtttgaaga agaggttcca agaatcttta aagaccttgg gtatcctttt gcactatcca 600

aaagctccat gtacacagtg ggggctcctc atacatggcc tcacattgtg gcagccttag 660

tttggctaat agactgcatc aagatacata ctgccatgaa agaaagctca cctttatttg 720

atgatgggca gccttgggga gaagaaactg aagatggaat tatgcataat aagttgtttt 780

tggactacac cataaaatgc tatgagagtt ttatgagtgg tgccgacagc tttgatgaga 840

tgaatgcaga gctgcagtca aaactgaagg atttatttaa tgtggatgct tttaagctgg 900

aatcattaga agcaaaaaac agagcattga atgaacagat tgcaagattg gaacaagaaa 960

gagaaaaaga accgaatcgt ctagagtcgt tgagaaaact gaaggcttcc ttacaaggag 1020

atgttcaaaa gtatcaggca tacatgagca atttggagtc tcattcagcc attcttgacc 1080

agaaattaaa tggtctcaat gaggaaattg ctagagtaga actagaatgt gaaacaataa 1140

aacaggagaa cactcgacta cagaatatca ttgacaacca gaagtactca gttgcagaca 1200

ttgagcgaat aaatcatgaa agaaatgaat tgcagcagac tattaataaa ttaaccaagg 1260

acctggaagc tgaacaacag aagttgtgga atgaggagtt aaaatatgcc agaggcaaag 1320

aagcgattga aacacaatta gcagagtatc acaaattggc tagaaaatta aaacttattc 1380

ctaaaggtgc tgagaattcc aaaggttatg actttgaaat taagtttaat cccgaggctg 1440

gtgccaactg ccttgtcaaa tacagggctc aagtttatgt acctcttaag gaactcctga 1500

atgaaactga agaagaaatt aataaagccc taaataaaaa aatgggtttg gaggatactt 1560

tagaacaatt gaatgcaatg ataacagaaa gcaagagaag tgtgagaact ctgaaagaag 1620

aagttcaaaa gctggatgat ctttaccaac aaaaaattaa ggaagcagag gaagaggatg 1680

aaaaatgtgc cagtgagctt gagtccttgg agaaacacaa gcacctgcta gaaagtactg 1740

ttaaccaggg gctcagtgaa gctatgaatg aattagatgc tgttcagcgg gaataccaac 1800

tagttgtgca aaccacgact gaagaaagac gaaaagtggg aaataacttg caacgtctgt 1860

tagagatggt tgctacacat gttgggtctg tagagaaaca tcttgaggag cagattgcta 1920

aagttgatag agaatatgaa gaatgcatgt cagaagatct ctcggaaaat attaaagaga 1980

ttagagataa gtatgagaag aaagctactc taattaagtc ttctgaagaa tgaagataaa 2040

atgttgatca tgtatatata tccatagtga ataaaattgt ctcagtaaaa aaaaaaaaaa 2100

aaaaaaaaaa aaaaaaaaaa aaaaaaaaaa aaaaaaaaaa aaaaaaaaaa 2150

<210> 22
<211> 5297
<212> DNA
<213> Homo sapiens

<400> 22
caacaaacta agcagttgct caatcgttga ttgatcctca ggcacgtcta gattttccca 60

cttacatagg tcttgttaag aaaccagtcc tgccttcttg ccactcgtgt ctttcgatgg 120

ctcccttccc gaagtcccgc tgcctctaag cggagtgtta agcggggctc tcggaagccg 180

gtggaccgag atttccccgg gtggggcggg cgcggtgtag cggcccgcgg gctgacttgc 240

tcccggctgt cccccggccc cagcgaccat gccccgtaaa ggcacccagc cctccactgc 300

ccggcgcaga gaggaagggc cgccgccgcc gtcccctgac ggcgccagca gcgacgcgga 360

gcctgagccg ccgtccggcc gcacggagag cccagccacc gccgcagaga ctgcaagtga 420

ggaacttgat aatagaagtt tagaagagat tttgaacagc attcctcctc ccccgcctcc 540

agcaatgacc aatgaagctg gagctcctcg gcttatgata actcatattg taaaccagaa 540

cttcaaatcc tatgctgggg agaaaattct gggacctttc cataagcgct tttcctgtat 600

tatcgggcca aatggcagtg gcaaatccaa tgttattgat tctatgcttt ttgtgtttgg 660

ctatcgagca caaaaaataa gatctaaaaa actctcagta ttaatacata attctgatga 720

acacaaggac attcagagtt gtacagtaga agttcatttt caaaagataa ttgataagga 780

aggggatgat tatgaagtca ttcctaacag taatttctat gtatccagaa cggcctgcag 840

agataatact tctgtctatc acataagtgg aaagaaaaag acatttaagg atgttggaaa 900
tcttcttcga agccatggaa ttgacttgga ccataataga tttttaattt tacagggtga 960
agttgaacaa attgctatga tgaaaccaaa aggccagact gaacacgatg agggtatgct 1020
tgaatattta gaagatataa ttggttgtgg acggctaaat gaacctatta aagtcttgtg 1080
tcggagagtt gaaatattaa atgaacacag aggagagaag ttaaacaggg taaagatggt 1140
ggaaaaggaa aaggatgcct tagaaggaga gaaaaacata gctatcgaat ttcttacctt 1200
ggaaaatgaa atatttagaa aaaagaatca tgtttgtcaa tattatattt atgagttgca 1260
gaaacgaatt gctgaaatgg aaactcaaaa ggaaaaaatt catgaagata ccaaagaaat 1320
taatgagaag agcaatatac tatcaaatga aatgaaagct aagaataaag atgtaaaaga 1380
tacagaaaag aaactgaata aaattacaaa atttattgag gagaataaag aaaaatttac 1440
acagctagat ttggaagatg ttcaagttag agaaaagtta aaacatgcca cgagtaaagc 1500
caaaaaactg gagaaacaac ttcaaaaaga taaagaaaag gttgaagaat ttaaaagtat 1560
acctgccaag agtaacaata tcattaatga aacaacaacc agaaacaatg ccctcgagaa 1620
ggaaaaagag aaagaagaaa aaaaattaaa ggaagttatg gatagcctta aacaggaaac 1680
acaagggctt cagaaagaaa aagaaagtcg agagaaagaa cttatgggtt tcagcaaatc 1740
ggtaaatgaa gcacgttcaa agatggatgt agcccagtca gaacttgata tctatctcag 1800
tcgtcataat actgcagtgt ctcaattaac taaggctaag gaagctctaa ttgcagcttc 1860
tgagactctc aaagaaagga aagctgcaat cagagatata gaaggaaaac tccctcaaac 1920
tgaacaagaa ttaaaggaga aagaaaaaga acttcaaaaa cttacacaag aagaaacaaa 1980
ctttaaaagt ttggttcatg atctctttca aaaagttgaa gaagcaaaga gctcattagc 2040
aatgaatcga agtaggggga aagtccttga tgcaataatt caagaaaaaa aatctggcag 2100
gattccagga atatatggaa gattggggga cttaggagcc attgatgaaa aatacgacgt 2160
ggctatatca tcctgttgtc atgcactgga ctacattgtt gttgattcta ttgatatagc 2220
ccaagaatgt gtaaacttcc ttaaaagaca aaatattgga gttgcaacct ttataggttt 2280
agataagatg gctgtatggg cgaaaaagat gaccgaaatt caaactcctg aaaatactcc 2340
tcgtttattt gatttagtaa aagtaaaaga tgagaaaatt cgccaagctt tttattttgc 2400
tttacgagat accttagtag ctgacaactt ggatcaagcc acaagagtag catatcaaaa 2460
agatagaaga tggagagtgg taactttaca gggacaaatc atagaacagt caggtacaat 2520
gactggtggt ggaagcaaag taatgaaagg aagaatgggt tcctcacttg ttattgaaat 2580
ctctgaagaa gaggtaaaca aaatggaatc acagttgcaa aacgactcta aaaaagcaat 2640
gcaaatccaa gaacagaaag tacaacttga agaaagagta gttaagttac ggcatagtga 2700
acgagaaatg aggaacacac tagaaaaatt tactgcaagc atccagcgtt taatagagca 2760
agaagaatat ttgaatgtcc aagttaagga acttgaagct aatgtacttg ctacagcccc 2820
tgacaaaaaa aagcagaaat tgctagaaga aaacgttagt gctttcaaaa cagaatatga 2880
tgctgtggct gagaaagctg gtaaagtaga agctgaggtt aaacgcttac acaataccat 2940

cgtagaaatc aataatcata aactcaaggc ccaacaagac aaacttgata aaataaataa 3000
gcaattagat gaatgtgctt ctgctattac taaagcccaa gtagcaatca agactgctga 3060
cagaaacctt caaaaggcac aagactctgt cttgcgtaca gagaaagaaa taaaagatac 3120
tgagaaagag gtggatgacc taacagcaga gctgaaaagt cttgaggaca aagcagcaga 3180
ggtcgtaaag aatacaaatg ctgcagagga atccttacca gagatccaga aagaacatcg 3240
caatctgctt caagaattaa aagttattca agaaaatgaa catgctcttc aaaaagatgc 3300
acttagtatt aagttgaaac ttgaacaaat agatggtcac attgctgaac ataattctaa 3360
aataaaatat tggcacaaag agatttcaaa aatatcactg catcctatag aagataatcc 3420
tattgaagag atttcggttc taagcccaga ggatcttgaa gcgatcaaga atccagattc 3480
tataacaaat caaattgcac ttttggaagc ccggtgtcat gaaatgaaac caaacctcgg 3540
tgccatcgca gagtataaaa agaaggaaga attgtatttg caacgggtag cagaattgga 3600
caaaattact tatgaaagag acagttttag acaggcatat gaagatcttc ggaaacaaag 3660
gcttaatgaa tttatggcag gtttttatat aataacaaat aaattaaagg aaaattacca 3720
aatgcttact ttgggagggg acgccgaact cgagcttgta gacagcttgg atcctttctc 3780
tgaaggaatc atgttcagtg ttcgaccacc taagaaaagt tggaaaaaga tcttcaacct 3840
ttcgggagga gagaaaacac ttagttcatt ggctttagta tttgctcttc accactacaa 3900
gcccactccc ctttacttca tggatgagat tgatgcagcc cttgatttta aaaatgtgtc 3960
cattgttgca ttttatatat atgaacaaac aaaaaatgca cagttcataa taatttctct 4020
tcgaaataat atgtttgaga tttcggatag acttattgga atttacaaga catacaacat 4080
aacaaaaagt gttgctgtaa atccaaaaga aattgcatct aagggacttt gttgaacttt 4140
atgctgaaga ttcttcaagt tgattcagtg tattactgat ttttttctat ttgtaaagga 4200
ttatgagttg tataaaatac atactcccta aactagatca tgaaactggt ttctgtttta 4260
tgcagttgtc atttgtaaag tctaataaaa tattctctat aattgcttct agattacaaa 4320
aatatgacaa tcttgtaagt agcagactat ggagaaaaat gagttacctg gagggtcagg 4380
taacttgcca aactaaaaag tatgttagtt gaggcaaagt cctaagcaag gttgtgctat 4440
caaggctcag cataccttcg tgggcctttg atttaccaac actggaaatg cctgccaact 4500
aatcttggat agattcttta aggcattcca cttagcttgc cagttgagac aatcaccaca 4560
gttattaccc aaatactatg aacatatttt tgtaaaccag tcattctgaa ttatagtgat 4620
gagaatttaa atatatgctt ttctagaatt tgatgtttga ccatttatga cttaattacc 4680
agagagccag taaattagga cagtgtttca acaagcctag gctatctcgt aagttgaaaa 4740
atatcccact atagttgctt catgagtatg aagtaagatg gcctctgatt tacactggtt 4800
caatttacaa attttcaact ttatgatagg tttatccggg tactaaatgc atttcaactt 4860
gatagtttca acttatgata ggtttaccag gatgtagtcc cactgttgag gagcatctat 4920
ttaggggtta attactttag taataagtgg aaagtaagat accttgagta atgtttgcct 4980

ataaaattgt cagcgtattt ttacactatt ggctcaagaa tgttataatg ctaagggaca 5040

taagttggca accacttggt ttttggaagg actttcggta ttgtattaga agtctgccct 5100

agctgttaaa tttctgggta tttatcctaa ggaattaatt aaagagttaa ttgttccttt 5160

cttcagtggg ccattgtttt agatatttaa aaaatccaac agtttctatc ataatgtaac 5220

tgtaaaaatg taaacacatt attagcatgg acttttaaat aaagatttaa agaaagcaaa 5280

aaaaaaaaaa aaaaaaa 5297

<210> 23
<211> 3486
<212> DNA
<213> Homo sapiens

<400> 23
   gagccgactg cggctgcgcg gggtattcga atcggcggcg gcttctagtt tgcggttcag 60
   gtttggccgc tgccggccag cgtcctctgg ccatggacac cccggaaaat gtccttcaga 120
   tgcttgaagc ccacatgcag agctacaagg gcaatgaccc tcttggtgaa tgggaaagat 180
   acatacagtg ggtagaagag aattttcctg agaataaaga atacttgata actttactag 240
   aacatttaat gaaggaattt ttagataaga agaaatacca caatgaccca agattcatca 300
   gttattgttt aaaatttgct gagtacaaca gtgacctcca tcaatttttt gagtttctgt 360
   acaaccatgg gattggaacc ctgtcatccc ctctgtacat tgcctgggcg gggcatctgg 420
   aagcccaagg agagctgcag catgccagtg ctgtccttca gagaggaatt caaaaccagg 480
   ctgaacccag agagttcctg caacaacaat acaggttatt tcagacacgc ctcactgaaa 540
   cccatttgcc agctcaagct agaacctcag aacctctgca taatgttcag gttttaaatc 600
   aaatgataac atcaaaatca aatccaggaa ataacatggc ctgcatttct aagaatcagg 660
   gttcagagct ttctggagtg atatcttcag cttgtgataa agagtcaaat atggaacgaa 720
   gagtgatcac gatttctaaa tcagaatatt ctgtgcactc atctttggca tccaaagttg 780
   atgttgagca ggttgttatg tattgcaagg agaagcttat tcgtggggaa tcagaatttt 840
   cctttgaaga attgagagcc cagaaataca atcaacggag aaagcatgag caatgggtaa 900
   atgaagacag acattatatg aaaaggaaag aagcaaatgc ttttgaagaa cagctattaa 960
   aacagaaaat ggatgaactt cataagaagt tgcatcaggt ggtggagaca tcccatgagg 1020
   atctgcccgc ttcccaggaa aggtccgagg ttaatccagc acgtatgggg ccaagtgtag 1080
   gctcccagca ggaactgaga gcgccatgtc ttccagtaac ctatcagcag acaccagtga 1140
   acatggaaaa gaacccaaga gaggcacctc ctgttgttcc tcctttggca aatgctattt 1200
   ctgcagcttt ggtgtcccca gccaccagcc agagcattgc tcctcctgtt cctttgaaag 1260
   cccagacagt aacagactcc atgtttgcag tggccagcaa agatgctgga tgtgtgaata 1320
   agagtactca tgaattcaag ccacagagtg gagcagagat caaagaaggg tgtgaaacac 1380
   ataaggttgc caacacaagt tcttttcaca caactccaaa cacatcactg ggaatggttc 1440
   aggcaacgcc atccaaagtg cagccatcac ccaccgtgca cacaaaagaa gcattaggtt 1500
   tcatcatgaa tatgtttcag gctcctacac ttcctgatat ttctgatgac aaagatgaat 1560
   ggcaatctct agatcaaaat gaagatgcat ttgaagccca gtttcaaaaa aatgtaaggt 1620
   catctggggc ttggggagtc aataagatca tctcttcttt gtcatctgct tttcatgtgt 1680
   ttgaagatgg aaacaaagaa aattatggat taccacagcc taaaaataaa cccacaggag 1740
   ccaggacctt tggagaacgc tctgtcagca gacttccttc aaaaccaaag gaggaagtgc 1800
   ctcatgctga agagtttttg gatgactcaa ctgtatgggg tattcgctgc aacaaaaccc 1860
   tggcacccag tcctaagagc ccaggagact tcacatctgc tgcacaactt gcgtctacac 1920
   cattccacaa gcttccagtg gagtcagtgc acattttaga agataaagaa aatgtggtag 1980
   caaaacagtg tacccaggcg actttggatt cttgtgagga aaacatggtg gtgccttcaa 2040
   gggatggaaa attcagtcca attcaagaga aaagcccaaa acaggccttg tcgtctcaca 2100
   tgtattcagc atccttactt cgtctgagcc agcctgctgc aggtggggta cttacctgtg 2160
   aggcagagtt gggcgttgag gcttgcagac tcacagacac tgacgctgcc attgcagaag 2220
   atccaccaga tgctattgct gggctccaag cagaatggat gcagatgagt tcacttggga 2280
   ctgttgatgc tccaaacttc attgttggga acccatggga tgataagctg attttcaaac 2340
   ttttatctgg gctttctaaa ccagtgagtt cctatccaaa tacttttgaa tggcaatgta 2400
   aacttccagc catcaagccc aagactgaat ttcaattggg ttctaagctg gtctatgtcc 2460
   atcaccttct tggagaagga gcctttgccc aggtgtacga agctacccag ggagatctga 2520
   atgatgctaa aaataaacag aaatttgttt taaaggtcca aaagcctgcc aacccctggg 2580
   aattctacat tgggacccag ttgatggaaa gactaaagcc atctatgcag cacatgttta 2640
   tgaagttcta ttctgcccac ttattccaga atggcagtgt attagtagga gagctctaca 2700
   gctatggaac attattaaat gccattaacc tctataaaaa tacccctgaa aaagtgatgc 2760
   ctcaaggtct tgtcatctct tttgctatga gaatgcttta catgattgag caagtgcatg 2820
   actgtgaaat cattcatgga gacattaaac cagacaattt catacttgga aacggatttt 2880
   tggaacagga tgatgaagat gatttatctg ctggcttggc actgattgac ctgggtcaga 2940
   gtatagatat gaaacttttt ccaaaaggaa ctatattcac agcaaagtgt gaaacatctg 3000
   gttttcagtg tgttgagatg ctcagcaaca aaccatggaa ctaccagatc gattactttg 3060
   gggttgctgc aacagtatat tgcatgctct ttggcactta catgaaagtg aaaaatgaag 3120
   gaggagagtg taagcctgaa ggtcttttta gaaggcttcc tcatttggat atgtggaatg 3180
   aattttttca tgttatgttg aatattccag attgtcatca tcttccatct ttggatttgt 3240
   taaggcaaaa gctgaagaaa gtatttcaac aacactatac taacaagatt agggccctac 3300
   gtaataggct aattgtactg ctcttagaat gtaagcgttc acgaaaataa aatttggata 3360
   tagacagtcc ttaaaaatca cactgtaaat atgaatctgc tcactttaaa cctgtttttt 3420
   tttcatttat tgtttatgta aatgtttgtt aaaaataaat cccatggaat atttccatgt 3480
   aaaaaa 3486

<210>  24
<211>  2130
<212>  DNA
<213>  Homo sapiens

<400>  24
ggttaaacgg ggcccaaggc aggggtggcg ggtcagtgct gctcgggggc ttctccatcc      60

aggtccctgg agttcctggt ccctggagct ccgcacttgg cggcgcaacc tgcgtgaggc     120

agcgcgactc tggcgactgg ccggccatgc cttcccgggc tgaggactat gaagtgttgt     180

acaccattgg cacaggctcc tacggccgct gccagaagat ccggaggaag agtgatggca     240

agatattagt ttggaaagaa cttgactatg gctccatgac agaagctgag aaacagatgc     300

ttgtttctga agtgaatttg cttcgtgaac tgaaacatcc aaacatcgtt cgttactatg     360

atcggattat tgaccggacc aatacaacac tgtacattgt aatggaatat tgtgaaggag     420

gggatctggc tagtgtaatt acaaagggaa ccaaggaaag gcaatactta gatgaagagt     480

ttgttcttcg agtgatgact cagttgactc tggccctgaa ggaatgccac agacgaagtg     540

atggtggtca taccgtattg catcgggatc tgaaaccagc caatgttttc ctggatggca     600

agcaaaacgt caagcttgga gactttgggc tagctagaat attaaaccat gacacgagtt     660

ttgcaaaaac atttgttggc acaccttatt acatgtctcc tgaacaaatg aatcgcatgt     720

cctacaatga gaaatcagat atctggtcat tgggctgctt gctgtatgag ttatgtgcat     780

taatgcctcc atttacagct tttagccaga aagaactcgc tgggaaaatc agagaaggca     840

aattcaggcg aattccatac cgttactctg atgaattgaa tgaaattatt acgaggatgt     900

taaacttaaa ggattaccat cgaccttctg ttgaagaaat tcttgagaac cctttaatag     960

cagatttggt tgcagacgag caaagaagaa atcttgagag aagagggcga caattaggag    1020

agccagaaaa atcgcaggat tccagccctg tattgagtga gctgaaactg aaggaaattc    1080

agttacagga gcgagagcga gctctcaaag caagagaaga aagattggag cagaaagaac    1140

aggagctttg tgttcgtgag agactagcag aggacaaact ggctagagca gaaaatctgt    1200

tgaagaacta cagcttgcta aaggaacgga agttcctgtc tctggcaagt aatccagaac    1260

ttcttaatct tccatcctca gtaattaaga agaaagttca tttcagtggg gaaagtaaag    1320

agaacatcat gaggagtgag aattctgaga gtcagctcac atctaagtcc aagtgcaagg    1380

acctgaagaa aaggcttcac gctgcccagc tgcgggctca agccctgtca gatattgaga    1440

aaaattacca actgaaaagc agacagatcc tgggcatgcg ctagccaggt agagagacac    1500

agagctgtgt acaggatgta atattaccaa cctttaaaga ctgatattca aatgctgtag    1560

tgttgaatac ttggttccat gagccatgcc tttctgtata gtacacatga tatttcggaa    1620

ttggttttac tgttcttcag caactattgt acaaaatgtt cacatttaat ttttctttct     1680

tcttttaaga acatattata aaaagaatac tttcttggtt gggcttttaa tcctgtgtgt    1740

gattactagt aggaacatga gatgtgacat tctaaatctt gggagaaaaa ataatgttag    1800

gaaaaaaata tttatgcagg aagagtagca ctcactgaat agttttaaat gactgagtgg    1860

tatgcttaca attgtcatgt ctagatttaa attttaagtc tgagatttta aatgtttttg    1920

agcttagaaa acccagttag atgcaatttg gtcattaata ccatgacatc ttgcttataa 1980

atattccatt gctctgtagt tcaaatctgt tagctttgtg aaaattcatc actgtgatgt 2040

ttgtattctt tttttttttc tgtttaacag aatatgagct gtctgtcatt tacctacttc 2100

tttcccacta aataaaagaa ttcttcagtt 2130

<210> 25
<211> 1682
<212> DNA
<213> Homo sapiens

<400> 25
cggcagccag cctattcttt ggccgggtcg gtgcgagtgg tcggctgggc agagtgcacg 60

ctgcttggcg ccgcaggctg atcccgccgt ccactcccgg gagcagtgat gttgggcaac 120

tctgcgccgg ggcctgcgac ccgcgaggcg ggctcggcgc tgctagcatt gcagcagacg 180

gcgctccaag aggaccagga gaatatcaac ccggaaaagg cagcgcccgt ccaacaaccg 240

cggacccggg ccgcgctggc ggtactgaag tccgggaacc cgcggggtct agcgcagcag 300

cagaggccga agacgagacg ggttgcaccc cttaaggatc ttcctgtaaa tgatgagcat 360

gtcaccgttc ctccttggaa agcaaacagt aaacagcctg cgttcaccat tcatgtggat 420

gaagcagaaa aagaagctca gaagaagcca gctgaatctc aaaaaataga gcgtgaagat 480

gccctggctt ttaattcagc cattagttta cctggaccca gaaaaccatt ggtccctctt 540

gattatccaa tggatggtag ttttgagtca ccacatacta tggacatgtc aattgtatta 600

gaagatgaaa agccagtgag tgttaatgaa gtaccagact accatgagga tattcacaca 660

taccttaggg aaatggaggt taaatgtaaa cctaaagtgg gttacatgaa gaaacagcca 720

gacatcacta acagtatgag agctatcctc gtggactggt tagttgaagt aggagaagaa 780

tataaactac agaatgagac cctgcatttg gctgtgaact acattgatag gttcctgtct 840

tccatgtcag tgctgagagg aaaacttcag cttgtgggca ctgctgctat gctgttagcc 900

tcaaagtttg aagaaatata ccccccagaa gtagcagagt ttgtgtacat tacagatgat 960

acctacacca agaaacaagt tctgagaatg gagcatctag ttttgaaagt ccttactttt 1020

gacttagctg ctccaacagt aaatcagttt cttacccaat actttctgca tcagcagcct 1080

gcaaactgca aagttgaaag tttagcaatg tttttgggag aattaagttt gatagatgct 1140

gacccatacc tcaagtattt gccatcagtt attgctggag ctgcctttca tttagcactc 1200

tacacagtca cgggacaaag ctggcctgaa tcattaatac gaaagactgg atataccctg 1260

gaaagtctta agccttgtct catggacctt caccagacct acctcaaagc accacagcat 1320

gcacaacagt caataagaga aaagtacaaa aattcaaagt atcatggtgt ttctctcctc 1380

aacccaccag agacactaaa tctgtaacaa tgaaagactg cctttgtttt ctaagatgta 1440

aatcactcaa agtatatggt gtacagtttt taacttaggt tttaatttta caatcatttc 1500

tgaatacaga agttgtggcc aagtacaaat tatggtatct attacttttt aaatggtttt 1560

aatttgtata tcttttgtat atgtatctgt cttagatatt tggctaattt taagtggttt 1620

tgttaaagta ttaatgatgc cagctgtcag gataataaat tgatttggaa aacttaaaaa 1680

aa 1682

<210> 26
<211> 3379
<212> DNA
<213> Homo sapiens

<400> 26
ctggcctggg cctgaagtga gtgagaggca catgaagaga agtattcaag tatttataca 60

gataggaatc aagataatca acaatgtctg tcactgagga agacctgtgc caccatatga 120

aagtagtagt tcgtgtacgt ccggaaaaca ctaaagaaaa agcagctgga tttcataaag 180

tggttcatgt tgtggataaa catatcctag tttttgatcc caaacaagaa gaagtcagtt 240

ttttccatgg aaagaaaact acaaatcaaa atgttataaa gaaacaaaat aaggatctta 300

aatttgtatt tgatgctgtt tttgatgaaa cgtcaactca gtcagaagtt tttgaacaca 360

ctactaagcc aattcttcgt agttttttga atggatataa ttgcacagta cttgcctatg 420

gtgccactgg tgctgggaag acccacacta tgctaggatc agctgatgaa cctggagtga 480

tgtatctaac aatgttacac ctttacaaat gcatggatga gattaaagaa gagaaaatat 540

gtagtactgc agtttcatat ctggaggtat ataatgaaca gattcgtgat ctcttagtaa 600

attcagggcc acttgctgtc cgggaagata cccaaaaagg ggtggtcgtt catggactta 660

ctttacacca gcccaaatcc tcagaagaaa ttttacattt attggataat ggaaacaaaa 720

acaggacaca acatcccact gatatgaatg ccacatcttc tcgttctcat gctgttttcc 780

aaatttactt gcgacaacaa gacaaaacag caagtatcaa tcaaaatgtc cgtattgcca 840

agatgtcact cattgacctg gcaggatctg agcgagcaag tacttccggt gctaagggga 900

cccgatttgt agaaggcaca aatattaata gatcactttt agctcttggg aatgtcatca 960

atgccttagc agattcaaag agaaagaatc agcatatccc ttacagaaat agtaagctta 1020

ctcgcttgtt aaaggattct cttggaggaa actgtcaaac tataatgata gctgctgtta 1080

gtccttcctc tgtattctac gatgacacat ataacactct taagtatgct aaccgggcaa 1140

aggacattaa atcttctttg aagagcaatg ttcttaatgt caataatcat ataactcaat 1200

atgtaaagat ctgtaatgag cagaaggcag agattttatt gttaaaagaa aaactaaaag 1260

cctatgaaga acagaaagcc ttcactaatg aaaatgacca agcaaagtta atgatttcaa 1320

accctcagga aaaagaaatc gaaaggtttc aagaaatcct gaactgcttg ttccagaatc 1380

gagaagaaat tagacaagaa tatctgaagt tggaaatgtt acttaaagaa aatgaactta 1440

aatcattcta ccaacaacag tgccataaac aaatagaaat gatgtgttct gaagacaaag 1500

tagaaaaggc cactggaaaa cgagatcata gacttgcaat gttgaaaact cgtcgctcct 1560

acctggagaa aaggagggag gaggaattga agcaatttga tgagaatact aattggctcc 1620

atcgtgtcga aaaagaaatg ggactcttaa gtcaaaacgg tcatattcca aaggaactca 1680

agaaagatct tcattgtcac catttgcacc tccagaacaa agatttgaaa gcacaaatta 1740

gacatatgat ggatctagct tgtcttcagg aacagcaaca caggcagact gaagcagtat 1800
tgaatgcttt acttccaacc ctaagaaaac aatattgcac attaaaagaa gccggcctgt 1860
caaatgctgc ttttgaatct gacttcaaag agatcgaaca tttggtagag aggaaaaaag 1920
tggtagtttg ggctgaccaa actggcgaac aaccaaagca aaacgatcta cccgggattt 1980
ctgttcttat gaccttttca caacttggac cagttcagcc tattccttgt tgctcatctt 2040
caggtggaac taatctggtt aagattccta cagaaaaaag aactcggaga aaactaatgc 2100
catctccctt gaaaggacag catactctaa agtctccacc atctcaaagt gtgcagctca 2160
atgattctct tagcaaagaa cttcagccta ttgtatatac accagaagac tgtagaaaag 2220
cttttcaaaa tccgtctaca gtaaccttaa tgaaaccatc atcatttact acaagttttc 2280
aggctatcag ctcaaacata aacagtgata attgtctgaa aatgttgtgt gaagtagcta 2340
tccctcataa tagaagaaaa gaatgtggac aggaggactt ggactctaca tttactatat 2400
gtgaagacat caagagctcg aagtgtaaat tacccgaaca agaatcacta ccaaatgata 2460
acaaagacat tttacaacgg cttgatcctt cttcattctc aactaagcat tctatgcctg 2520
taccaagcat ggtgccatcc tacatggcaa tgactactgc tgccaaaagg aaacggaaat 2580
taacaagttc tacatcaaac agttcgttaa ctgcagacgt aaattctgga tttgccaaac 2640
gtgttcgaca agataattca agtgagaagc acttacaaga aaacaaacca acaatggaac 2700
ataaaagaaa catctgtaaa ataaatccaa gcatggttag aaaatttgga agaaatattt 2760
caaaaggaaa tctaagataa atcacttcaa aaccaagcaa aatgaagttg atcaaatctg 2820
cttttcaaag tttatccaat accctttcaa aaatatattt aaaatctttg aaagaagacc 2880
catcttaaag ctaagtttac ccaagtactt tcagcaagca gaaaaatgaa actctttgtt 2940
ttcttctttt gtgttctaaa aaaataaaat ttcaaaagaa aaggttgtct tttaagtttt 3000
ttaaatattt gttgcctttt aaaatccctg agtgtaagtt accatggtgg cagcttagtt 3060
ttactatgcc acaacaagtt gactaggaca ttttagtaaa tggtagtgag ttaaattatc 3120
tttattattt tttaaaaata agaatttaga agtggtaaaa ttatggccca agatgtattt 3180
ggttctctat tatgttttga tacattattt taatcatata tatgactttc cttttcaaaa 3240
atactttaat gtacaagtgt aaatatatgt gcccataaaa tcattgtaaa tattatttag 3300
tcatcacaaa taaaatattg tcccttgcta cttgatatat taaagatgta gattttaaag 3360
tgaaaaaaaa aaaaaaaaa 3379

<210> 27
<211> 47
<212> DNA
<213> Synthetic oligo nucleotide

<400> 27
gcagttgacc tagatgagat tcaagagatc tcatctaggt caactgc 47

<210> 28
<211> 20

<212>  DNA
<213>  Synthetic oligo nucleotide

<400>  28
cgctttcatt ggtccatttc                                                    20


<210>  29
<211>  20
<212>  DNA
<213>  Synthetic oligo nucleotide

<400>  29
ccggccaaag aatagtcgta                                                    20


<210>  30
<211>  24
<212>  DNA
<213>  Synthetic oligo nucleotide

<400>  30
aatctgtaac aatgaaagac tgcc                                               24


<210>  31
<211>  24
<212>  DNA
<213>  Synthetic oligo nucleotide

<400>  31
gataccataa tttgtacttg gcca                                               24


<210>  32
<211>  20
<212>  DNA
<213>  Synthetic oligo nucleotide

<400>  32
gtccagagtt atattggcac                                                    20


<210>  33
<211>  21
<212>  DNA
<213>  Synthetic oligo nucleotide

<400>  33
aacagcaaca atgaatttct c                                                  21



1/107